# EUROPÄISCHE PATENTSCHRIFT

(11) **EP 1 789 019 B1**
(45) Veröffentlichungstag und Bekanntmachung des Hinweises auf die Patenterteilung: **16.01.2008**
(21) Anmeldenummer: 05745334.2
(22) Anmeldetag: 04.05.2005
(51) Int. Cl.: A61K 9/16, A61K 9/19, A61K 47/18, A61K 47/26

(54) **PULVER ENTHALTEND NEUARTIGE OLIGOSACCHARIDGEMISCHE UND VERFAHREN ZU DEREN HERSTELLUNG**
POWDERS CONTAINING NOVEL OLIGOSACCHARIDE MIXTURES AND METHODS FOR PRODUCING THE SAME
POUDRES CONTENANT DE NOUVEAUX MELANGES D'OLIGOSACCHARIDES ET PROCEDES DE REALISATION ASSOCIES

(30) Priorität: 10.05.2004 DE 102004022928
(43) Veröffentlichungstag der Anmeldung: 30.05.2007
(73) Patentinhaber: Boehringer Ingelheim Pharma GmbH & Co. KG, 55216 Ingelheim (DE)
(72) Erfinder: FUHRHERR, Richard, 90453 Nürnberg (DE); BASSARAB, Stefan, 88400 Biberach (DE); BECHTOLD-PETERS, Karoline, 88400 Biberach (DE); FRIESS, Wolfgang, 82393 Iffeldorf (DE); GARIDEL, Patrick, 22846 Norderstedt (DE); SCHULTZ-FADEMRECHT, Torsten, 88437 Maselheim, OT Apfingen (DE)
(74) Vertreter: Hammann, Heinz
(86) Internationale Anmeldenummer: PCT/EP2005/004808
(87) Internationale Veröffentlichungsnummer: WO 2005/113007

(56) Entgegenhaltungen:
- EP-A- 0 630 651
- WO-A-98/16205
- WO-A-03/104473

## Beschreibung

### Gebiet der Erfindung

Die Erfindung betrifft die Verwendung von neuartigen Oligosacchariden / Oligosacchnidgemischen zur Herstellung und Stabilisierung von pharmazeutischen Zusammensetzungen, vorwiegend von Pulvern, die einen pharmazeutischen Wirkstoff enthalten. Die Herstellung der Pulver erfolgt vorzugsweise durch Sprühtrocknung oder Gefriertrocknung. Die vorliegende Erfindung betrifft insbesondere entsprechende antikörperhaltige Pulver sowie Verfahren zu deren Herstellung.

### Hintergrund

In wässrigen Lösungen formulierte Wirkstoffe/Wirkstoffzubereitungen unterliegen teilweise Instabilitäten, welche zu verminderter Wirksamkeit bzw. Bioaktivität und erhöhter Toxizität bzw. zu Unverträglichkeiten führen können. Dies trifft sowohl für klassische Pharmazeutika wie auch für peptid- bzw. proteinhaltige Wirkstoffe zu. Die Stabilität pharmazeutischer Wirkstoffe kann durch Veränderung der Struktur (intern) oder durch Zugabe geeigneter Hilfsstoffe (extern) positiv beeinflusst werden.

Ein übliches Verfahren zur externen Stabilisierung pharmazeutischer Wirkstoffe ist die Verwendung geeigneter Hilfsstoffe. Wirkstoffstabilisierende Hilfsstoffe können grob in folgende Klassen eingeteilt werden: Zucker und Polyole, Aminosäuren, Amine, Salze, Polymere und Tenside.

Zucker und Polyole werden häufig als unspezifische Stabilisatoren eingesetzt. Ihr stabilisierender Effekt wird bei biologischen Wirkstoffen vornehmlich der "preferentialexclusion " zugeschrieben (Xie and Timasheff, 1997a, Biophysical Chemistry, 64(1-3), 25-43; Xie and Timasheff, 1997b, Protein Science, 6(1), 211-221; Timasheff, 1993, Annual review of biophysics and biomolecular structure, 22, 67-97). Bei der Auswahl von Zuckern werden bei biologischen Wirkstoffen zumeist reduzierende Zucker vermieden. Saccharose und Trehalose als nicht reduzierende Zucker werden bevorzugt eingesetzt. Weitere Beispiele für geeignete Hilfsstoffe sind Glucose, Sorbitol, Glycerol (Boctor and Mehta, 1992, Journal of Pharmacy and Pharmacology, 44 (7), 600-3; Timasheff, 1993 (supra); Chang et al, 1993 Pharmaceutical Research, 10(10), 1478-83) und Mannitol (Hermann et al 1996, Pharmaceutical Biotechnology, 9 (Formulation, Characterization, and Stability of Protein Drugs), 303-328; Chan et al 1996 Pharmaceutical Research, 13(5), 756-761). Ferner ist bekannt, dass verschiedenste Polymere stabilisierend auf pharmazeutische Wirkstoffe, vorwiegend auf Proteine, wie zum Beispiel Antikörper, wirken. Das in der Vergangenheit häufig eingesetzte humane Serumalbumin (HSA) verfügt zwar über sehr gute stabilisierende und aggregationshemmende Eigenschaften, ist aber aufgrund seiner potentiellen Kontamination mit "bloodbourne" Erregern mittlerweile ungeeignet. Unter den bisher bekannten Polymeren erweist sich Hydroxypropyl-β-Cyclodextrin (HP-β-CD) als besonders geeignet, da es auch parenteral unbedenklich applizierbar ist. Weitere Beispiele sind höhermolekulare Dextrane (18 bis 82 kD), PVP, Heparin, Gelatine Typ A und B sowie HydroxyethylStärke (HES), Heparin, Dextran-Sulfat, Polyphosphorsäure, Poly-L-Glutaminsäure, Poly-L-Lysin.

Neben Zuckern und Polyolen können auch Aminosäuren stabilisierend eingesetzt werden, alleine oder in Kombination mit anderen Hilfsstoffen. Vorzugsweise werden Aminosäuren bei der Stabilisierung von Proteinen verwendet. Beispielsweise inhibiert die Zugabe von Histidin, Glycin, Natrium-Aspartat (Na-Asp), Glutamat und Lysinhydrochorid (Lys-HCI) die Aggregation von rhKGF in 10 mM Natriumphosphatpuffer (pH 7,0) zusammen mit 5% Mannitol (Zhang et al., 1995 Biochemistry , 34 (27), 8631-41). Die Kombination von Aminosäuren und Propylenglykol verbessert beispielsweise die strukturelle Stabilität von rhCNTF (Dix et al, 1995, Pharmaceutical Research (Supplement), 12, S97). Lysin und Arginin erhöhen die Thermostabilität von IL-1R (Tm-Erhöhung), wogegen Glycin und Alanin destabilisierend wirken (Remmele et al., 1998 Pharmaceutical Research, 15(2), 200-208).

Darüber hinaus lässt sich die Stabilität pharmazeutischer Wirkstoffe durch verschiedene Trocknungsverfahren erhöhen. Die Trocknung erfolgt allerdings zumeist ebenfalls in Gegenwart von Hilfsstoffen, die die Stabilität der Wirkstoffe erhalten und die Eigenschaften der trockenen Pulver verbessern sollen. Ein entscheidender Faktor bei der Stabilisierung durch Trocknung ist die Immobilisierung des Wirkstoffs in einer amorphen Matrix. Der amorphe Zustand besitzt eine hohe Viskosität mit geringer molekularer Beweglichkeit und geringer Reaktivität. Vorteilhafte Hilfsstoffe müssen also in der Lage sein eine amorphe Matrix mit möglichst hoher Glasübergangstemperatur zu bilden, in die der Wirkstoff eingebettet wird. Die Auswahl der Hilfsstoffe hängt somit insbesondere von ihren Stabilisierungsfähigkeiten ab. Darüber hinaus spielen aber auch Faktoren wie die pharmazeutische Akzeptanz des Hilfsstoffs sowie dessen Einfluss auf die Teilchenbildung, die Dispergierbarkeit und die Fließeigenschaft eine entscheidende Rolle, insbesondere wenn es sich um Sprühtrocknungsverfahren handelt.

Die Sprühtrocknung stellt ein besonders geeignetes Verfahren zur Erhöhung der chemischen und physikalischen Stabilität von peptid-/proteinartigen pharmazeutischen Wirkstoffen dar (Maa et al., 1998, Pharmaceutical Research, 15(5), 768-775). Besonders im Bereich der pulmonalen Therapie wird die Sprühtrocknung vermehrt eingesetzt (US 5,626,874; US 5,972,388; Broadhead et al., 1994, J. Pharm. Pharmacol., 46(6), 458-467), da die inhalative Applikation auch bei der Behandlung von systemischen Erkrankungen mittlerweile eine Alternative darstellt (WO 99/07340). Voraussetzung ist, dass die mittlere Teilchengröße der Pulver im Bereich von 1-10 µm, vorzugsweise 1-7,5 µm liegt, so dass die Partikel in tiefere Lungenabschnitte und somit in den Blutkreislauf gelangen können. Die DE-A-179 22 07 beschreibt beispielhaft die Herstellung von entsprechenden Sprühtrocknungspartikeln. Mittlerweile sind eine Vielzahl an Verfahren zur Herstellung entsprechender Pulver beschrieben (WO 95/31479; WO 96/09814; WO 96/32096; WO 96/32149; WO 97/41833; WO 97/44013; WO 98/16205; WO 98/31346; WO 99/66903; WO 00/10541; WO 01/13893; Maa et al., 1998, *supra;* Vidgrén et al., 1987, Int. J. Pharmaceutics, 35, 139-144; Niven et al., 1994, Pharmaceutical Research, 11 (8), 1101-1109).

Als Hilfsstoffe eignen sich ebenfalls Zucker und deren Alkohole (z.B. Trehalose, Lactose, Saccharose oder Mannitol) sowie verschiedene Polymere (Maa et al., 1997, Pharm. Development and Technology, 2(3), 213-223; Maa et al., 1998, supra; Dissertation Adler, 1998, Universität Erlangen; Costantino, et al., 1998, J. Pharm. Sci., 87(11), 1406-1411). Die vorwiegend eingesetzten Hilfsstoffe haben allerdings verschiedene Nachteile. Der Zusatz von Trehalose und Mannitol beispielsweise verschlechtert die Fließeigenschaften von Sprühtrocknungsformulierungen (C. Bosquillon et al., 2001 Journal of Controlled Release, 70(3), 329-339). Mannitol neigt zudem bei einem Gehalt von mehr als 20 Gewichtsprozent zur Rekristallisation (Costantino et al., 1998, *supra),* wodurch stabilisierende Effekte dramatisch abnehmen. Lactose, ein häufig verwendeter Hilfsstoff, verbessert zwar die Fließeigenschaften von Sprühtrocknungsformulierungen (C. Bosquillon et al., 2001, *supra),* ist aber insbesondere bei der Formulierung von peptid-/proteinhaltigen Wirkstoffen problematisch, da Lactose aufgrund ihrer reduzierenden Eigenschaft destabilisierende Maillard-Reaktionen mit Peptiden/Proteinen eingehen kann.

Bei der Sprühtrocknung von Antikörpern ohne Zusatz von Stabilisatoren kommt es regelmäßig durch Dehydrierung, Hitze und Scherung zu einer Entfaltung der nativen Sekundärstruktur und somit zu einem dramatischen Verlust an Bioaktivität. Zuvor nach innen gewandte hydrophobe Anteile des Antikörpers kehren dabei nach aussen. Dies geschieht vermehrt an den hydrophoben Grenzflächen der im Verlauf der Sprühtrockung entstehenden Wassertropfen zur Luft. Außerdem lagern sich Antikörper innerhalb der wässrigen Phase zu Dimeren oder Aggregaten höherer Ordnung zusammen. Diese Aggregation ist oftmals irreversibel. Weiterhin stellt die hohe Temperatur, bei der die Proteine versprüht werden, einen kritischen Parameter dar. Durch den hohen Energieeintrag kann es zu einer Destabilisierung der peptitischen Bindungen und zur Denaturierung des Antikörpers kommen. Ferner kommt es zur Aggregatbildung von sprühgetrockneten Antikörpern während der Lagerung der Pulver. Negativ wirkt sich hierbei im Besonderen der Restwassergehalt im Pulver aus. Proteinaggreagate zeichnen sich durch eine verminderte oder fehlende biologische Aktivität sowie eine verstärkte Antigenität aus.

Als Coupling Sugars bezeichnete Mehrfachzucker (Oligosaccharide) mit den Hauptkomponenten Maltosylsucrose und Glucosylsucrose sowie Lactosucrose werden im Lebensmittelbereich eingesetzt. Sie werden als Füll- und Dispergierstoffe neben Süßstoffen wie Aspartam, als mäßig süße Komponenten in Kaugummis, zur Stabilisierung gegen Auskristallisieren von Trehalosesirupen oder als sogenannte NDOs (nichtverdaubare Oligosaccharide) verwendet. Eine Verbesserung und Stabilisierung der Süßungsqualität von Asparagylpeptiden oder des Süß-Sauerverhältnisses in ballast- und süßstoffhaltigen Getränken ist ebenfalls bekannt (US 2003/0059511, EP 1 223 175, DE 199 53 727). Aus US 5,489,577 und EP 0630 651 ist ferner die Verwendung von Oligosacchariden zur Stabilisierung von Suspensionen aus therapeutischen Proteinen und Fett- oder Ölbasen bekannt. Es wird ausgeführt, dass die Proteine ohne eine Vormischung mit den Oligosacchariden bei Vermengung und Verknetung mit den hydrophoben, halbfesten Massen ihre Aktivität verlieren würden. Das Stabilisierungspotential über die Lagerung, in hydrophilen Mischungen oder in Pulvern wird in keiner Weise erwähnt.

WO 98/16205 offenbart Inhalationspulver, die oligosaccharide als Hilfsstoffe enthalten.

Eine Aufgabe der Erfindung bestand darin, neue Hilfsstoffe/Hilfsstoffgemische für die Herstellung von pharmazeutischen Zubereitungen zu Verfügung zu stellen. Die entsprechenden Zubereitungen sollten sich u.a. durch eine gute Langzeitstabilität auszeichnen.

Eine weitere Aufgabe der vorliegenden Erfindung bestand darin neue Hilfsstoffe/Hilfsstoffgemische für die Herstellung von getrockneten pharmazeutischen Zubereitungen bereit zu stellen. Die entsprechenden pulverartigen pharmazeutischen Zubereitungen sollten sich durch eine gute Langzeitstabilität und wenn möglich, durch Inhalierbarkeit auszeichnen.

Eine weitere Aufgabe der vorliegenden Erfindung bestand darin neue Hilfsstoffe/Hilfsstoffgemische für die Herstellung von peptid/-proteinhaltigen pharmazeutischen Formulierungen, insbesondere für solche die durch Sprühtrocknung entstehen, bereit zu stellen. Die entsprechenden peptid/-proteinhaltigen pharmazeutischen Zubereitungen sollten sich wiederum durch eine gute Langzeitstabilität und wenn möglich, durch Inhalierbarkeit auszeichnen.

Eine weitere Aufgabe der vorliegenden Erfindung bestand darin neue Hilfsstoffe/Hilfsstoffgemische für Formulierung von therapeutischen Antikörpern oder Antikörperderivaten, insbesondere für solche die durch Sprühtrocknung entstehen, bereit zu stellen. Die entsprechenden antikörperhaltigen pharmazeutischen Zubereitungen sollten sich wiederum durch eine gute Langzeitstabilität und wenn möglich, durch Inhalierbarkeit auszeichnen.

Eine weitere Aufgabe der vorliegenden Erfindung bestand darin entsprechende pharmazeutische Zubereitungen für die inhalative Applikation bereit zu stellen, sei es in Form eines trockenen Pulvers, eines treibgashaltigen Dosieraerosols oder einer treibgasfreien Inhalationslösung.

Die der Erfindung zugrunde liegenden Aufgaben werden durch die nachfolgenden Ausführungen sowie durch die in den Patentansprüchen dargestellten GegenständeNerfahren gelöst.

### Zusammenfassung der Erfindung

Die vorliegende Erfindung betrifft Pulver enthaltend einen pharmazeutischen Wirkstoff und eine Hilfsstoffkombination enthaltend zumindest ein 1,4 O-verknüpfte(s) Saccharose-Derivat ausgewählt aus den Verbindungen: 1,4 O-verknüpfte D-Gal-Saccharose (Lactosucrose), 1,4 O-verknüpfte D-Glu-Saccharose (Glucosyl-Sucrose), oder 1,4 O-verknüpfte Glu-Glu-Saccharose (Maltosyl-Sucrose) in Kombination mit zumindest einem weiteren Hilfsstoff. Bei dem weiteren Hilfsstoff handelt es sich vorzugsweise um eine Aminosäure, ein Peptid und/oder um ein Mono-, Di- und/oder Oligosaccharid, wobei es sich bei den Oligosaccharid auch um ein zweites 1,4 O-verknüpftes Saccharose-Derivat handeln kann, sofern dieses von dem ersten verschieden ist.

Der Begriff Lactosucrose meint Moleküle mit der nachfolgenden Strukturformel:

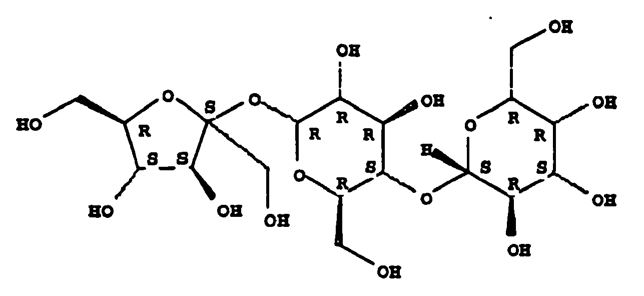

Unter Glycosyl-Sucrose im Sinne der vorliegenden Erfindung versteht man Moleküle mit der nachfolgenden Strukturformel:

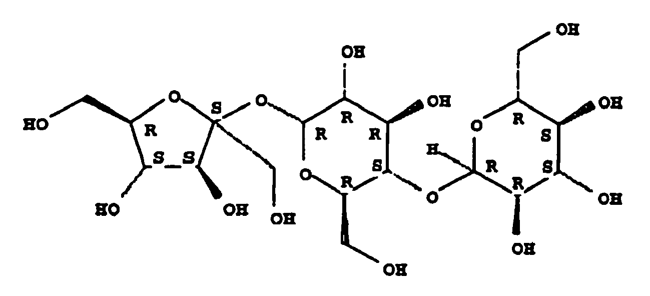

Der Begriff Maltosyl-Sucrose meint Moleküle mit der nachfolgenden Strukturformel:

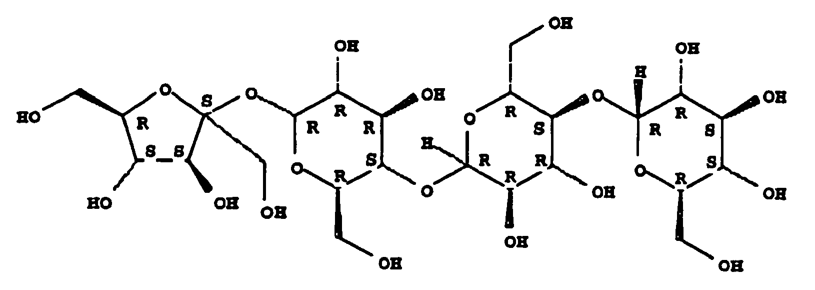

Eine erfindungsgemäße Hilfsstoffkombination ist beispielsweise ein Zuckergemisch welches neben zumindest einem 1,4 O-verknüpften Saccharose-Derivat zumindest einen weiteren Zucker in Form eines Mono-, Di-, und/oder Oligosaccharids enthält. Nach einer bevorzugten Ausführungsform handelt es sich bei dem Zuckergemisch beispielsweise um Lactosucrose in Kombination mit Lactose und Saccharose oder um eine Kombination aus Glucosyl- und Maltosyl-Sucrose, die wieiderum weitere Mono-, Di- oder Oligosaccharide enthalten kann.

Nach einer weiteren Ausführungsform handelt es sich bei der erfindungsgemäßen Hilfsstoffkombination um ein Gemisch aus zumindest einem 1,4 O-verknüpften Saccharose-Derivat in Kombination mit einer Aminosäure, vorzugsweise Isoleucin.

Bei einer weiteren erfindungsgemäßen Hilfsstoffkombination handelt es sich um ein Gemisch aus zumindest einem 1,4 O-verknüpften Saccharose-Derivat in Kombination mit einen Peptid welches nicht dem pharmazeutischen Wirkstoff entspricht. Vorzugsweise enthält das Peptid ein oder mehrere Isoleucinreste. Nach einer weiter bevorzugten Ausführungsform handelt es sich bei dem Peptid um ein Di- oder Tri-Peptid, besonders bevorzugt um ein Isoleucin-haltiges Tri-Peptid oder Di-Peptid,, wobei die Verwendung von Tri-Isoleucin besonders bevorzugt ist.

Nach einer weiteren Ausführungsform der vorliegenden Erfindung handelt es sich bei der Hilfsstoffkombination um ein Zuckergemisch enthaltend zumindest ein 1,4 O-verknüpftes Saccharose-Derivat und zumindest ein weiteres Mono-, Di- und/oder Oligosaccharid, in Kombination mit einer Aminosäure, vorzugsweise Isoleucin, und/oder einem Peptid, vorzugsweise einem Di- oder Tri-Peptid, welches wiederum vorzugsweise zumindest einen Isoleucinrest enthält oder nach einer besonders bevorzugten Ausführungsform aus Isoleucinresten besteht.

Überraschenderweise wurde gefunden, dass die entsprechenden Pulver, nach Trocknung i) eine amorphe Struktur bilden, ii) in einer relativ hohe Ausbeute (zumindest 75% bezogen auf den eingesetzten Feststoff) resultieren, iii) über eine sehr hohe Glasübergangstemperatur größer 40°C verfügen und iv) über eine geringe Tendenz zur Rekristallisation verfügen.

Bei dem pharmazeutischen Wirkstoff handelt es vorzugsweise um ein biologisches Makromolekül, welches ein Polypeptid oder ein Protein, beispielsweise ein Wachstumsfakor, Enzym oder Antikörper sein kann. Besonders erfindungsgemäß sind demnach Pulver mit (a) einem Anteil von 25 bis 99,99°/a (w/w), vorzugsweise von 80 bis 90% (w/w) an einer der erfindungsgemäßen Hilfsstoffkombinationen enthaltend zumindest ein 1,4 O-verknüpften Saccharose-Derivat (bezogen auf die Trockenmasse des Pulvers) und (b) mit einem biologischen Makromolekül als pharmazeutischen Wirkstoff, vorzugsweise in einer Konzentration zwischen 0,01 und 75% (w/w), wiederum bezogen auf die Trockenmasse des Pulvers, wobei die Summe der Gewichtsprozente aus Hilfsstoffkombination enthaltend zumindest ein 1,4 O-verknüpften Saccharose-Derivat und biologischem Makromolekül maximal 100 % (w/w) beträgt.

Nach einer weiteren Ausführungsform betrifft die vorliegende Erfindung somit Pulver, die bezogen auf ihre Trockenmasse (a) zwischen 25 und 90% (w/w) zumindest eines 1,4 O-verknüpften Saccharose-Derivats oder einer Zuckermischung enthaltend zumindest ein 1,4 O-verknüpftes Saccharose-Derivat, (b) zwischen 1 und 39,99% (w/w) zumindest einer Aminosäure und/oder zumindest eines Peptids als weiteren Hilfsstoff und (c) zumindest 0,01 % (w/w) eines pharmazeutischen Wirkstoffs, enthalten. Vorzugsweise handelt es sich bei dem weiteren Hilfsstoff um die Aminosäure Isoleucin oder um ein Di- oder Tri-Peptid, vozugsweise mit zumindest einem Isoleucinrest.

Nach einer speziellen Ausführungsform betrifft die vorliegende Erfindung Pulver die in Bezug auf ihre Trockenmasse einen Anteil von (a) ungefähr 25 bis 80% (w/w) zumindest eines 1,4 O-verknüpften Saccharose-Derivats oder einer Zuckermischung enthaltend zumindest ein 1,4 O-verknüpften Saccharose-Derivat, (b) ungefähr 10 bis 19,99% (w/w) einer Aminosäure, vorzugsweise Isoleucin und (c) ungefähr 0,01 bis 65% (w/w) eines pharmazeutischen Wirkstoffs, vorzugsweise eines Peptids/Proteins, beispielsweise eines Antikörpers, enthalten.

Nach einer weiteren speziellen Ausführungsform betrifft die vorliegende Erfindung Pulver, die in Bezug auf ihre Trockenmasse (a) ungefähr 25 bis 90% (w/w) zumindest eines 1,4 O-verknüpftes Saccharose-Derivats oder einer Zuckermischung enthaltend zumindest ein 1,4 O-verknüpftes Saccharose-Derivat enthält, (b) ungefähr 1 bis 19,99% (w/w) eines Peptids, vorzugsweise eines Isoleucin-haltigen Peptids, noch weiter bevorzugt eines Isoleucin-haltigen Tri-Peptids, besonders bevorzugt Tri-Isoleucin und (c) ungefähr 0,01 bis 74% (w/w) eines pharmazeutischen Wirkstoffs, vorzugsweise eines Peptids/Proteins, beispielsweise eines Antikörpers, enthalten. Die entsprechenden Pulver, insbesondere nach Beimischung von Aminosäure wie zum Beispiel Isoleucin, oder eines Peptids, vorzugsweise eines Isoleucin-haltiges Tri-Peptid zeigen sehr gute Fließeigenschaften und zeichnen sich durch einen sehr hohen Anteil an inhalierbaren Teilchen aus. Ferner verfügen die entsprechenden Pulver über eine sehr gute Prozess- und Lagerstabilität.

Nach einer weiteren Ausführungsform betrifft die vorliegende Erfindung Pulver, die eine der in dieser Anmeldung beschriebenen Hilfsstoffkombination enthalten, die ein oder mehrere 1,4 O-verknüpfte(s) Saccharose-Derivat(e) oder eine Zuckermischung enthaltend zumindest ein 1,4 O-verknüpftes Saccharose-Derivat und zumindest einen pharmazeutischen Wirkstoff, enthalten, wobei das entsprechende Pulver über eine Glasübergangstemperatur von größer 40°C, vorzugsweise von größer 45°C, weiter bevorzugt von größer 50°C, noch weiter bevorzugt von größer 55°C und besonders bevorzugt von größer 60°C beträgt. Üblicherweise verfügen die entsprechenden erfindungsgemäßen Pulver über eine maximale Glasübergangstemperatur von ca. 96 bis 110°C. In Einzelfällen kann der Wert aber auch noch höher liegen. Für die entsprechende Glasübergangstemperatur ist primär der Anteil an dem zugesetzten Hilfsstoff, insbesondere der Anteil an 1,4 O-verknüpftes Saccharose-Derivat bzw. der Anteil des Derivatgemisches im Pulver verantwortlich.

Bei den erfindungsgemäßen Pulvern handelt es sich vorzugsweise um sprühgetrocknete oder gefriergetrocknete Pulver, wobei sprühgetrocknete Pulver besonders bevorzugt sind.

Nach einer weiteren Ausführungsform betrifft die vorliegende Erfindung pharmazeutische Zusammensetzungen für inhalative Applikationen, die eines der hier beschriebenen erfindungsgemäßen Pulver enthalten oder aus diesen bestehen oder aus diesen hergestellt werden. In diesem Zusammenhang bevorzugt sind pharmazeutische Zusammensetzungen, die die erfindungsgemäßen Pulver als Inhalationspulver, treibgashaltige Dosieraerosole oder als treibgasfreie Inhalationslösungen nach Rekonstitution enthalten. Die zur Herstellung der pharmazeutischen Zusammensetzung verwendeten erfindungsgemäßen getrockneten, vorzugsweise sprühgetrockneten Pulver zeichnen sich nach einer weiteren Ausführungsform durch einen hohen Anteil an inhalierbaren Teilchen mit einem mittleren aerodynamischen Teilchendurchmesser (MMAD) von kleiner 10 µm, vorzugsweise von 0,5 - 7,5 µm, weiter bevorzugt vom 0,5 - 5,5 µm, besonders bevorzugt von 0,5 - 5,0 µm aus.

Die Erfindung stellt ferner Verfahren zur Herstellung der entsprechenden erfindungsgemäßen Pulver zur Verfügung, dadurch gekennzeichnet, dass eine Lösung oder Suspension, die zumindest ein oder mehrere 1,4 O-verknüpfte(s) Saccharose-Derivat(e) der oben beschriebenen Verbindungen oder eine diese enthaltende Zuckermischung und zumindest einen pharmazeutischen Wirkstoff enthält, hergestellt wird und diese unter geeigneten Bedingungen versprüht wird. Die Temperatur für den Sprühprozess liegt vorzugsweise zwischen 50 und 200°C (Einströmtemperatur) und 30 und 150°C (Ausströmtemperatur).

### Beschreibung der Abbildungen

Sämtlich in den Beschreibungen aufgeführten Prozentangaben beziehen sich auf Konzentrationsangaben der Feststoffe in den Lösungen (w/v). Sämtliche Legenden der unten beschriebenen Diagramme beziehen sich auf die prozentuale (w/w) Zusammensetzung der durch Sprühtrocknung und Gefriertrocknung mit anschließender Pulverisierung erzielten Pulver. Weiterhin werden in den Legenden die Gesamtfeststoffkonzentration der Lösungen (Total Solid = TS) in Prozent (w/w) genannt. In der Legende des Diagrammes 8 wird Coupling Sugar mit CS abgekürzt. In den Legenden der Diagramme 18, 19, 20 und 21 wird Tri-Isoleucin mit Ile3 abgekürzt. In den Diagrammen 15, 16, 17, 18, 19, 20 und 21 wird weiterhin die während des Sprühtrocknungsprozesses am Büchi B290 eingestellte Zerstäubungsrate (AAF = Atomizing Air Flow) aufgeführt. Dabei entspricht 40 einem realen Volumenfluss von - 0,67 m³/h, 50 einem realen Volumenfluss von - 1,05 m³/h und 60 einem realen Volumenfluss von - 1,74 m³/h. Bei allen anderen Diagrammen betrug die Zerstäubungsrate 40 enstprechend einem realen Volumenfluss von -0,67 m³/h.

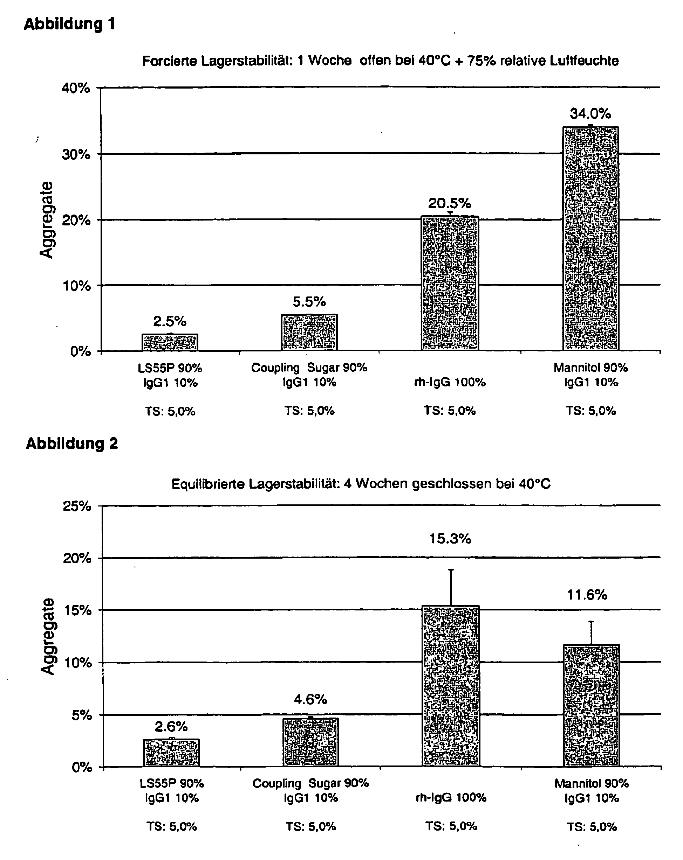

**Abbildung 1** zeigt den Aggregatgehalt nach Gefriertrocknung, Pulverisierung, einwöchiger offener Lagerung bei 75% relativer Luftfeuchtigkeit und 40°C (Forcierte Lagerstabilität) und Rekonstitution. Gefriergetrocknet wurden wässrige Lösungen mit a) 4,5% LS55P-Anteil und 0,5% IgG-Anteil, b) 4,5%Coupling Sugar-Anteil und 0,5% IgG-Anteil, c) 5,0% IgG-Anteil und d) 4,5% Mannitol-Anteil und 0,5% IgG-Anteil. Sowohl die LS55P als auch die Coupling Sugar enthaltenden Pulver zeichnen sich durch einen geringen Anteil an Aggregaten aus.

**Abbildung 2** zeigt den Aggregatgehalt nach Gefriertrocknung, Pulverisierung, Equilibrierung, vierwöchiger trockener Lagerung bei 40°C (Equilibrierte Lagerstabilität) und Rekonstitution. Gefriergetrocknet wurden wässrige Lösungen mit a) 4,5% LS55P-Anteil und 0,5% IgG-Anteil, b) 4,5%Coupling Sugar-Anteil und 0,5% IgG-Anteil, c) 5,0% IgG-Anteil und d) 4,5% Mannitol-Anteil und 0,5% IgG-Anteil. Sowohl die LS55P als auch die Coupling Sugar enthaltenden Pulver zeichnen sich durch einen geringen Anteil an Aggregaten aus.

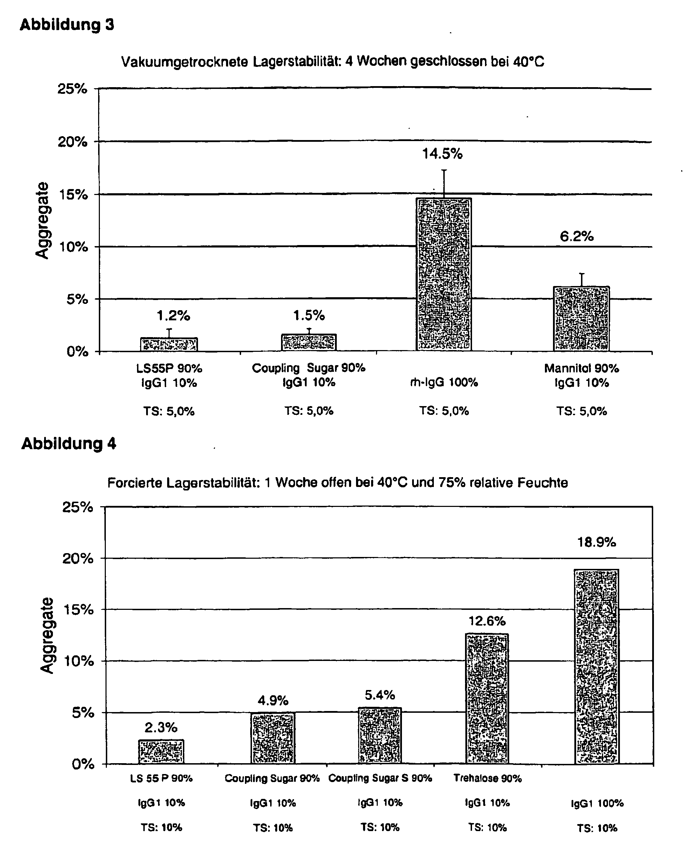

**Abbildung 3** zeigt den Aggregatgehalt nach Gefriertrocknung, Pulverisierung, Vakuumtrocknung, vierwöchiger trockener Lagerung bei 40°C (Vakuumgetrocknete Lagertstabilität) und Rekonstitution. Gefriergetrocknet wurden wässrige Lösungen mit a) 4,5% LS55P-Anteil und 0,5% IgG-Anteil, b) 4,5%Coupling Sugar-Anteil und 0,5% IgG-Anteil, c) 5,0% IgG-Anteil und d) 4,5% Mannitol-Anteil und 0,5% IgG-Anteil. Sowohl die LS55P als auch die Coupling Sugar enthaltenden Pulver zeichnen sich durch einen geringen Anteil an Aggregaten aus.

**Abbildung 4** zeigt den Aggregatgehalt nach Sprühtrocknung, einwöchiger offener Lagerung bei 75% relativer Luftfeuchtigkeit und 40°C (Forcierte Lagerstabilität) und Rekonstitution. Sprühgetrocknet wurden wässrige Lösungen mit a) 9% LS55P-Anteil und 1 % IgG-Anteil, b) 9% Coupling Sugar-Anteil und 1 % IgG-Anteil, c) 9% Coupling Sugar S-Anteil und 1 % IgG-Anteil, d) 9% Trehalose-Anteil und 1 % IgG-Anteil sowie e) 10% IgG-Anteil. Sowohl die LS55P als auch die Coupling Sugar und Coupling Sugar S enthaltenden Pulver zeichnen sich durch einen geringen Anteil an Aggregaten aus.

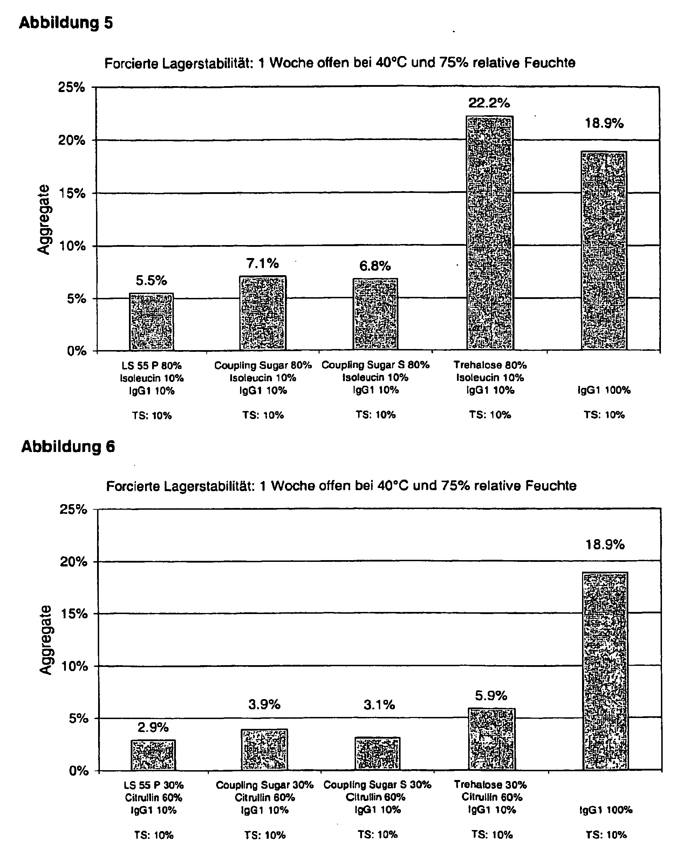

**Abbildung 5** zeigt den Aggregatgehalt nach Sprühtrocknung, einwöchiger offener Lagerung bei 75% relativer Luftfeuchtigkeit und 40°C (Forcierte Lagerstabilität) und Rekonstitution. Sprühgetrocknet wurden wässrige Lösungen mit a) 8% LS55P-Anteil, 1 % Isoleucin-Anteil und 1 % IgG-Anteil, b) 8% Coupling Sugar-Anteil, 1 % Isoleucin-Anteil und 1 % IgG-Anteil, c) 8% Coupling Sugar S-Anteil, 1 % Isoleucin-Anteil und 1 % IgG-Anteil, d) 8% Trehalose-Anteil, 1 % Isoleucin-Anteil und 1 % IgG-Anteil sowie e) 10% IgG-Anteil. Sowohl die LS55P als auch die Coupling Sugar und Coupling Sugar S enthaltenden Pulver zeichnen sich durch einen geringen Anteil an Aggregaten aus.

**Abbildung 6** zeigt den Aggregatgehalt nach Sprühtrocknung, einwöchiger offener Lagerung bei 75% relativer Luftfeuchtigkeit und 40°C (Forcierte Lagerstabilität) und Rekonstitution. Sprühgetrocknet wurden wässrige Lösungen mit a) 3% LS55P-Anteil, 6% Citrullin-Anteil und 1 % IgG-Anteil, b) 3% Coupling Sugar-Anteil, 6% Anteil und 1 % IgG-Anteil, c) 3% Coupling Sugar S-Anteil, 6% Citrullin-Anteil und 1 % IgG-Anteil, d) 3% Trehalose-Anteil, 6% Citrullin -Anteil und 1 % IgG-Anteil sowie e) 10% IgG-Anteil. Sowohl die LS55P als auch die Coupling Sugar und Coupling Sugar S enthaltenden Pulver zeichnen sich durch einen geringen Anteil an Aggregaten aus.

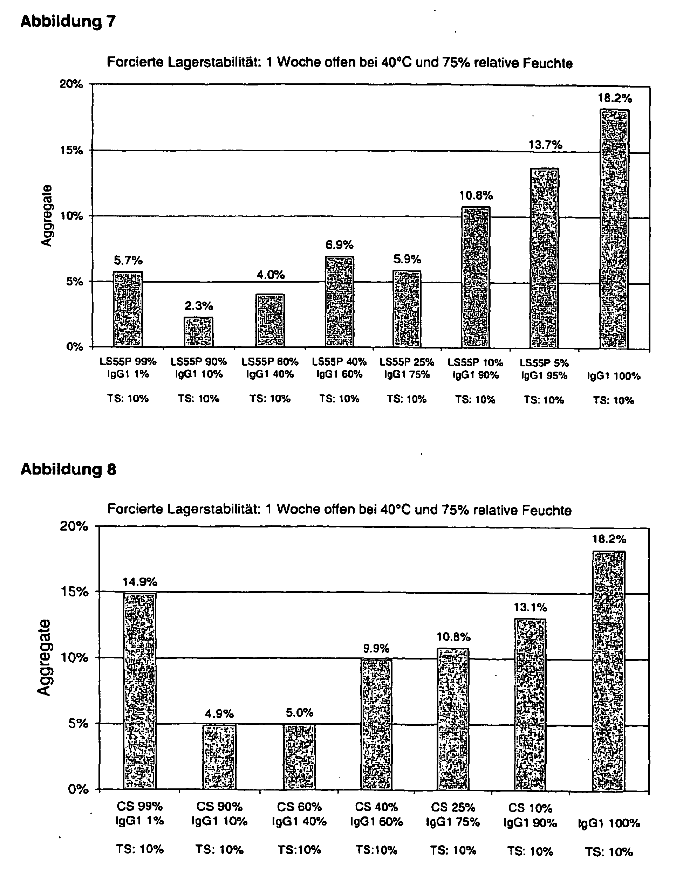

**Abbildung 7** zeigt den Aggregatgehalt nach Sprühtrocknung, einwöchiger offener Lagerung bei 75% relativer Luftfeuchtigkeit und 40°C (Forcierte Lagerstabilität) und Rekonstitution. Sprühgetrocknet wurden wässrige Lösungen mit a) 9,9% LS55P-Anteil 0,1% IgG-Anteil, b) 9% LS55P-Anteil 1% IgG-Anteil, c) 6% LS55P-Anteil 4% IgG-Anteil, d) 4% LSS5P-Anteil 6% IgG-Anteil, e) 2,5% LS55P-Anteil 7,5% IgG-Anteil, f) 1% LS55P-Anteil 9% IgG-Anteil g) 0,5% LS55P-Anteil 9,5% IgG-Anteil sowie e) 10% IgG-Anteil. Die LS55P enthaltenden Pulver zeichnen sich durch einen geringen Anteil an Aggregaten aus.

**Abbildung 8** zeigt den Aggregatgehalt nach Sprühtrocknung, einwöchiger offener Lagerung bei 75% relativer Luftfeuchtigkeit und 40°C (Forcierte Lagerstabilität) und Rekonstitution. Sprühgetrocknet wurden wässrige Lösungen mit a) 9,9% Coupling Sugar-Anteil 0,1% IgG-Anteil, b) 9% Coupling Sugar-Anteil 1% lgG-Anteil, c) 6% Coupling Sugar-Anteil 4% IgG-Anteil, d) 4% Coupling Sugar-Anteil 6% IgG-Anteil, e) 2,5% Coupling Sugar-Anteil 7,5% IgG-Anteil, f) 1 % Coupling Sugar-Anteil 9% IgG-Anteil sowie e) 10% IgG-Anteil. Die Coupling Sugar enthaltenden Pulver zeichnen sich durch einen geringen Anteil an Aggregaten aus.

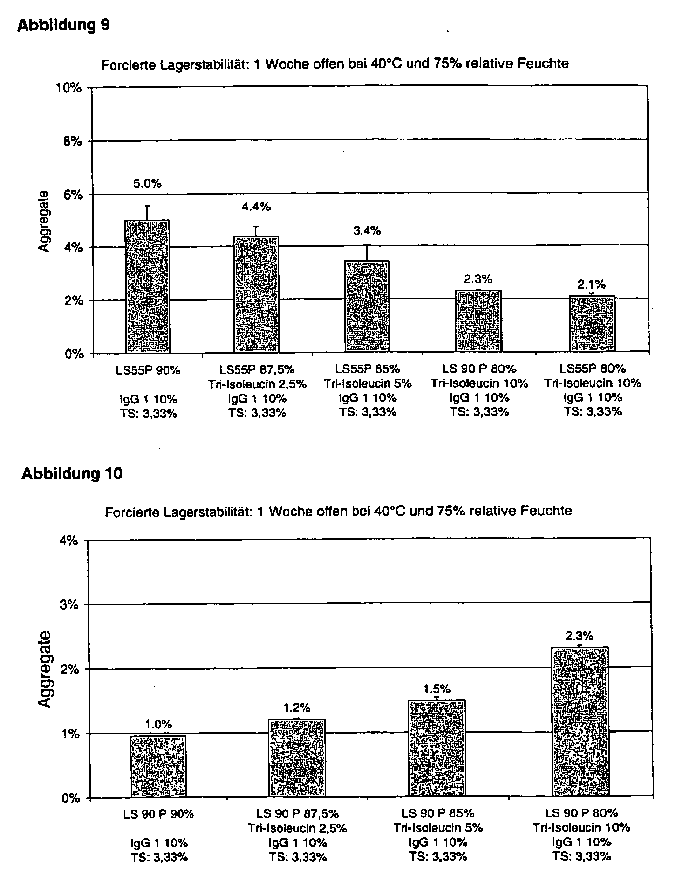

**Abbildung 9** zeigt die Aggregatgehalt nach Sprühtrocknung, einwöchiger offener Lagerung bei 75% relativer Luftfeuchtigkeit und 40°C (Forcierte Lagerstabilität) und Rekonstitution. Sprühgetrocknet wurden wässrige Lösungen mit a) 3,00% LS55P-Anteil und 0,33% IgG-Anteil, b) 2,9166% LS55P-Anteil, 0,0833% Tri-Isoleucin-Anteil und 0,33% IgG-Anteil, c) 2,833% LS55P -Anteil, 0,166% Tri-Isoleucin-Anteil und 0,33% lgG-Anteil sowie d) 2,66% LS55P-Anteil, 0,33% Tri-Isoleucin-Anteil, und 0,33% IgG-Anteil. Die LS55P enthaltenden Pulver zeichnen sich durch einen geringen Anteil an Aggregaten aus. Weiterhin wird die Proteinaggregagation durch eine Erhöhung des Tri-Isoleucin Anteils von 0% auf 10% bezogen auf den Gesamtfeststoffgehalt in den LS55P-haltigen Pulvern signifikant gesenkt.

**Abbildung 10** zeigt den Aggregatbildung nach Sprühtrocknung,einwöchiger offener Lagerung bei 75% relativer Luftfeuchtigkeit und 40°C (Forcierte Lagerstabilität) und Rekonsitution. Sprühgetrocknet wurden wässrige Lösungen mit a) 3,00% LS90P-Anteil und 0,33% lgG-Anteil, b) 2,9166% LS90P-Anteil, 0,0833% Tri-Isoleucin-Anteil und 0,33% lgG-Anteil, c) 2,833% LS90P -Anteil, 0,166% Tri-Isoleucin-Anteil und 0,33% 1gG-Anteil sowie d) 2,66% LS90P-Anteil, 0,33% Tri-Isoleucin-Anteil, und 0,33% IgG-Anteil. Die LS90P enthaltenden Pulver zeichnen sich durch einen geringen Anteil an Aggregaten aus.

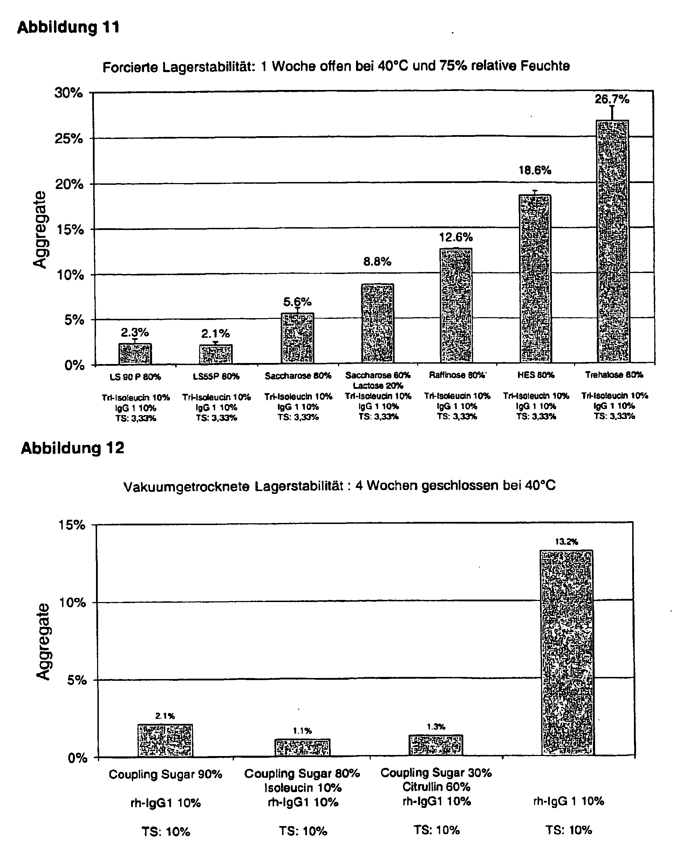

**Abbildung 11** zeigt den Aggregatgehalt nach Sprühtrocknung, einwöchiger offener Lagerung bei 75% relativer Luftfeuchtigkeit und 40°C (Forcierte Lagerstabilität) und Rekonstitution. Sprühgetrocknet wurden wässrige Lösungen mit a) 2,66% LS90P-Anteil, 0,33% Tri-Isoleucin-Anteil, und 0,33% IgG-Anteil, b) 2,66% LS55P-Anteil, 0,33% Tri-Isoleucin-Anteil, und 0,33% IgG-Anteil, c) 2,66% Saccharose-Anteil, 0,33% Tri-Isoleucin-Anteil und 0,33% IgG-Anteil d) 2,00% Saccharose-Anteil, 0,66% Lactose-Anteil, 0,33% Tri-Isoleucin-Anteil und 0,33% IgG-Anteil, e) 2,66% Raffinose-Anteil, 0,33% Tri-Isoleucin-Anteil und 0,33% IgG-Anteil, f) 2,66% Hydroxyethylstärke (HES)-Anteil, 0,33% Tri-Isoleucin-Anteil und 0,33% IgG-Anteil sowie g) 2,66% Trehalose-Anteil, 0,33% Tri-Isoleucin-Anteil und 0,33% IgG-Anteil. Die LS90P und LS55P enthaltenden Pulver zeichnet sich durch einen geringen Anteil an Aggregaten aus. Besonders im Vergleich zur im Stand der Technik aufgeführten Raffinose und Hydroxyethylstärke (HES).

**Abbildung 12** zeigt den Aggregatgehalt nach Sprühtrocknung, Vakuumtrocknung, vierwöchiger trockener Lagerung bei 40°C (Vakuumgetrocknete Lagertstabilität) und Rekonstitution. Sprühgetrocknet wurden wässrige Lösungen mit a) 9% Coupling Sugar-Anteil und 1 % IgG-Anteil, b) 8% Coupling Sugar-Anteil, 1 % (w/) Isoleucin-Anteil und 1% 1gG-Anteil, c) 3% Coupling Sugar-Anteil, 6% Citrullin-Anteil und 1 % IgG-Anteil sowie d) 10% IgG-Anteil Die Coupling Sugar enthaltenden Pulver zeichnen sich durch einen geringen Anteil an Aggregaten aus.

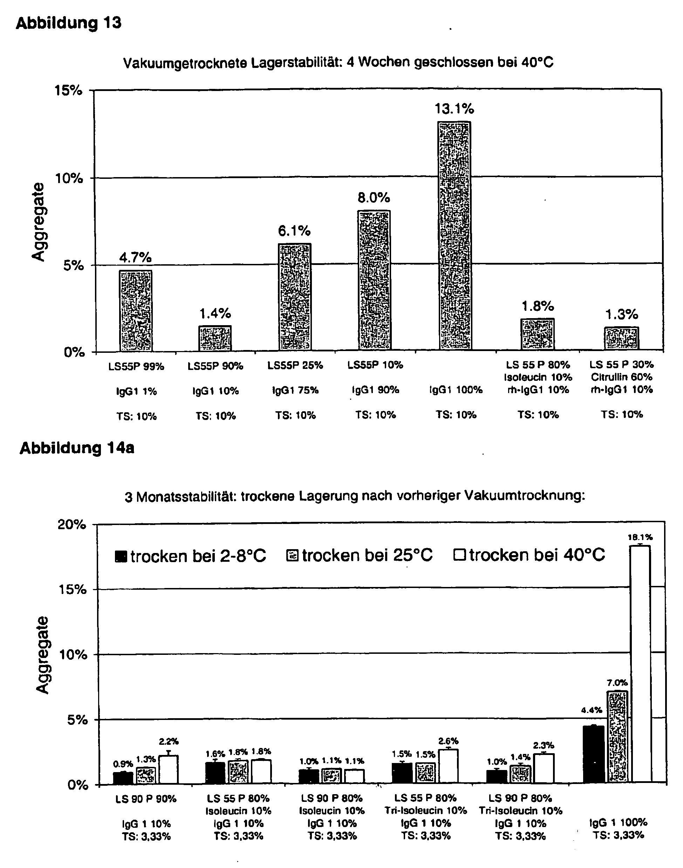

**Abbildung 13** zeigt den Aggregatgehalt nach Sprühtrocknung, Vakuumtrocknung, vierwöchiger trockener Lagerung bei 40°C (Vakuumgetrocknete Lagertstabilität) und Rekonstitution. Sprühgetrocknet wurden wässrige Lösungen mit a) 9,9% LS55P-Anteil 0,1% IgG-Anteil, b) 9% LS55P-Anteil 1 % lgG-Anteil, c) 2,5% LS55P-Anteil 7,5% IgG-Anteil, d) 1% LS55P-Anteil 9% IgG-Anteil, sowie e) 10% lgG-Anteil, f) 8% LS55P-Anteil, 1 % Isoleucin-Anteil und 1 % lgG-Anteil sowie g) 3% LS55P-Anteil, 6% Citrullin-Anteil und 1 % lgG-Anteil. Die LS55P enthaltenden Pulver zeichnen sich durch einen geringen Anteil an Aggregaten aus.

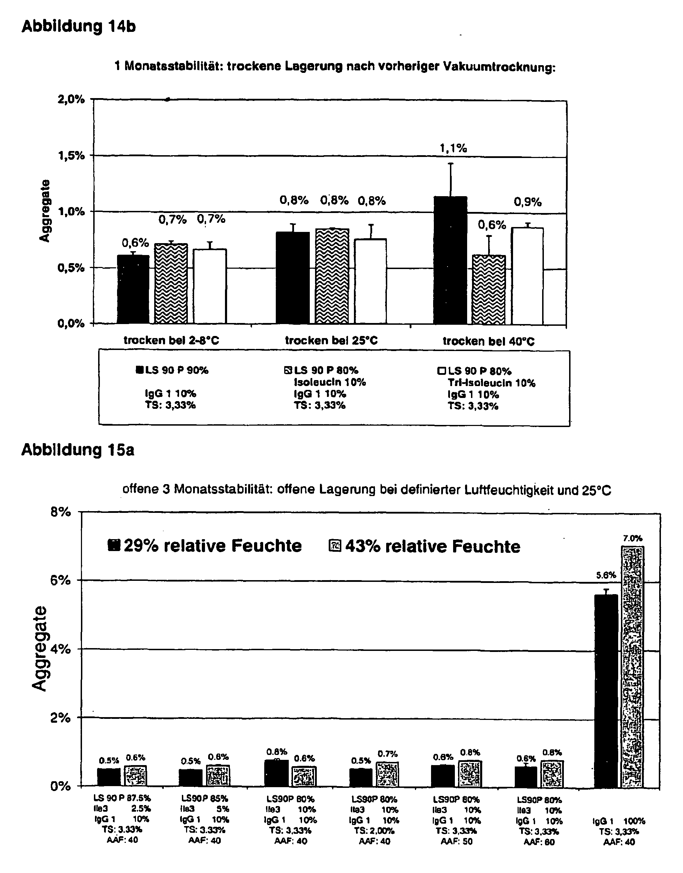

**Abbildung 14a+b** zeigt den Aggregatgehalt nach Sprühtrocknung, Vakuumtrocknung, ein- bzw. dreimonatiger trockener Lagerung bei 2-8°C, 25°C und 40°C (1 bzw. 3 Monatsstabilität). Sprühgetrocknet wurden wässrige Lösungen mit a) 3,00 % LS90P-Anteil und 0,33% IgG-Anteil sowie , b) 2,66 % LS55P-Anteil, 0,33% Isoleucin-Anteil und 0,33% IgG-Anteil, c) 2,66 % LS90P-Anteil, 0,33% Isoleucin-Anteil und 0,33% IgG-Anteil, d) 2,66 % LS55P-Anteil, 0,33% Tri-Isoleucin-Anteil und 0,33% IgG-Anteil, e) 2,66 % LS90P-Anteil, 0,33% Tri-Isoleucin-Anteil und 0,33% IgG-Anteil, sowie f) 3,33 % IgG-Anteil. Sowohl die LS55P als auch die LS90P enthaltenden Pulver zeichnen sich nach drei Monaten Lagerung durch einen besonders geringen Aggregatgehalt aus.

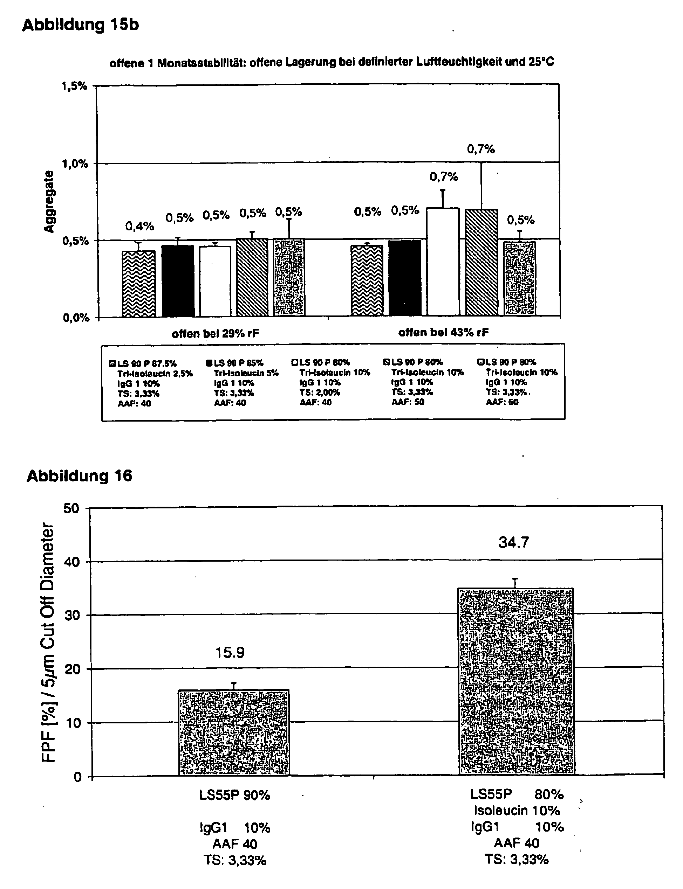

**Abbildung 15a+b** zeigt den Aggregatgehalt nach Sprühtrocknung und offener ein- bzw. dreimonatiger trockener Lagerung bei 29% relativer Luftfeuchtigkeit und 43% relativer Luftfeuchtigkeit bei jeweils 25°C (offene 1 + 3 Monatsstabilität) und Rekonstitution. Sprühgetrocknet wurden wässrige Lösungen mit a) 2,9166% LS90P-Anteil, 0,0833% Tri-Isoleucin-Anteil und 0,33% IgG-Anteil bei einem AAF von 40, b) 2,833% LS90P -Anteil, 0,166% Tri-Isoleucin-Anteil und 0,33% IgG-Anteil bei einem AAF von 40, c) 2,66% LS90P -Anteil, 0,33% Tri-Isoleucin-Anteil und 0,33% IgG-Anteil bei einem AAF von 40, d) 1,60 % LS90P -Anteil, 0,20% Tri-Isoleucin-Anteil und 0,33% IgG-Anteil bei einem AAF von 40, e) 2,66 % LS90P-Anteil, 0,33% Tri-Isoleucin-Anteil und 0,33% IgG-Anteil bei einem AAF von 50, f) 2,66 % LS90P-Anteil, 0,33% Tri-Isoleucin-Anteil und 0,33% 1gG-Anteil bei einem AAF von 60 und g) 3,33% lgG-Anteil bei einem AAF von 40. Die LS90P enthaltenden Pulver zeichnen sich nach drei Monaten Lagerung durch einen besonders geringen Aggregatgehalt aus.

**Abbildung 16** zeigt die Feinpartikelfraktion (FPF) mit einem Cut Off Diameter kleiner 5 µm für verschiedene Pulver. Die Pulver wurden durch Sprühtrocknung wässriger Lösungen hergestellt welche LS55P und IgG1 oder LS55P, Isoleucin und lgG1 enthalten haben. Die Lösungen wurden wie unter BEISPIELE beschrieben hergestellt und versprüht. Isoleucin enthaltendes Pulver hat eine FPF von -35% während Isoleucin freies Pulver lediglich eine FPF von -16 % hat.

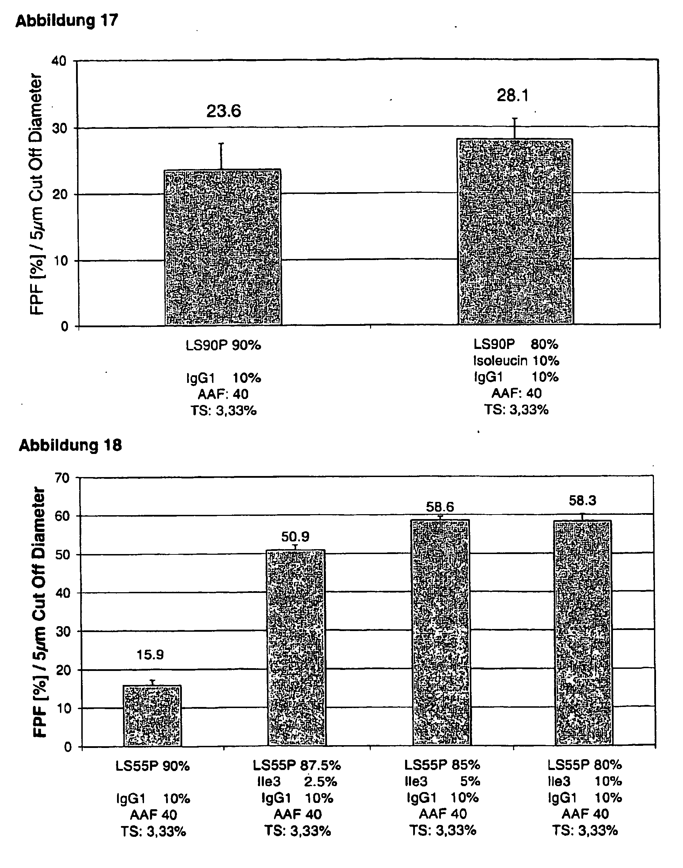

**Abbildung 17** zeigt die Feinpartikelfraktion (FPF) mit einem Cut Off Diameter kleiner 5 *µ*m für verschiedene Pulver. Die Pulver wurden durch Sprühtrocknung wässriger Lösungen hergestellt welche LS90P und lgG1 oder LS90P, Isoleucin und lgG1 enthalten haben. Die Lösungen wurden wie unter BEISPIELE beschrieben hergestellt und versprüht. Isoleucin enthaltendes Pulver hat eine FPF von ~28% während Isoleucin freies Pulver eine FPF von -23 % hat.

**Abbildung 18** zeigt die Feinpartikelfraktion (FPF) mit einem Cut Off Diameter kleiner 5 *µ*m von verschiedenen Pulvern. Die Pulver wurden durch Sprühtrocknung wässriger Lösungen hergestellt welche LS55P und IgG1 oder LS55P, Tri-Isoleucin und IgG1 enthalten haben. Die Lösungen wurden wie unter BEISPIELE beschrieben hergestellt und versprüht. Tri-Isoleucin enthaltende Pulver haben eine FPF von größer 50%, bzw. 58%, während Tri-Isoleucin freies Pulver lediglich eine FPF von ~16% hat.

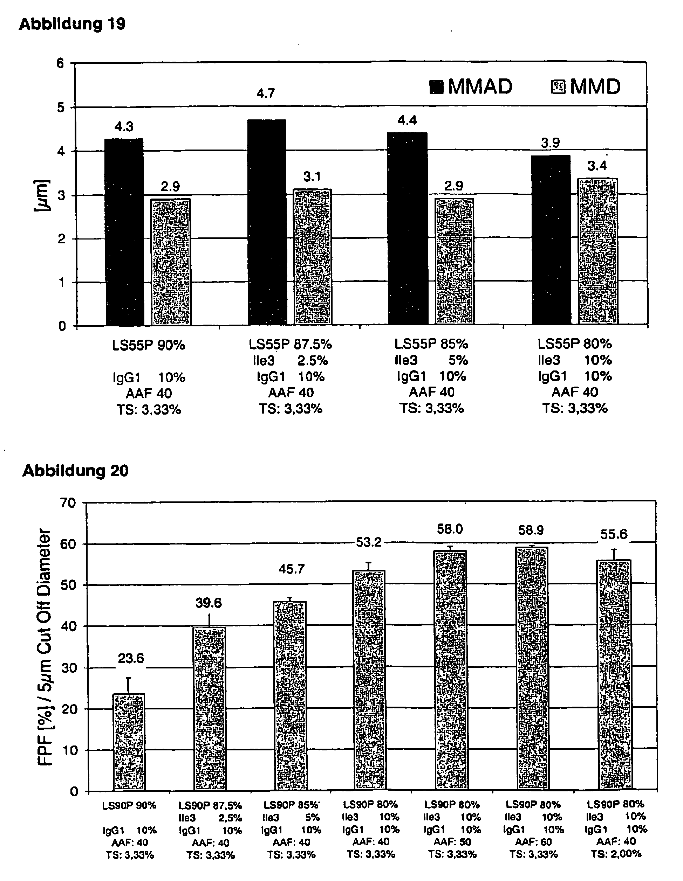

**Abbildung 19** zeigt die Mass Mean Aerodynamic Diameter (MMAD) und Mass Mean Diameter (MMD) verschiedener Pulver. Die Pulver wurden durch Sprühtrocknung wässriger Lösungen hergestellt welche LS55P und IgG1 oder LS55P, Tri-Isoleucin und IgG1 enthalten haben. Die Lösungen wurden wie unter BEISPIELE beschrieben hergestellt und versprüht. Sämtliche Pulver haben einen MMAD von kleiner 5 *µ*m sowie einen MMD kleiner 3,5*µ*m. Das Diagramm zeigt den Einfluss des Tri-Isoleucin-Anteils bei konstanten Gesamtfeststoffkonzentrationen und Sprühparametern auf den MMAD und MMD. Ein 10%iger Tri-Isoleucin-Anteil bezogen auf den Gesamtfeststoffgehalt der Formulierung setzt den MMAD signifikant herab.

**Abbildung 20** zeigt die Feinpartikelfraktion (FPF) mit einem Cut Off Diameter kleiner 5 *µ*m von verschiedenen Pulvern. Die Pulver wurden durch Sprühtrocknung wässriger Lösungen hergestellt welche LS90P und IgG1 oder LS90P, Tri-Isoleucin und IgG1 enthalten haben. Die Lösungen wurden wie unter BEISPIELE beschrieben hergestellt und versprüht. Tri-Isoleucin enthaltende Pulver haben eine FPF von -40% bis -59% während Tri-Isoleucin freies Pulver lediglich eine FPF von ~24% hat.

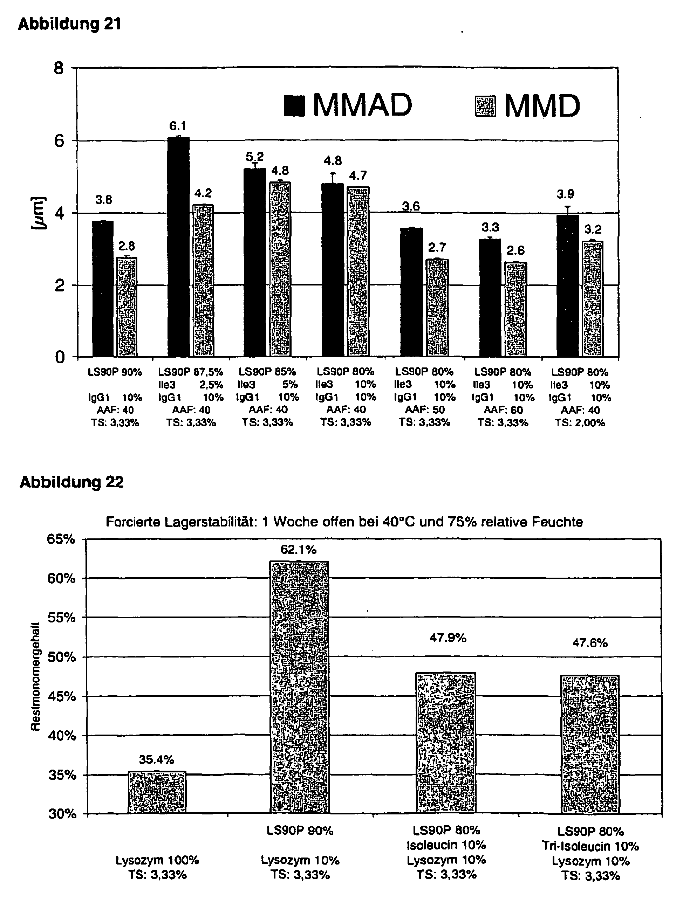

**Abbildung 21** zeigt die Mass Mean Diameter (MMD) und Mass Mean Aerodynamic Diameter (MMAD) verschiedener Pulver. Die Pulver wurden durch Sprühtrocknung wässriger Lösungen hergestellt welche LS90P und IgG1 oder LS90P, Tri-Isoleucin und IgG1 enthalten haben. Die Lösungen wurden wie unter BEISPIELE beschrieben hergestellt und versprüht. Sämtliche Pulver haben einen MMAD von kleiner 6,5 *µ*m sowie einen MMD kleiner 5*µ*m. Das Diagramm zeigt den Einfluss des Tri-Isoleucin-Anteils bei konstanten Gesamtfeststoffkonzentrationen und Sprühparametern auf den MMAD und MMD. Ein 10%iger Tri-Isoleucin-Anteil bezogen auf den Gesamtfeststoffgehalt der Formulierung setzt den MMAD im Vergleich zu 2.5% und. 5%igen Tri-Isoleucin-Anteil signifikant herab. Sowohl ein niedrigerer Feststoffgehalt (z. B. TS: 2%) als auch ein höherer Sprühdruck (AAF 50 oder 60) setzen den MMAD und MMD abermals signifikant herab.

**Abbildung 22** zeigt den Restmonomergehalt nach Sprühtocknung, forcierter Lagerung und Rekonstitution. Versprüht wurden wässrige Lösungen mit a) 3,33% (w/w) Lysozym-Anteil, b) 0,33% (w/w) Lysozym- und 3,0% LS90P-Anteil, c) 0,33% (w/w) Lysozym-, 0,33% (w/w) Isoleucin- und 2,66% (w/w) LS90P-Anteil sowie d) 0,33% (w/w) Lysozym-, 0,33% (w/w) Tri-Isoleucin- und 2,66% (w/w) LS90P-Anteil. Das LS90P entahltende Pulver zeichnet sich durch hohen Restmonomergehalt aus.

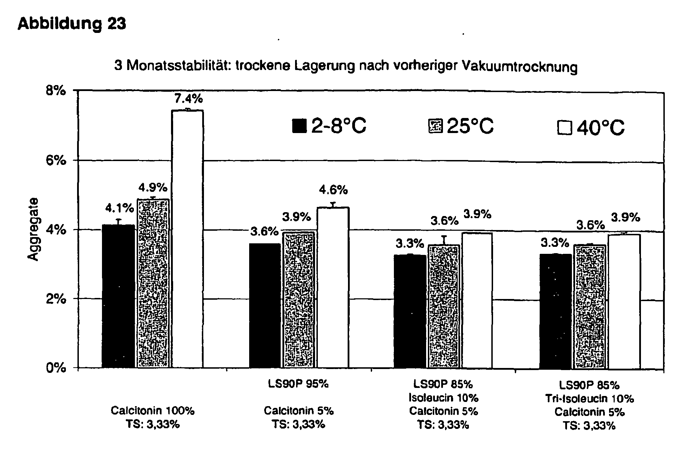

**Abbildung 23** zeigt den Aggregatgehalt nach Sprühtrocknung, Vakuumtrocknung, dreimonatiger trockener Lagerung bei 2-8°C, 25°C und 40°C (3 Monatsstabilität) und Rekonstitution. Versprüht wurden wässrige Lösungen mit a) 3,33% (w/w) Calcitonin-Anteil, b) 0,166% (w/w) Calcitonin- und 3,166% LS90P-Anteil, c) 0.166% (w/w) Calcitonin-, 0,33% (w/w Isoleucin- und 2,833% (w/w) LS90P-Anteil sowie d) 0,166% (w/w) Calcitonin-, 0,33% (w/w) Tri-Isoleucin- und 2,833% (w/w) LS90P-Anteil. Das LS90P entahltende Pulver zeichnet sich durch geringen Aggregatgehalt aus.

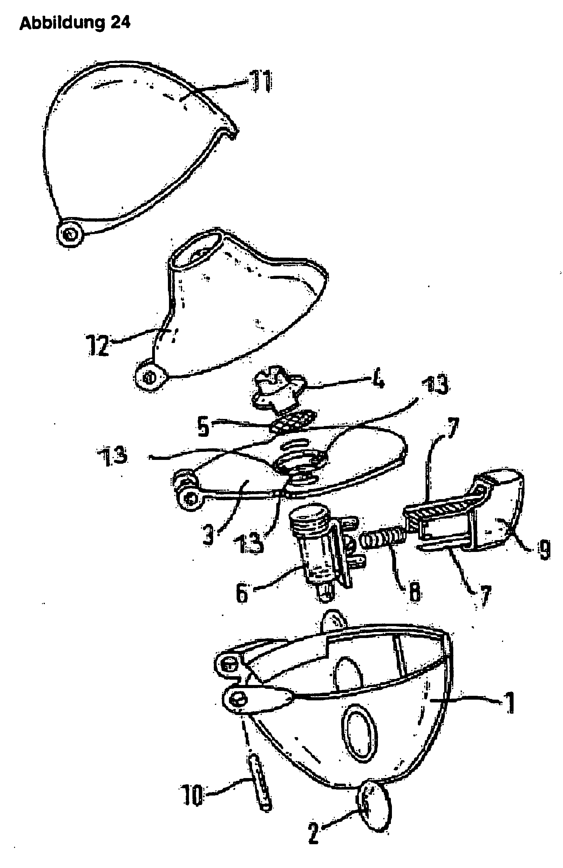

**Abbildung 24** zeigt einen Inhalator zur inhalativen Applikation von trockenen Pulverzubereitungen.

### Detaillierte Beschreibung der Erfindung

### Definitionen

Im Rahmen dieser Erfindungsbeschreibung verwendete Begriffe und Bezeichnungen haben folgende im Anschluss definierte Bedeutungen. Die Gewichts- und Gewichtsprozentangaben beziehen sich, sofern nichts anderes erwähnt wird, jeweils auf die Trockenmasse der Pulver oder den Feststoffgehalt der zu versprühenden Lösungen /Suspensionen. Die allgemeinen Ausführungsfomnen "enthaltend" oder "enthält" schließt die speziellere Ausführungsform "bestehend aus" mit ein. Ferner werden "einzahl" und "Mehrzahl" nicht begrenzend verwendet.

Der Ausdruck "1,4 O-verknüpftes Saccharose-Derivat" meint ein 1,4 O-verknüpftes Saccharose-Derivat ausgewählt aus den Verbindungen: 1,4 O-verknüpfte D-Gal-Saccharose (Lactosucrose), 1,4 O-verknüpfte D-Glu-Saccharose (Glucosyl-Sucrose), oder 1,4 O-verknüpfte Glu-Glu-Saccharose (Maltosyl-Sucrose) jeweils mit der in dieser Patentschrift angegebenen Formel.

Der Ausdruck "Zuckergemisch" oder "Zuckermischung enthaltend zumindest ein 1,4 O-verknüpftes Saccharose-Derivat" meint i) eine 1,4 O-verknüpftes Saccharose-Derivat mit einer der in dieser Patentschrift angegebenen Formel, ii) ein Gemisch aus zumindest zwei unterschiedlichen 1,4 O-verknüpften Saccharose-Derivaten mit jeweils der in dieser Patentschrift angegebenen Formel, vorzugsweise ein Gemisch aus Maltosyl- und Glucosylsucrose, iii) ein Gemisch aus zumindest einem 1,4 O-verknüpften Saccharose-Derivat mit einer der oben angegebenen Formel und weiteren Zuckern, vorzugsweise ein Gemisch aus Lactosucrose, Lactose und Saccharose, oder aus Glucosyl- und/oder Maltosyl-Sucrose, Saccharose, Fructose und Glucose, iv) ein Gemisch aus zumindest 55% (w/w) Lactosucrose, maximal 25 (w/w) Lactose und maximal 10% (w/w) Saccharose v) ein Gemisch aus zumindest 88% (w/w) Lactosucrose, maximal 10% (w/w) Lactose und Saccharose vi) einem Gemisch aus jeweils 25% (w/w) Glucosyl- und/oder Maltosyl-Sucrose, zwischen 48 und 56% (w/w) Saccharose und nicht mehr als 10% (w/w) Glucose und Fructose, vii) ein Gemisch aus jeweils 18% (w/w) Glucosyl- und Maltosyl-Sucrose, zwischen 11 und 15% (w/w) Saccharose und jeweils zwischen 5 und 9% (w/w) Glucose, viii) eine als Nyuka-Oligo® LS40L (kurz LS40L), Nyuka-Oligo® LS55L (kurz LS55L), Nyuka-Oligo® LS55P (kurz LS55P), Nyuka-Oligo® LS-90P (kurz LS90P), Coupling Sugar® oder Coupling Sugar S® bezeichnete Zuckermischung der Firma Hayashibara Shoji, Inc., Japan.

Der Ausdruck "Oligosaccharid" oder "Polysaccharid" meint Mehrfachzucker die zumindest aus drei monomeren Zuckermolekülen aufgebaut sind.

Mit dem Ausdruck "sprühgetrocknete Pulverformulierung" oder "trockene Pulverformulierung" sind Pulverformulierungen gemeint, welche üblicherweise weniger als ca. 10% (w/w) Restfeuchte, vorzugsweise weniger als 7% (w/w) Restfeuchte, besonders bevorzugt weniger als 5% (w/w) Restfeuchte und noch weiter bevorzugt weniger als 3% (w/w) Restfeuchte aufweisen. Die Restfeuchte ist bei konstanten Sprüh-, Vakuum- oder Gefriertrocknungsbedinungen und identischen Hilfsstoffen im Wesentlichen von Art und Anteil des pharmazeutischen Wirkstoffs in der Pulverformulierung abhängig.

Der Begriff "amorph" meint, dass die pulverförmigen Formulierung weniger als 10% kristalline Anteile enthalten, vorzugsweise weniger als 7%, weiter bevorzugt weniger als 5%, insbesondere weniger als 4, 3, 2, oder 1%.

Der Begriff "inhalierbar" meint, dass die Pulver zur pulmonalen Applikation geeignet sind. Inhalierbare Pulver lassen sich mit Hilfe eines Inhalationsgerätes dispergieren und inhalieren, so dass die Partikel die Lunge erreichen und gegebenenfalls über die Alveolen systemische Wirkung entfalten können. Inhalierbare Partikel weisen beispielsweise eine mittlere Teilchengröße zwischen 0,4-10 *µ*m (MMD = Mass median diameter), meistens zwischen 0,5-5 *µ*m, bevorzugt zwischen 1-3 *µ*m und/oder einen mittleren aerodynamischen Teilchendurchmesser (MMAD = Mass median aerodynamic diameter) zwischen 0,5-10 µm, vorzugsweise zwischen 0,5-7,5 µm, weiter bevorzugt zwischen 0,5-5,5 µm, noch weiter bevorzugt 1-5 µm und in besonders bevorzugter Weise zwischen 1-4,5 *µ*m auf.

Der Ausdruck "Mass Median Diameter" oder "MMD" ist eine Messgröße für die durchschnittliche Partikelgrößenverteilung, da die Pulver der Erfindung generell polydispers sind. Die Ergebnisse werden ausgedrückt als Durchmesser der Volumensummenverteilung bei 50% Durchgangssumme. Die MMD-Werte lassen sich beispielhaft mittels Laserdiffraktometrie (vgl. hierzu: Kapitel BEISPIELE, Methode) bestimmen, wobei natürlich auch jede andere übliche Methode verwendet werden kann (z.B. Elektronenmikroskopie, Zentrifugalsedimentation).

Der Begriff "mittlerer aerodynamischer Teilchendurchmesser" (= mass median aerodynamic diameter (MMAD)) gibt die aerodynamische Teilchengröße an, bei der normalerweise 50% der Teilchen des Pulvers einen kleineren aerodynamischen Durchmesser aufweisen. Als Referenzmethode zur Bestimmung des MMAD dient im Zweifel die in dieser Patentschrift angegebene Methode (vgl. hierzu: Kapitel BEISPIELE, Methode).

Der Begriff *"Feinpartikelfraktion"* (FPF) beschreibt den inhalierbaren Teil eines Pulvers bestehend aus Teilchen mit einer Teilchengröße von ≤ 5 *µ*m MMAD. Bei gut inhalierbaren Pulvern beträgt die FPF mehr als 20%, vorzugsweise mehr als 30%, besonders bevorzugt mehr als 40%, noch weiter bevorzugt mehr als 50%, noch weiter bevorzugt mehr als 55%. Der in diesem Zusammenhang verwendete Begriff "Cut Off Diamenter" gibt an, welche Partikel bei der Bestimmung der FPF berücksichtigt werden. Eine FPF von 30% bei einem Cut Off Diameter von 5 µm (FPF ₅) meint, dass zumindest 30% aller Partikel im Pulver einen mittleren aerodynamischen Teilchendurchmesser von kleiner 5 *µ*m aufweisen.

Der Begriff "Sprühlösung" meint wässrige Lösungen oder Suspensionen, in denen der pharmazeutische Wirkstoff zusammen mit zumindest einem Hilfsstoff gelöst / suspendiert ist.

Der Begriff " time of flight" ist die Bezeichnung für eine Standardmessmethode wie im Kapitel BEISPIELE näher beschrieben. Bei einer time of flight Messung werden simultan der MMAD und FPF bestimmt (vgl. hierzu: Kapitel BEISPIELE, Methode).

Der Begriff "pharmazeutisch akzeptable Hilfsstoffe", "Trägermaterial" oder "Matrizes" bezieht sich auf Hilfsstoffe, die optional im Rahmen der Erfindung in der Formulierung enthalten sein können. Die Hilfsstoffe können beispielsweise pulmonal appliziert werden ohne hierbei signifikant ungünstige toxikologische Effekte auf den Probanden oder die Probandenlunge zu haben.

Der Ausdruck "pharmazeutisch akzeptable Salze" umfasst beispielsweise folgende Salze, ist aber nicht begrenzt auf diese: Salze aus anorganischen Säuren, wie Chlorid, Sulfat, Phosphat, Diphosphat, Bromid und Nitrat Salze. Des weiteren Salze aus organischen Säuren, wie Malat, Maleat, Fumarat, Tartrat, Succinat, Ethylsuccinat, Citrat, Acetat, Lactat, Methansulfonat, Benzoat, Ascorbat, Para-Toluolsulfonat, Palmoat, Salicylat und Stearat, ebenso wie Estolat, Gluceptat und Lactobionat Salze.

Der Begriff "pharmazeutisch akzeptable Kationen" umfasst, ohne auf diese begrenzt zu sein, beispielsweise Lithium, Natrium, Kalium, Calcium, Aluminium sowie Ammonium (inklusive substituiertem Ammonium).

Unter einem "pharmazeutischen Wirkstoff" ist eine Substanz, ein Arzneimittel, eine Zusammensetzung oder eine Kombination hieraus zu verstehen, die einen pharmakologischen, zumeist positiven, Effekt auf einen Organismus, ein Organ, und/oder eine Zelle ausübt, wenn der Wirkstoff mit dem Organismus, Organ oder der Zelle in Kontakt gebracht wird. Eingebracht in einen Patienten, kann der Effekt lokal oder systemisch sein.

Der Begriff "biologisches Makromolekül" meint Peptide, Proteine, Fette, Fettsäuren, oder auch Nukleinsäuren.

Mit dem Begriff "Peptid" oder "Polypeptid" sind Polymere von Aminosäuren bestehend aus zwei bis hundert Aminosäureresten gemeint. Der Begriff Peptid oder Polypeptid wird als pseudonym verwendet und umfasst sowohl Homo- als auch Heteropeptide, also Polymere von Aminosäuren bestehend aus identischen oder verschiedenen Aminosäureresten. Ein "Di-Peptid" ist somit aus zwei peptidisch verknüpften Aminosäuren, ein "Tri-Peptid" aus drei peptidisch verknüpften Aminosäuren aufgebaut. Der hier verwendete Begriff "Protein" meint Polymere von Aminosäuren mit mehr als 100 Aminosäureresten.

Der Begriff "Analoge" bezeichnet Peptide/Proteine in denen einzelnen oder mehrere Aminosäuren substituiert, eliminiert (z.B. Fragmente), addiert (z.B. Derivate mit einer C- oder N-terminalen Verlängerung) oder anderweitig von der nativen (Wildtyp) Sequenz modifiziert wurden, Ebenso ist auch eine Derivatisierung des nativen Proteins, z.B. durch Zucker, Polyethylenglykol o. a. möglich. Analoge haben eine Bioaktivität von zumindest 10, 20, 30 oder 40%, bevorzugt von zumindest 50, 60 oder 70% und besonders bevorzugt von zumindest 80, 90, 95, 100% oder mehr als 100% Bioaktivität des nativen, nicht synthetischen Proteins.

Der Ausdruck "Aminosäure" meint Verbindungen, welche mindestens eine Amino- und mindestens eine Carboxylgruppe enthalten. Obwohl die Aminogruppe üblicherweise in α-Position zur Carboxylgruppe steht ist auch jede andere Anordnung im Molekül denkbar. Die Aminoäure kann auch weitere funktionelle Gruppen, wie z.B. Amino-, Carboxamid-, Carboxyl-, Imidazol-, Thiogruppen und andere Gruppen, enthalten. Verwendung finden Aminosäuren natürlichen oder synthetischen Ursprungs, razemisch oder optisch aktiv (D-, oder L-) inklusive verschiedener stereoisomerer Verhältnisse. Beispielsweise umfasst der Begriff Isoleucin sowohl D- Isoleucin, L- Isoleucin, razemisches Isoleucin und verschiedene Verhältnisse der beiden Enantiomere.

Der Ausdruck "pure oder reine Proteinformulierung" meint sprühgetrocknete Pulver bestehend aus einem oder mehreren Proteinen und optional einem geeigneten Puffer (typischerweise von 0 bis 15% (w/w) bezogen auf das Gewicht des trockenen Pulvers). Das Pulver enthält grundsätzlich keine weiteren Hilfsstoffe, d.h. der Gehalt an eventuellen weiteren Hilfsstoffen ist weniger als 1% (w/w) bezogen auf das Gewicht des trockenen Pulvers.

Eine "oberflächenaktive" Substanz ist in der Lage die Oberflächenspannung der Lösung, in welcher sie gelöst ist, herabzusetzen. Die Oberflächenaktivität wird beispielsweise durch die Tensiometer - Methode nach Lecomte du Noüy (Bauer, Frömming, Führer, 6. Auflage) gemessen.

Der Ausdruck "Oligosaccharid" oder "Polysaccharid" meint Mehrfachzucker die zumindest aus drei monomeren Zuckermolekülen aufgebaut sind.

### Erfindungsgemäße Pulver

Die vorliegende Erfindung betrifft Pulver, vorzugsweise sprühgetrocknete Pulver, enthaltend einen pharmazeutischen Wirkstoff und eine Hilfsstoffkombination enthaltend zumindest ein 1,4 O-verknüpftes Saccharose-Derivat ausgewählt aus den Verbindungen: 1,4 O-verknüpfte D-Gal-Saccharose (Lactosucrose), 1,4 O-verknüpfte D-Glu-Saccharose (Glucosyl-Sucrose), oder 1,4 O-verknüpfte Glu-Glu-Saccharose (Maltosyl-Sucrose) in Kombination mit zumindest einem weiteren Hilfsstoff. Bei dem weiteren Hilfsstoff handelt es sich vorzugsweise um eine Aminosäure, ein Peptid und/oder um ein Mono-, Di- und/oder Oligosaccharid, wobei es sich bei den Oligosaccharid auch um ein zweites 1,4 O-verknüpftes Saccharose-Derivat handeln kann, sofern dieses von dem ersten verschieden ist.

Nach einer besonderen Ausführungsform der Erfindung enthalten die entsprechenden Pulver als Hilfsstoffkombination neben dem 1,4 O-verknüpften Saccharose-Derivat zusätzlich ein oder mehrere Mono-, Di- und/oder Oligosaccharide, wobei die zusätzliche Verwendung von Mono- und/oder Di-Sacchariden bevorzugt ist. Beispiele für Einfachzucker sind Fructose, Maltose, Galactose, Glucose, D-Mannose, Sorbose und der gleichen. Geeignete Zweifachzucker im Sinne der Erfindung sind beispielsweise Lactose, Saccharose, Trehalose, Cellobiose, und der gleichen. Als Mehrfachzucker oder Oligo bzw. Polysaccharide eignen sich insbesondere Raffinose, Melezitose, Dextrin, Stärke und der gleichen. Die Erfindung umfasst folglich auch entsprechende Pulver mit Lactosucrose, Lactose und Saccharose, wobei der Anteil an Lactosucrose in Bezug auf den gesamten Zuckeranteil im Pulver ≥ 40% (w/w), vorzugsweise ≥ 55% (w/w), sowie auch ≥ 88% (w/w) beträgt. Nach einer bevorzugten Ausführungsform enthalten die erfindungsgemäßen Pulver neben dem pharmazeutischen Wirkstoff eine als Nyuka-Oligo^{®} LS55P, oder kurz LS55P, bezeichnete Zuckermischung der Firma Hayashibara Shoji, Inc., Japan, die zumindest 55% Lactosucrose, maximal 25% (w/w) Lactose und maximal 10% (w/w) Saccharose enthält. Nach einer weiterbevorzugten Ausführungsform enthalten die erfindungsgemäßen Pulver neben dem pharmazeutischen Wirkstoff eine als Nyuka-Oligo^{®} LS90P, oder kurz LS90P, bezeichnete Zuckermischung der Firma Hayashibara Shoji, Inc., Japan, die zumindest 88% Lactosucrose und maximal 10% (w/w) Lactose und Saccharose enthält.

Darüber hinaus haben sich auch Pulver aus einer Kombination aus Glucosyl- und Maltosyl-Sucrose als erfindungsgemäß erwiesen, vorzugsweise wiederum in Kombination mit weiteren Mono-, Di- und /oder Polysacchariden. Folglich umfasst die vorliegende Erfindung auch entsprechende Pulver, die eine Hilfsstoffkombination aus Glucosyl- und Maltosyl-Sucrose, Saccharose, Glucose und/oder Fructose enthalten, wobei der Anteil an Glucosyl- und Maltosyl-Sucrose in Bezug auf den gesamten Zuckeranteil im Pulver vorzugsweise 25% (w/w) oder mehr beträgt. Nach einer weiter bevorzugten Ausführungsform beträgt der jeweilige Anteil an Glucosyl- und Maltosyl-Sucrose zumindest 18% (w/w) am Gesamtzuckeranteil des Pulvers. Nach einer weiter bevorzugten Ausführungsform enthalten die erfindungsgemäßen Pulver neben dem pharmazeutischen Wirkstoff eine als Coupling Sugar® bezeichnete Zuckermischung der Firma Hyashibara Hayashibara Shoji, Inc., Japan, die zumindest jeweils 18% (w/w) Glucosyl- und Maltosyl-Sucrose, zwischen 11 und 15% (w/w) Saccharose und jeweils zwischen 5 und 9% (w/w) Glucose und Fructose enthält. Ferner betrifft die vorliegende Erfindung auch solche Pulver, die neben dem pharmazeutischen Wirkstoff eine als Coupling Sugar S® bezeichnete Zuckermischung der Firma Hyashibara Hayashibara Shoji, Inc., Japan, enthalten, die zumindest 25% (w/w) Glucosyl- und/oder Maltosyl-Sucrose, zwischen 48 und 56 % (w/w) Saccharose und nicht mehr als 10% (w/w) Glucose und Fructose enthält.

Ferner haben sich Pulver als erfindungsgemäß erwiesen, die als Hilfsstoffkombination neben mindestens einem 1,4 O-verknüpften Saccharose-Derivat oder einer Zuckermischung, enthaltend mindestens ein 1,4 O-verknüpfte Saccharose-Derivat als weitere Hilfsstoffe Aminosäuren, Peptide, Alkohole, Polyole, nicht-biologische und/oder biologische Polymere, enthalten. Weitere, im Stand der Technik bekannte Hilfsstoffe die in Kombination mit mindestens einem 1,4 O-verknüpften Saccharose-Derivat oder einer Zuckermischung, enthaltend mindestens ein 1,4 O-verknüpfte Saccharose-Derivat, erfindungsgemäß eingesetzt werden können sind beispielsweise Lipide, Fettsäuren Fettsäureester, Steroide (z.B. Cholesterol) oder Chelatbildner (z.B. EDTA) sowie diverse Kationen. Besonders bevorzugt sind Hilfsstoffe mit einer hohen Glasübergangstemperatur, beispielsweise von größer 40°C, vorzugsweise von größer 45°C, oder von größer 55°C. Eine Auflistung geeigneter Hilfsstoffe findet sich beispielsweise in Kippe (Eds.), "Handbook of Pharmaceutical Excipients" 3rd Ed., 2000.

Geeignete proteinhaltige Hilfsstoffe sind beispielsweise Albumin (humanen order rekombinanten Ursprungs), Gelatine, Kasein, Hämoglobin und der gleichen. Als Zuckeralkohole kommen neben Mannitol, Xylitol, Maltitol, Galactitol, Arabinitol, Adonitol, Laktitol, Sorbitol (Glucitol), Pyranosylsorbitol, Inositol, Myoinositol und der gleichen als Hilfsstoffe in Betracht. Geeignete Aminosäuren umfassen beispielsweise Alanin, Glycin, Arginin, Histidin, Glutamat, Asparagin, Cystein, Leucin, Lysin, Isoleucin, Valin, Tryptophan, Methionin, Phenylalanin, Tyrosin, Citrullin, L-Aspartyl-L-Phenylalanin-Methylester (= Aspartam), Trimethylammonioacetat (= Betain) und der gleichen. Vorzugsweise werden solche Aminosäuren verwendet, die als Puffer (z.B. Glycin oder Histidin) und/oder als dispergierendes Agens wirken. Unter die letzten Gruppen fallen insbesondere vorwiegend hydrophobe Aminosäuren, wie z.B. Leucin, Valin, Isoleucin, Tryptophan, Alanin, Methionin, Phenylalanin, Tyrosin, Histidin, oder Prolin. Im Rahmen der vorliegenden Erfindung hat sich insbesondere die Verwendung von Isoleucin neben dem 1,4 O-verknüpften Saccharose-Derivat oder einer Zuckermischung enthaltend zumindest ein 1,4 O-verknüpften Saccharose-Derivat, als vorteilhaft erwiesen, vorzugsweise in einer Konzentration von 1 bis 19,99% (w/w), besonders bevorzugt von 5 bis 19,99% (w/w), noch weiter bevorzugt von 10 bis 19,99% (w/w).

Besonders vorteilhaft ist auch die Verwendung von Di-, Tri-, Oligo-, oder Polypeptiden als weiteren Hilfsstoff, die eine oder mehrere dieser vornehmlich hydrophoben Aminosäurereste beinhalten. Besonders bevorzugt sind Peptide mit bis zu 20 Aminosäuren, weiter bvorzugt mit bis zu 15 Aminosäuren, noch weiter bevorzugt mit bis zu 12 Aminosäuren, noch weiter bevorzugt mit bis zu 11 Aminosäuren, noch weiter bevorzugt mit bis zu 10 Aminosäuren, noch weiter bevorzugt mit bis zu 9 Aminosäuren noch weiter bevorzugt mit bis zu 8 Aminosäuren, noch weiter bevorzugt mit bis zu 7 Aminosäuren, noch weiter bevorzugt mit bis zu 7, 6, 5, 4 oder 3 Aminsäuren. Die zur Stabilisierung verwendeten Peptide entsprechen hierbei nicht gleichzeitig dem pharmazeutischen Wirkstoff.

Geeignete Beispiele für Tri-Peptide umfassen beispielsweise ein oder mehrere der nachfolgenden Tri-Peptide: Leu-Leu-Gly, Leu-Leu-Ala, Leu-Leu-Val, Leu-Leu-Leu, Leu-Leu-Met, Leu-Leu-Pro, Leu-Leu-Phe, Leu-Leir-Trp, Leu-Leu-Ser, Leu-Leu-Thr, Leu-Leu-Cys, Leu-Leu-Tyr, Leu-Leu-Asp, Leu-Leu-Glu, Leu-Leu-Lys, Leu-Leu-Arg, Leu-Leu-His, Leu-Gly-Leu, Leu-Ala-Leu, Leu-Val-Leu, Leu-Met-Leu, Leu-Pro-Leu, Leu-Phe-Leu, Leu-Trp-Leu, Leu-Ser-Leu, Leu-Thr-Leu, Leu-Cys-Leu, Leu-Try-Leu, Leu-Asp-Leu, Leu-Glu-Leu, Leu-Lys-Leu, Leu-Arg-Leu und Leu-His-Leu. Als besonders vorteilhaft hat sich die Verwendung von Tri-Peptiden der allgemeinen Formeln: Ile-X-X; X-Ile-X; X-X-Ile erwiesen, wobei X eine der folgenden Aminosäuren sein kann: Alanin, Glycin, Arginin, Histidin, Glutaminsäure, Glutamin, Asparagin, Asparaginsäure, Cystein, Leucin, Lysin, Isoleucin (IIe), Valin, Tryptophan, Methionin, Phenylalanin, Prolin, Serin, Threonin, Tyrosin, L-Aspartyl-L-phenylalanin-methylester (= Aspartam), Trimethylammonio-acetat. Besonders bevorzugt sind entsprechende Tri-Peptide der Formel (IIe)₂-X, beispielsweise IIe-IIe-X, IIe-X-IIe, oder X-IIe-IIe, wobei X wiederum eine der oben aufgeführten Aminosäuren sein kann. Hierunter fallen beispielsweise die Tri-Peptide: Ile-Ile-Gly, Ile-Ile-Ala, Ile-Ile-Val, Ile-Ile-Ile, Ile-Ile-Met, Ile-Ile-Pro, Ile-Ile-Phe, Ile-Ile-Trp, Ile-Ile-Ser, Ile-Ile-Thr, Ile-Ile-Cys, Ile-Ile-Tyr, Ile-Ile-Asp, Ile-Ile-Glu, Ile-Ile-Lys, Ile-Ile-Arg, Ile-Ile-His, Ile-Gly-Ile, Ile-Ala-Ile, Ile-Val-Ile, Ile-Met-Ile, Ile-Pro-Ile, Ile-Phe-Ile, Ile-Trp-Ile, Ile-Ser-Ile, Ile-Thr-Ile, Ile-Cys-Ile, Ile-Try-Ile, Ile-Asp-Ile, Ile-Glu-Ile, Ile-Lys-Ile, Ile-Arg-Ile, Ile-His-Ile. Besonders vorteilhaft ist die Verwendung von Ile-Ile-Ile.

Geeignete Polymere umfassen beispielsweise die oben bereits als Hilfsstoffe erwähnte Polyvinylpyrrolidone, derivatisierte Zellulosen, wie z.B. Hydroxymethyl-, Hydroxyethyl-, oder Hydroxypropyl-ethylzellulose, polymere Zucker wie z.B. Fiscoll, Särke wie z.B. Hydroxyethyl- oder Hydroxypropylstärke, Dextrine wie z.B. Cyclodextrine (2-Hydroxypropyl-β-cyclodextrin, Sulfobutylether-β-cyclodextrin), Polyethylene, Glykole und/oder Pektine.

Bei den Salzen handelt es sich beispielsweise um anorganische Salze wie Chloride, Sulfate, Phosphate, Di-Phosphate, Hydrobromide und/oder Nitrat-Salze. Ferner können die erfindungsgemäßen Pulver auch organische Salze beinhalten, wie z.B. Malate, Maleate, Fumarate, Tartrate, Succinate, Ethylsuccinate, Citrate, Acetate, Lactate, Methansulfonate, Benzoate, Ascorbate, Paratoluensulfonate, Palmoate, Salicylate, Stearate, Estolate, Gluceptate oder Lactobionat-Salze. Gleichzeitig können entsprechende Salze pharmazeutisch akzeptable Kationen, wie beispielsweise Natrium, Kalium, Calzium, Aluminium, Lithium oder Ammonium enthalten. Besonders bevorzugt ist die Verwendung von entsprechenden Kationen in Verbindung mit der Stabilisierung von Proteinen. Demzufolge betrifft die vorliegende Erfindung nach einer weiteren Ausführungsform sprühgetrocknete Pulver, die neben dem 1,4 O-verknüpften Saccharose-Derivat oder einer Zuckermischung enthaltend zumindest ein 1,4 O-verknüpften Saccharose-Derivat und dem pharmazeutischen Wirkstoff ein pharmazeutisch verträgliches Salz enthalten.

Bei den erfindungsgemäßen Pulvern handelt es sich beispielsweise um sprühgetrocknete oder gefriergetrocknete Pulver, wobei sprühgetrocknete Pulver die bevorzugte Ausführungsform darstellen.

Als besonders vorteilhaft haben sich Pulver, vorzugsweise gefrier- oder sprühgetrocknete Pulver erwiesen, die einen pharmazeutischen Wirkstoff und eine der oben angegebenen Hilfsstoffkombination enthalten, die zumindest ein 1,4 O-verknüpfte(s) Saccharose-Derivat in Kombination mit zumindest einem weiteren Hilfsstoff enthält, wobei der Anteil an der Hilfsstoffkombination in Bezug auf die Trockenmasse des Pulvers zwischen 25 und 99,99% (w/w), vorzugsweise zwischen 40 und 99% (w/w), weiter bevorzugt zwischen 60 und 99% (w/w) und noch weiter bevorzugt zwischen 60 und 90% (w/w), beispielsweise 25, 25,1, 25,2, 25,3, ... 25,7, 25,8, 25,9 etc.; 26, 27, 28, 29, 30 etc.; 31,32,33,... 38, 39, 40 etc.; 41, 42, 43, ... 48, 49, 50 etc. 51, 52, 53, ... 58, 59, 60 etc.; 61, 62, 63, ... 68, 69, 70 etc.; 71, 72, 73, ... 78 ,79, 80 etc.; 81, 82, 83, ... 88 ,89, 90 etc.; 91, 92, 93, ... 98 etc, 99, 99,1, 99,2, 99,3, ... 99,8 ,99,9, etc.; 99,91, 99,92, 99,93, ... 99,98, 99,99% (w/w) beträgt. Im Zusammenhang mit der Verwendung von LS55P hat sich ein Anteil von 80 - 90% (w/w) als besonders vorteilhaft erwiesen. Insgesamt sollte der Anteil an der Hilfsstoffkombination aus mindestens einem 1,4 O-verknüpften Saccharose-Derivat und einem weiteren Hilfsstoff so gewählt werden, dass das Pulver zumindest teilamorph, vorzugsweise vollständig amorph ist.

Der Anteil an pharmazeutischem Wirkstoff an der Trockenmasse der erfindungsgemäßen Pulver beträgt in der Regel zwischen 0,01 und 75% (w/w), vorzugsweise zwischen 0,01 und 50% (w/w), weiter bevorzugt zwischen 0,33 und 50% (w/w), noch weiter bevorzugt zwischen 0,33 und 40% (w/w). Nach einer weiter bevorzugten Ausführungsform beträgt der Anteil des pharmazeutischen Wirkstoffs am Feststoffgehalt des erfindungsgemäßen Pulvers zwischen 0,33 und 35% (w/w), vorzugsweise zwischen 0,33 und 30% (w/w), weiter bevorzugt zwischen 0,33 und 25% (w/w) und noch weiter bevorzugt zwischen 0,33 und 10% (w/w). Der Anteil beträgt somit beispielsweise 0,01, 0,02, 0,03 ... 0,08, 0,09, etc.; 0,1, 0,2 0,3, ... 0,8, 0,9 etc.; 1, 2, 3, ... 8 ,9, 10 etc.; 11, 12, 13, ... 18, 19, 20 etc.; 21, 22, 23, ... 28 ,29, 30 etc.; 31, 32, 33, ... 38 ,39, 40 etc.; 41, 42, 43, ... 48 ,49, 50 etc.; 51, 52, 53, ... 58, 59, 60 etc.; 61, 62, 63, ... 68, 69, 70 etc.; 71, 72, 73, 74, 74,1, 74,2, 74,3, ... 74,8 ,74,9, etc.; 74,91, 74,92, 74,93, ... 74,98, 74,99, 75% (w/w).

Erfindungsgemäß sind demnach Pulver mit einem Verhältnis von Hilfsstoffkombination enthaltend mindestens ein 1,4 O-verknüpften Saccharose-Derivat und einen weiteren Hilfsstoff zu pharmazeutischen Wirkstoff von beispielsweise 25/75, 26/74, 27/73, 28/72, 29/71, 30/70, 31/69, 32/68, 33/67, 34/66, 35/65, 36/64, 37/63, 38/62, 39/61, 40/60, 41/59, 42/58, 43/57, 44/56, 45/55, 46/54, 47/53, 48/52, 49/51, 50/50, 51/49, 52/48, 53/47, 54/46, 55/45, 56/44, 57/43, 58/42, 59/41, 60/40, 61/39, 62/38, 63/37, 64/36, 65/35, 66/34, 67/33, 68/32, 69/31, 70/30, 71/29, 72/28, 73/27, 74/26, 75/25, 76/24, 77/23, 78/22, 79/21, 80/20, 81/19, 82/18, 83/17, 84/16, 85/15, 86/14, 87/13, 88/12, 89/11, 90/10, 91/9, 92/8, 93/7, 94/6, 95/5, 96/4, 97/3, 98/2, 99/1, 99,1/0,9, 99,2/0,8, 99,3/0,7, 99,4/0,6, 99,5/0,5, 99,6/0,4, 99,66/0,33, 99,7/0,3, 99,8/0,2, 99,9/0,1, 99,99/0,01 (w/w). Der Anteil an der Hilfsstoffkombination enthaltend zumindest ein 1,4 O-verknüpftes Saccharose-Derivat und einen weiteren Hilfsstoff beträgt vorzugsweise einen der Werte zwischen 80 und 90% (w/w) in Bezug auf die Trockenmasse des Pulvers.

Pharmazeutische Wirkstoffe im Sinne der Erfindung sind, neben denen, die unter die allgemeine Definition fallen, unter anderem Antibiotika, anti-virale Wirkstoffe, Anepileptika, Schmerzmittel (analgesics), anti-inflammatorische Wirkstoffe oder Bronchodilatoren. Ferner zählen hierzu Wirkstoffe die beispielsweise auf das periphere Nervensystem, auf adrenergische Rezeptoren, cholinergische Rezeptoren, die skeletale Muskulatur, das cardiovaskuläre System, die glatte Muskulatur, das Blut zirkulierende System, auf synaptische Stellen, neuroeffektorische Verbindungsstellen, das endokrine System, das Immunsystem, das reproduktive System, das skeletale System, die autakoiden Systeme, die alimentarischen und excretorischen Systeme, das Histaminsystem und das zentrale Nervensystem wirken. Geeignete Wirkstoffe umfassen ferner beispielsweise Hypnotika und Sedativa, psychische Energizer, Tranquilizer, anti-Convulsanten, Muskelrelaxanten, anti-parkinson Wirkstoffe, Schmerzmittel, anti-inflammatorische Wirkstoffe, Muskelkontraktanten, anti-mikrobielle Wirkstoffe, hormonale Wirkstoffe, wie beispielsweise Kontrazeptiva, Sympathomimetika, Diuretika, Fettstoffwechsel regulierende Wirkstoffe, anti-androgenische Wirkstoffe, Antiparasitika, Neoplastika, Antineoplastika und Hypoglycemika.

Ferner sind von dem Begriff pharmazeutischer Wirkstoff beispielhaft solche Wirkstoffe umfasst, die auf das respiratorische System, beispielsweise gegen eine der folgenden Krankenheiten wirken: Asthma, chronische obstruktive pulmonarische Erkrankungen (COPD), emphysemische chronische Bronchitis, bronchopulmonarische Dysplasie (BPD), neonatales respiratorisches Distress Syndrom (RDS), Bronchiolitis, Krupp, post-extubation Stridor, pulmonarische Fibrose, Pneumonie oder cystische Fibrose (CF).

Repräsentative Beispiele für Bronchodilatoren umfassen neben anderen beta-Agonisten, Anticholinergika oder Methylxanthine. Beispiele für anti-inflammatorische Wirkstoffe sind Steroide, Cromolyn, Nedokromil und leukotriene Inhibitoren. Beispiele für Steroide umfassen Beclomethason, Betamethason, Biclomethason, Dexamethason, Triamcinolon, Budesonid, Butixocort, Ciclesonid, Flutikason, Flunisolid, Icomethason, Mornetason, Tixocortol, und loteprednol. Weitere Beispiele sind Budesonid, Fluticason propionate, Beclomethason dipropionat, Fometerol und Triamcinolon acetonid.

Beispiele für anti-mikrobiell wirkende Wirkstoffe sind Erythromycin, Oleandomycin, Troleandomycin, Roxithromycin, Clarithromycin, Davercin, Azithromycin, Flurithromycin, Dirithromycin, Josamycin, Spiromycin, Midecamycin, Leucomycin, Miocamycin, Rokitamycin, Andazithromycin und Swinolide A; Fluoroquinolone beispielsweise Ciprofloxacin, Ofloxacin, Levofloxacin, Trovafloxacin, Alatrofloxacin, Moxifloxicin, Norfloxacin, Eoxacin, Grepafloxacin, Gatifloxacin, Lomefloxacin, Sparfloxacin, Temafloxacin, Pefloxacin, Amifloxacin, Fleroxacin, Tosufloxacin, Prulifloxacin, Irloxacin, Pazufloxacin, Clinafloxacin und Sitafloxacin; Aminoglykoside wie zum Beispiel Gentamicin, Netilmicin, Paramecin, Tobramycin, Amikacin, Kanamycin, Neomycin; Streptomycin, Vancomycin, Teicoplanin, Rampolanin, Mideplanin, Colistin, Daptomycin, Gramicidin, Colistimethate; Polymixine wie zum Beispiel Polymixin B, Capreomycin, Bacitracin, Peneme, Penicilline einschließlich penicillinase-sensitive Wirkstoffe wie Penicillin G, Penicillin V, penicillinase-resistente Wirkstoffe wie Methicillin, Oxacillin, Cloxacillin, Dicloxacillin, Floxacillin, Nafcillin; aktive Wirkstoffe gegen Gram-negative Bakterien wie Ampicillin, Amoxicillin, Hetacillin, Cillin, und Galampicillin; antipseudomonale Penicilline wie Carbenicillin, Ticarcillin, Azlocillin, Mezlocillin, Andpiperacillin; Cephalosporine wie Cefpodoxim, Cefprozil, Ceftbuten, Ceftizoxime, Ceftriaxon, Cephalothin, Cephapirin, Cephalexin, Cephradrin, Cefoxitin, Cefamandol, Cefazolin, Cephaloridin, Cefaclor cefadroxil, Cephaloglycin, Cefuroxim, Ceforanid, Cefotaxim, Cefatrizin, Cephacetril, Cefepim, Cefixim, Cefonizid, Cefoperazon, Cefotetan, Cefmetazol, Ceftazidim, Loracarbef und Moxalactam; Monobaktame wie Aztreonam; und Carbapeneme wie zum Beispiel Imipenem, Meropenem pentamidin isethiouat, Aalbuterol sulfat, Lidocain, Metaproterenol sulfat, Beclomethason diprepionat, Triamcinolon acetamid, Budesonid acetonid, Fluticason, Ipratropium bromid, Flunisolid, Cromolyn Natrium, Ergotamin tartrat und wo anwendbar, Analoge, Agonisten, Antagonisten, Inhibitoren und pharmazeutische verwendbare Salzformen und der gleichen hiervon.

Bei dem pharmazeutischen Wirkstoff handelt es sich nach einer weiteren Ausführungsform um ein biologisches Makromolekül. Entsprechend der oben angegebenen Definition sind hierunter beispielsweise Peptide, Proteine, Fette, Fettsäuren, oder auch Nukleinsäuren zu verstehen.

Biopharmazeutisch bedeutsame Proteine/Polypeptide umfassen z.B. Antikörper, Enzyme, Wachstumsfaktoren z.B. Steroide, Cytokine, Lymphokine, Adhäsions-moleküle, Rezeptoren sowie deren Derivate bzw. Fragmente, sind aber nicht auf diese beschränkt. Im Allgemeinen sind alle Polypeptide bedeutsam, die als Agonisten oder Antagonisten wirken und/oder therapeutische oder diagnostische Anwendung finden können.

Geeignete Peptide oder Proteine im Sinne der Erfindung sind beispielsweise Insulin, Insulin-ähnlicher Wachstumsfaktor, humanes Wachstumshormon (hGH) und andere Wachstumsfaktoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 und VEGF. Weitere Beispiele sind monoklonale, polyklonale, multispezifische und einzelkettige (*single chain*) Antikörper und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente (Chamov et al., 1999, Antibody Fusion Proteins, Wiley-Liss Inc.). Die Antikörper können humanen oder nicht-humanen Ursprungs sein. Hierunter fallen beispielsweise die im Menschen bekannten Klassen: IgA, IgD, IgE, IgG and IgM, mit ihren verschiedenen Subklassen, beispielsweise IgA1, IgA2 and IgG1, IgG2, IgG3 and IgG4. Auch humanisierte und chimäre Antikörper kommen in Frage. Von besonderer therapeutischer Bedeutung und somit Gegenstand der vorliegenden Erfindung sind Pulverformulierungen die Antikörper gegen beispielsweise verschiedene Oberflächenantigene wie CD4, CD20 oder CD44, verschiedene Cytokine, beispielsweise 1L2, 1L4 oder IL5. Weitere Beispiele sind Antikörper gegen bestimmte Immunglobulin-Klassen (z.B. anti-igE-Antikörper) oder gegen virale Proteine (zb. anti-RSV-, anti-CMV Antikörper, etc.).

Fab-Fragmente (Fragment antigen-binding = Fab) bestehen aus den variablen Regionen beider Ketten, die durch die angrenzenden konstanten Regionen zusammengehalten werden. Weitere Antikörperfragmente sind F(ab')₂-Fragmente, die durch proteolytischen Verdau mit Pepsin hergestellt werden können. Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Solche Antikörperfragmente werden auch als *single-chain* Fv-Fragment (scFv) bezeichnet. Beispiele von scFv-Antikörpern sind bekannt und beschrieben, siehe z.B. Huston et al., 1988, Proc. Natl. Acad. Sci. USA, 16, 5879ff.

In den vergangenen Jahren wurden verschiedene Strategien entwickelt um multimere scFv-Derivate herzustellen, wie z.B. Dia-, Tri- und Pentabodies. Als "Diabody" bezeichnet ein Fachmann ein bivalentes homodimeres scFv-Derivat. Die Verkürzung des Peptidlinkers im scFv-Molekül auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur, z.B. bei Perisic et al., 1994 (Structure, 2, 1217ff). Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region. Beispiele solcher Minibodies sind bei Hu et al.,1996, Cancer Res., 56, 3055ff beschrieben. Mit "Triabody" bezeichnet der Fachmann ein trivalentes homotrimeres scFv-Derivat (Kortt et al., 1997, Protein Engineering, 10, 423ff). Die direkte Fusion von VHVL ohne Verwendung einer Linkersequenz führt zur Ausbildung von Trimeren.

Bei den vom Fachmann als Mini-Antikörper bezeichneten Fragmenten, die eine bi-, tri-oder tetravalente Struktur haben, handelt es sich ebenfalls um Derivate von scFv-Fragmenten. Die Multimerisierung wird dabei über di-, tri- oder tetramere "coiled coil"-Strukturen erzielt (Pack. P. et al., 1993, Biotechnology, 11, 1271ff; Lovejoy, B. et al., 1993, Science, 259, 1288ff; Pack, P. et al., 1995, J. Mol. Biol., 246, 28ff).

Eine besonders bevorzugte Ausführung der Erfindung umfasst ein Protein aus der Klasse der Antiköper, genauer Typ 1 Immunglobulin G. Es handelt sich dabei um einen humanisierten monoklonalen Antikörper, mit 95% humanen und 5% murinen Antikörpersequenzen. Der Antikörper hat ein Molekulargewicht von ca. 148 Kilodalton (kDa), bestehend aus zwei leichten und zwei schweren Ketten und insgesamt vier Disulfidbrücken.

Besonders vorteilhaft sind Pulver, vorzugsweise gefrier- oder sprühgetrocknete Pulver, die als Wirkstoff ein Peptid oder Protein oder eine Kombination aus Peptid/Peptid, Peptid/Protein oder Protein/Protein enthalten. Die entsprechenden biologischen Makromoleküle können zwischen 0,01 bis 75% (w/w), bevorzugt zwischen 0,01 bis 50% (w/w) der Trockenmasse des Pulver ausmachen. Der Anteil beträgt somit beispielsweise 0,01, 0,02, 0,03 ... 0,08, 0,09, 0,1, 0,2, 0,3 ... 0,8, 0,9 etc.; 1, 2, 3, ... 8, 9, 10 etc.; 11, 12, 13, ... 18, 19, 20 etc.; 21, 22, 23, ... 28, 29, 30 etc.; 31, 32, 33, ... 38 ,39, 40 etc.; 41, 42, 43, ... 48, 49, 49,1, 49,2, 49,3, ... 49,8 ,49,9 etc.; 49,91, 49,92, 49,93, ... 49,98, 49,99, 50% (w/w).

Besonders vorteilhaft und erfindungsgemäß sind Pulver, vorzugsweise gefrier- oder sprühgetrocknete Pulver, mit einem Verhältnis von Hilfsstoffkombination enthaltend mindestens ein 1,4 O-verknüpften Saccharose-Derivat und einen weiteren Hilfsstoff zu Peptid/Protein von 25/75, 26/74, 27/73, 28/72, 29/71, 30/70, 31/69, 32/68, 33/67, 34/66, 35/65, 36/64, 37/63, 38/62, 39/61, 40/60, 41/59, 42/58, 43/57, 44/56, 45/55, 46/54, 47/53, 48/52, 49/51, 50/50, 51/49, 52/48, 53/47, 54/46, 55/45, 56/44, 57/43, 58/42, 59/41, 60/40, 61/39, 62/38, 63/37, 64/36, 65/35, 66/34, 67/33, 68/32, 69/31, 70/30, 71/29, 72/28, 73/27, 74/26, 75/25, 76/24, 77/23, 78/22, 79/21, 80/20, 81/19, 82/18, 83/17, 84/16, 85/15, 86/14, 87/13, 88/12, 89/11, 90/10, 91/9, 92/8, 93/7, 94/6, 95/5, 96/4, 97/3, 98/2, 99/1, 99,1/0,9, 99,2/0,8, 99,3/0,7, 99,4/0,6, 99,5/0,5, 99,6/0,4, 99,66/0,33, 99,7/0,3, 99,8/0,2, 99,9/0,1, 99,99/0,01 (w/w).

Enthalten die erfindungsgemäßen Pulver sehr kleine Proteine/Peptide mit einem Molekulargewicht von < 10 kDa, vorzugsweise von < 5 kDa, wie zum Beispiel Wachstumsfaktoren, beispielsweise Cytokine, so beträgt der Anteil vorzugsweise zwischen 0,1 bis 10% (w/w), weiter bevorzugt zwischen 0,2 bis 5% (w/w) am Gesamtgewicht des Pulvers. Demnach sind Pulver bevorzugt deren Anteil an Cytokinen 0,2, 0,3, 0,4 ... 0,8, 0,9 etc.; 1, 2, 3, ... etc; 4,1, 4,2, 4,3, ... 4,8 ,4,9 etc.; 4,91, 4,92, 4,93, ... 4,98, 4,99% (w/w) beträgt.

Handelt es sich bei dem pharmazeutischen Wirkstoff hingegen um einen oder mehrere Antikörper oder ein Derivat hiervon, so beträgt der Wirkstoffanteil am Feststoffgehalt des Pulvers zwischen 0,01 bis 75% (w/w), bevorzugt zwischen 0,01 und 50% (w/w), vorzugsweise zwischen 0,1 und 50% (w/w), weiter bevorzugt zwischen 0,33 und 50% (w/w), beispielsweise 0,1, 0,2, 0,3, 0,33, ... 0,66, 0,7, 0,8, 0,9 etc.; 1, 2, 3, ... 8 ,9, 10 etc.; 11, 12, 13, ... 18, 19, 20 etc.; 21, 22, 23, ... 28 ,29, 30 etc.; 31, 32, 33, ... 38, 39, 40 etc.; 41, 42, 43, ... 48 ,49, etc; 49,1, 49,2, 49,3, ... 49,8, 49,9 etc.; 49,91, 49,92, 49,93, ... 49,98, 49,99, 50 (w/w).

Nach einer besonderen Ausführungsform beträgt der Antikörperanteil am Feststoffgehalt des Pulvers zwischen 10 und 50% (w/w), weiter bevorzugt zwischen 10 und 30% (w/w), noch weiter bevorzugt zwischen 10 und 20% (w/w). Besonders vorteilhaft und erfindungsgemäß sind Pulver, vorzugsweise gefrier- oder sprühgetrocknete Pulver, mit einem Verhältnis von Hilfsstoffkombination enthaltend mindestens ein 1,4 O-verknüpften Saccharose-Derivat und einen weiteren Hilfsstoff zu Antikörper von 50/50, 51/49, 52/48, 53/47, 54/46, 55/45, 56/44, 57/43, 58/42, 59/41, 60/40, 61/39, 62/38, 63/37, 64/36, 65/35, 66/34, 67/33, 68/32, 69/31, 70/30, 71/29, 72/28, 73/27, 74/26, 75/25, 76/24, 77/23, 78/22, 79/21, 80/20, 81/19, 82/18, 83/17, 84/16, 85/15, 86/14, 87/13, 88/12, 89/11, oder 90/10 (w/w).

Nach einer speziellen Ausführungsform betrifft die vorliegende Erfindung Pulver, vorzugsweise gefrier- oder sprühgetrocknete Pulver, dadurch gekennzeichnet, dass die Trockenmasse des Pulvers zumindest 25% (w/w), vorzugsweise zwischen 55 und 99,99% (w/w), besonders bevorzugt zwischen 60 und 90% (w/w) einer Zuckermischung enthaltend zumindest ein 1,4 O-verknüpften Saccharose-Derivat und bis zu 75% (w/w) eines pharmazeutischen Wirkstoff enthält, wobei der Anteil an Lactosucrose, Maltosyl-Sucrose und/oder Glucosyl-Sucrose mindestens 20% (w/w) in Bezug auf die Trockenmasse des Pulvers beträgt und die Summe der Gewichtsprozente maximal 100% (w/w) beträgt. Ein Fachmann ist in der Lage entsprechende Pulver herzustellen.

So weiß ein Fachmann, dass er bezogen auf den Gesamtfeststoffgehalt der zu trocknenden Lösung maximal 10% (w/w) eines pharmazeutischen Wirkstoffs beimischen kann, wenn der Anteil der Zuckermischung enthaltend zumindest ein 1,4 O-verknüpften Saccharose-Derivat 90% (w/w) betragen soll.

Die vorliegende Erfindung betrifft auch entsprechende Pulver, vorzugsweise gefrier- oder sprühgetrocknete Pulver, die neben dem pharmazeutischen Wirkstoff als Hilfsstoffkombination zumindest ein 1,4 O-verknüpftes Saccharose-Derivat oder eine Zuckermischung enthaltend zumindest ein 1,4 O-verknüpften Saccharose-Derivat und ein oder mehrere pharmazeutisch verträgliche Hilfsstoffe und/oder ein oder mehrere Salze enthalten. Bei den Hilfsstoffen handelt es sich beispielhaft um die oben erwähnten Aminosäuren, Peptide und deren Salze, Zucker, Polyole, Salze organischer Säuren und/oder Polymere.

Nach einer weiteren Ausführungsform betrifft die vorliegenden Erfindung auch Pulver, vorzugsweise gefrier- oder sprühgetrocknete Pulver, die neben dem pharmazeutischen Wirkstoff als Hilfsstoffkombination zumindest ein 1,4 O-verknüpftes Saccharose-Derivat oder eine Zuckermischung enthaltend zumindest ein 1,4 O-verknüpften Saccharose-Derivat und eine oder mehrere Aminosäure(n), vorzugsweise eine Aminosäure enthalten. In diesem Zusammenhang betrifft die vorliegende Erfindung Pulver, die in Bezug auf ihre Trockenmasse **a)** zumindest 25% (w/w), vorzugsweise zwischen 50 und 90% (w/w), besonders bevorzugt zwischen 60 und 90% (w/w) eines 1,4 O-verknüpften Saccharose-Derivats oder eine Zuckermischung enthaltend zumindest ein 1,4 O-verknüpften Saccharose-Derivat, **b)** zwischen 1 und 19,99% (w/w) Aminosäuren **c)** sowie zwischen 0,01 und 74% (w/w) eines pharmazeutischen Wirkstoffs, vorzugsweise eines biologischen Makromoleküls enthalten, wobei die Summe der Gewichtsanteile maximal 100% (w/w) ergeben kann. Nach einer bevorzugten Ausführungsform beträgt der Anteil an dem 1,4 O-verknüpften Saccharose-Derivat oder einer Zuckermischung enthaltend zumindest ein 1,4 O-verknüpften Saccharose-Derivat zumindest 60% (w/w), vorzugsweise zwischen 70 und 90% (w/w) in Bezug auf die Trockenmasse des Pulvers. Bei einer entsprechenden Formulierung beträgt der Anteil an Aminosäuren vorzugsweise zwischen 1 und 19,99% (w/w) und der Anteil des pharmazeutischen Wirkstoffs zwischen 0,01 bis 10% (w/w). Der Anteil an Aminosäuren kann auch auf bis zu 40% (w/w) erhöt werden. In diesen Fällen ist der Anteil an pharmazeutischen Wirkstoff und/oder dem 1,4 O-verknüpften Saccharose-Derivat oder einer Zuckermischung enthaltend zumindest ein 1,4 O-verknüpften Saccharose-Derivat entsprechedn zu reduzieren, so dass die Summe der Feststoffanteile maximal 100% (w/w) ergibt. Dies gilt insbesondere für die Verwendung von Isoleucin als Aminosäure.

Folglich betrifft die vorliegende Erfindung nach einer weiteren Ausführungsform auch Pulver, vorzugsweise gefrier- oder sprühgetrocknete Pulver, die beispielsweise 80% (w/w) an einem 1,4 O-verknüpften Saccharose-Derivat oder einer Zuckermischung enthaltend zumindest ein 1,4 O-verknüpften Saccharose-Derivat, / 19% (w/w) Aminosäure /1% (w/w) pharmazeutischen Wirkstoff (80/19/1); oder beispielsweise (80/18/2); (80/17/3); (80/16/4); (80/15/5); (80/14/6); (80/13/7); (80/12/8); (80/11/9); (80/10/10); (70/20/10); (70/19/11); (70/18/12); (70/17/13); (70/16/14); (70/15/15); (70/14/16); (70/13/17); (70/12/18); (70/11/19); (70/10/20); (60/20/20); (60/19/21); (60/18/22); (60/17/23); (60/16/24); (60/15/25); (60/14/26); (60/13/27); (60/12/28); (60/11/29) oder (60/10/30) enthalten oder aus diesen bestehen. Sofern der Wirkstoffanteil bei einem konstanten Aminosäureanteil von 20% (w/w) bis auf 0,01 % (w/w) reduziert wird, beispielsweise auf 9,99, ... 9,9, 9,8, 9,7 ... 9,3, 9,2, 9,1 ... 9, 8 7, 6, 5, 4, 3, 2, 1, ... 0,9, 08, 0,7, ... 0,66, ... 0,6, 0,5, 0,4, 0,3, 0,2, 0,1, 0,09, 0,08, 0,07, 0.06, 0,05, 0,04, 0,03 0,02, 0,01% (w/w), kann dementsprechend der Anteil an 1,4 O-verknüpften Saccharose-Derivat oder der Zuckermischung enthaltend zumindest ein 1,4 O-verknüpften Saccharose-Derivat, auf beispielsweise 80,01, ... 80,1, 80,2, 80,3 ... 80,8, 80,9, 81, 82, 83, 84, 85, 86, 87, 88, 89, ..., 89,1, 89,2, 89,3, ... 89,33, ... 89,4, 89,5, 89,6 , 89,7, 89,8, 89,9, ... 89,91, 89,92, 89,93, ... 89,97, 89,98, 89,99% (w/w) erhöht werden, so dass die Summe der Gewichtsanteile der einzelnen Pulverbestandteile in Bezug auf die Trockenmasse des Pulvers 100% (w/w) ergibt. Durch Zusatz von weiteren Hilfsstoffen oder Salzen kann der Anteil an dem 1,4 O-verknüpften Saccharose-Derivat oder der Zuckermischung enthaltend zumindest ein 1,4 O-verknüpften Saccharose-Derivat, Aminosäure/Peptiden und/oder pharmazeutischem Wirkstoff entsprechend angepasst/reduziert werden, so dass die Gewichtsanteile der einzelnen Bestandteile in der Summe 100% (w/w) ergibt.

Erfindungsgemäß sind ferner Pulver mit folgender Zusammensetzung: 79% (w/w) an 1,4 O-verknüpften Saccharose-Derivat oder einer Zuckermischung enthaltend zumindest ein 1,4 O-verknüpften Saccharose-Derivat, / 11 % (w/w) Aminosäure, / 10% (w/w) pharmazeutischer Wirkstoff (79/11/10); (78/12/10); (77/13/10); (76/14/10); (75/15/10); (74/16/10); (73/17/10); (72/18/10); (71/19/10); (70/20/10), wobei der Anteil der Aminosäure pharmazeutischen Wirkstoffs auch von 10 auf 0,01 % (w/w), beispielsweise auf 9,99, ... 9,9, 9,8, 9,7 ... 9,3, 9,2, 9,1 ... 9, 8 7, 6, 5, 4, 3, 2, 1, ... 0,9, 08, 0,7, ... 0,66, ... 0,6, 0,5, 0,4, 0,3, 0,2, 0,1, 0,09, 0,08, 0,07, 0,06, 0,05, 0,04, 0,03 0,02, 0,01% (w/w) reduziert werden kann und dementsprechend der Anteil an dem 1,4 O-verknüpften Saccharose-Derivat oder einer Zuckermischung enthaltend zumindest ein 1,4 O-verknüpften Saccharose-Derivat, auf beispielsweise 80,01, ... 80,1, 80,2, 80,3 ... 80,8, 80,9, 81, 82, 83, 84, 85, 86, 87, 88, 89, ..., 89,1, 89,2, 89,3, ... 89,33, ..., 89,4, 89,5, 89,6 , 89,7, 89,8, 89,9, ... 89,91, 89,92, 89,93, ..., 89,97, 89,98, 89,99% (w/w) erhöht werden kann, so dass die Summe der Gewichtsanteile in Bezug auf die Trockenmasse des Pulvers 100% (w/w) ergibt.

Handelt es bei der zugesetzten Aminosäure um Isoleucin, so sind nach einer weiteren Ausführungsform Pulver, vorzugsweise gefrier- oder sprühgetrocknete Pulver, mit einem Anteil a) an 1,4 O-verknüpften Saccharose-Derivat oder einer Zuckermischung enthaltend zumindest ein 1,4 O-verknüpften Saccharose-Derivat, von zumindest 25% (w/w), vorzugsweise von 50 bis 90% (w/w), besonders bevorzugt von 60 bis 90% (w/w), b) einem Anteil von 1 bis 19,99% (w/w) Isoleucin und c) von zumindest 0,01 % (w/w), vorzugsweise 0,01 bis maximal 74% (w/w) eines pharmazeutischen Wirkstoffs, vorzugsweise eines Peptids/Proteins, erfindungsgemäß. Vorzugsweise beträgt der Anteil an Isoleucin 5 bis 19,99% (w/w), weiter bevorzugt 10 bis 19,99% (w/w) am Gesamtfeststoffanteil des Pulvers. Auch hier gilt, dass die Summe der Gewichtsprozente der einzelnen Bestandteile 100% (w/w) nicht übersteigt. Erfindungsgemäß sind demnach auch Pulver mit folgender Zusammensetzung: 80% (w/w) an 1,4 O-verknüpften Saccharose-Derivat oder einer Zuckermischung enthaltend zumindest ein 1,4 O-verknüpften Saccharose-Derivat / 19% (w/w) Isoleucin / 1% (w/w) pharmazeutischer Wirkstoff (80/19/1); (80/18/2); (80/17/3); (80/16/4); (80/15/5); (80/14/6); (80/13/7); (80/12/8); (80/11/9); (80/10/10); (70/19/11); (70/18/12); (70/17/13); (70/16/14); (70/15/15); (70/14/16); (70/13/17); (70/12/18); (70/11/19); (70/10/20); (60/19/21); (60/18/22); (60/17/23); (60/16/24); (60/15/25); (60/14/26); (60/13/27); (60/12/28); (60/11/29); (60/10/30). Bei Zusatz von weiteren Hilfsstoffen oder Salzen ist der Anteil an 1,4 O-verknüpften Saccharose-Derivat oder einer Zuckermischung enthaltend zumindest ein 1,4 O-verknüpften Saccharose-Derivat, Isoleucin und/oder pharmazeutischem Wirkstoff entsprechend anzupassen, so dass die Gewichtsanteile der einzelnen Bestandteile in der Summe 100% (w/w) ergeben.

Eine weitere Ausführungsform der vorliegenden Erfindung betriff auch Pulver, vorzugsweise gefrier- oder sprühgetrocknete Pulver, die neben dem pharmazeutischen Wirkstoff eine Hilfsstoffkombination enthalten aus zumindest einem 1,4 O-verknüpften Saccharose-Derivat oder einer Zuckermischung enthaltend zumindest ein 1,4 O-verknüpften Saccharose-Derivat, und einem oder mehrere Peptide, vorzugsweise einem oder mehrere Di- und/oderTri-Peptide. Die vorliegende Patentschrift nennt beispielhaft einige Tri-Peptide, die zusammen mit dem 1,4 O-verknüpften Saccharose-Derivat oder einer Zuckermischung enthaltend zumindest ein 1,4 O-verknüpften Saccharose-Derivat, zur Herstellung der erfindungsgemäßen Pulver verwendet werden können. Nach einer besonderen Ausführungsform handelt es sich bei den Peptiden, vorzugsweise bei den Di- oder Tri-Peptiden um solche, die zumindest ein Isoleucinrest, vorzugsweise zwei Isoleucinreste enthalten, oder nach einer besonders vorteilhaften Ausführungsform, aus drei Isoleucinresten bestehen.

In diesem Zusammenhang gelten Pulver als erfindungsgemäß mit **a)** einem Anteil an zumindest einem 1,4 O-verknüpften Saccharose-Derivat oder einer Zuckermischung, enthaltend zumindest ein 1,4 O-verknüpften Saccharose-Derivat, von zumindest 25% (w/w), vorzugsweise von 60 bis 99% (w/w), besonders bevorzugt von 60 bis 90% (w/w), **b)** einem Anteil von 1 bis 19,99% (w/w) eines Peptids, vorzugsweise eines Di-oder Tri-Peptids, besonders bevorzugt ein Isoleucin-haltiges Peptid wie zum Beispiel Tri-Isoleucin und **c)** 0,01 bis maximal 74% (w/w) eines pharmazeutischen Wirkstoffs, vorzugsweise eines Peptids/Proteins. Auch hier gilt, dass die Summe der Einzelfeststoffe 100% (w/w) nicht übersteigen kann. Der Anteil an dem Peptid, welches als Hilfsstoff eingestzt wird und nicht dem pharmazeutischen Wirkstoff entspricht, kann auch auf bis zu 40% (w/w) erhöht werden. In diesen Fällen ist der Anteil an pharmazeutischen Wirkstoff und/oder dem 1,4 O-verknüpften Saccharose-Derivat oder einer Zuckermischung enthaltend zumindest ein 1,4 O-verknüpften Saccharose-Derivat entsprechend zu reduzieren, so dass die Summe der Feststoffanteile maximal 100% (w/w) ergibt. Dies gilt insbesondere für die Verwendung von Di- oder Tri-Peptiden vorzugsweise für Tri-Isoleucin.

Erfindungsgemäß sind ferner Pulver mit folgender Zusammensetzung: 89% (w/w) an zumindest einem 1,4 O-verknüpften Saccharose-Derivat oder einer Zuckermischung enthaltend zumindest ein 1,4 O-verknüpftes Saccharose-Derivat / 1 % (w/w) Peptid, vorzugsweise Di- oder Tri-Peptid, weiter bevorzugt ein Isoleucin-haltiges Di-oder Tri-Peptid, besonders bevorzugt Tri-Isoleucin / 10% (w/w) pharmazeutischer Wirkstoff (89/1/10); (88/2/10); (87/3/10); (86/4/10); (85/5/10); (84/6/10); (83/7/10); (82/8/10); (81/9/10); (80/10/10); (79/11/10); (78/12/10); (77/13/10); (76/14/10); (75/15/10); (74/16/10);, (73/17/10); (72/18/10) oder (71/19/10), wobei der Anteil am pharmazeutischen Wirkstoff auch von 10 auf 0,01 % (w/w), beispielsweise auf 9,99, ... 9,9, 9,8, 9,7 ... 9,3, 9,2, 9,1 ... 9, 8 7, 6, 5, 4, 3, 2, 1, ... 0,9, 08, 0,7, ... 0,66, ... 0,6, 0,5, 0,4, 0,3, 0,2, 0,1, 0,09, 0,08, 0,07, 0,06, 0,05, 0,04, 0,03 0,02, 0,01% (w/w) reduziert werden kann und sich dementsprechend der Anteil an dem 1,4 O-verknüpften Saccharose-Derivat oder der Zuckermischung enthaltend zumindest ein 1,4 O-verknüpften Saccharose-Derivat, auf beispielsweise 80,01, ... 80,1, 80,2, 80,3 ... 80,8, 80,9, 81, 82, 83, 84, 85, 86, 87, 88, 89, ... 89,1, 89,2, 89,3, ... 89,33, ... 89,4, 89,5, 89,6, 89,7, 89,8, 89,9, ... 89,91, 89,92, 89,93, ... 89,97, 89,98, 89,99% (w/w) erhöhen kann, so dass die Summe der Gewichtsanteile in Bezug auf die Trockenmasse des Pulvers 100% (w/w) ergibt. Erfindungsgemäß sind demnach auch Pulver mit folgender Zusammensetzung: 80% (w/w) an zumindest einem 1,4 O-verknüpften Saccharose-Derivat oder einer Zuckermischung enthaltend zumindest ein 1,4 O-verknüpften Saccharose-Derivat / 19% (w/w) Peptid, vorzugsweise ein Di-oder Tri-Peptid, weiter bevorzugt ein Isoleucin-haltiges Di-oder Tri-Peptid, noch weiter bevorzugt Tri-Isoleucin /1% (w/w) pharmazeutischer Wirkstoff (80/19/1); (80/18/2); (80/17/3); (80/16/4); (80/15/5); (80/14/6); (80/13/7); (80/12/8); (80/11/9); (80/10/10); (70/19/11); (70/18/12); (70/17/13); (70/16/14); (70/15/15); (70/14/16); (70/13/17); (70/12/1.8); (70/11/19); (70/10/20); (60/20/20); (60/19/21); (60/18/22); (60/17/23); (60/16/24); (60/15/25); (60/14/26); (60/13/27); (60/12/28); (60/11/29); (60/10/30), wobei der Anteil an Peptid, vorzugsweise an Di-oder Tri-Peptid, weiter bevorzugt an Isoleucin-haltigem Di- oder Tri-Peptid, noch weiter bevorzugt an Tri-Isoleucin auch von 10 auf 1 % (w/w), beispielsweise auf 9,99, ... 9,9, 9,8, 9,7 ... 9,3, 9,2, 9,1 ... 9, 8 7, 6, 5, 4, 3, 2 ,1,9, 1,8, 1,7, ... 1,66, ... 1,6, 1,5, 1,4, 1,3, 1,2, 1,1, 1% (w/w) reduziert werden kann und dementsprechend der Anteil an pharmazeutischen Wirkstoff, vorzugsweise an Peptid/Protein auf beispielsweise 30,1, 30,2, 30,3 ... 30,8, 30,9, 31, 32, 33, 34, 35, 36, 37, 38, 38,1, 38,2, 38,3, ... 38,33, ..., 38,4, 38,5, 38,6 , 38,7, 38,8, 38,9, ... 39% (w/w) erhöht werden kann, so dass die Summe der Gewichtsanteile in Bezug auf die Trockenmasse des Pulvers 100% (w/w) ergibt. Bei Reduktion des Anteils an Peptid, vorzugsweise an Di-oder Tri-Peptid, weiter bevorzugt an Isoleucin-haltigem Di- oder Tri-Peptid, noch weiter bevorzugt an Tri-Isoleucin von 10 auf 1% (w/w), wie hier dargestellt, kann auch der Anteil an dem 1,4 O-verknüpften Saccharose-Derivat oder einer Zuckermischung enthaltend zumindest ein 1,4 O-verknüpften Saccharose-Derivat im Pulver erhöht werden. Bei beispielsweise konstantem Wirkstoffanteil von 10% (w/w) lassen sich so Pulver mit einem Anteil an 1,4 O-verknüpften Saccharose-Derivat oder einer Zuckermischung enthaltend zumindest ein 1,4 O-verknüpften Saccharose-Derivat, von 80,1, 80,2, 80,3 ... 80,8, 80,9, 81, 82, 83, 84, 85, 86, 87, 88, 88,1, 88,2, 88,3, ... 88,33, ..., 88,4, 88,5, 88,6 , 88,7, 88,8, 88,9 bzw. 89% (w/w) herstellen.

Nach einer weiteren erfindungsgemäßen Ausführungsform können die Pulver zusätzlich oberflächenaktiven Substanzen wie Tween 20, 40, 60, 80, Brij 35, Pluronic F 88 und Pluronic F 127 enthalten. Diese werden vorzugsweise in einer Konzentration von 0,01-0,1% (w/w) eingesetzt. Besonders bevorzugt ist ein Pulver, dass als Hilfsstoffkombination ein 1,4 O-verknüpften Saccharose-Derivat oder einer Zuckermischung, enthaltend zumindest ein 1,4 O-verknüpften Saccharose-Derivat und zusätzlich Tween 20, vorzugsweise in einer Konzentration von 0,01-0,1% (w/w), als oberflächenaktive Substanz enthält.

Nach einer weiteren Ausführungsform verfügen die Partikel in den erfindungsgemäßen Pulvern über einen MMD zwischen 1 und 10 µm, vorzugsweise zwischen 1 und 5 *µ*m.

Nach einer weiteren Ausführungsform betrifft die vorliegende Erfindung Pulver, vorzugsweise gefrier- oder sprühgetrocknete Pulver, mit einer der hier beschriebenen Zusammensetzung die durch eine Glasübergangstemperatur größer 40°C gekennzeichnet sind. Üblicherweise verfügen die die entsprechenden erfindungsgemäßen Pulver über eine maximale Glasübergangstemperatur von ca. 96 bis 110°C. In Einzelfällen kann der Wert aber auch noch höher liegen.

Ferner betrifft die vorliegende Erfindung auch pharmazeutischen Zusammensetzungen, die zumindest eins der hier beschriebenen erfindungsgemäßen Pulver, vorzugsweise gefrier- oder sprühgetrocknete Pulver, enthalten.

### Herstellung der erfindungsgemäßen Pulver:

Die vorliegende Erfindung stellt auch Verfahren zur Herstellung eines der oben näher beschriebenen Pulvers, vorzugsweise eines sprühgetrockneten oder gefriergetrockneten Pulvers, zur Verfügung. Das Verfahren ist dadurch gekennzeichnet, dass ein pharmazeutischer Wirkstoff und eine Hilfsstoffkombination enthaltend zumindest ein 1,4 O-verknüpftes Saccharose-Derivat ausgewählt aus den Verbindungen: 1,4 O-verknüpfte D-Gal-Saccharose (Lactosucrose), 1,4 O-verknüpfte D-Glu-Saccharose (Glucosyl-Sucrose), oder 1,4 O-verknüpfte Glu-Glu-Saccharose (Maltosyl-Sucrose) in Kombination mit zumindest einem weiteren Hilfsstoff gemischt und unter geeigneten Bedingungen getrocknet werden. Bei dem weiteren Hilfsstoff handelt es sich vorzugsweise um Aminosäuren, vorzugsweise um Isoleucin, ein Peptid, vorzugsweise um Di-oder Tri-Peptide, und/oder um ein Mono-, Di- und/oder Oligosaccharid, wobei es sich bei den Oligosaccharid auch um ein zweites 1,4 O-verknüpftes Saccharose-Derivat handeln kann, sofern dieses von dem ersten verschieden ist.

Grundsätzlich lassen sich die erfindungsgemäßen Pulver herstellen, in dem der pharmazeutische Wirkstoff, vorzugsweise ein biologisches Makromolekül in Form eines Peptids oder Proteins, in einer wässrigen Lösung in Abhängigkeit der Löslichkeitsbedingungen des jeweiligen Wirkstoffs gelöst wird. Zumeist werden gepufferte Lösungen mit einem pH von 3-11, vorzugsweise von 3,5-9 verwendet. Bei der Herstellung von inhalierbaren Pulvern ist eine wässrige Lösung mit einem pH von 4-7,8 besonders vorteilhaft. Um eine hinreichende Löslichkeit zu gewähren, sollte der pH-Wert der Lösung unterhalb des pl-Werts des Peptids/Proteins und Hilfsstoffs liegen. Die wässrige Lösung kann optional zusätzliche wasserlösliche organische Lösungsmittel enthalten, wie z.B. Aceton, Alkohole oder der gleichen. Besonders geeignet sind niedere Alkohole wie z.B. Methanol, Ethanol, Propanol, (n- oder iso-Propanol) oder der gleichen. Solche gemischten Lösungsmittelsysteme enthalten normalerweise zwischen 10-20% (v/v) eines wasserlöslichen organischen Lösungsmittels. Der Feststoffanteil in der zu trocknenden Lösung beträgt üblicherweise zwischen 0,01-20% (w/w), vorzugsweise zwischen 0,05-10% (w/w), besonders bevorzugt zwischen 0.1-5% (w/w). In Rahmen der vorliegenden Erfindung wurden sprühgetrocknete und gefriergetrocknete Pulver ausgehend von einer wässrigen Lösung mit einem Feststoffanteil von 10% (w/w) sowie sprühgetrocknete Pulver mit einem Festsoffanteil von 3,33% (w/w) bzw. 2% (w/w) hergestellt.

Üblicherweise werden die Hilfsstoffkombinationen, wie oben beispielhaft aufgeführt, in einem zweiten Behältnis in Reinstwasser oder einer geeigneten Pufferlösung mit einem pH-Wert von 3 bis 11, bevorzugt von 3,5 bis 9 und besonders bevorzugt von 4,0 bis 7,8 gelöst und in einem zweiten Schritt mit der Wirkstofflösung vermischt. Anschließend wird die Lösung / Suspension mit Reinstwasser oder einer geeigneten Pufferlösung mit einem pH-Wert von 3 bis 11, bevorzugt von 3,5 bis 9 und besonders bevorzugt von 4,0 bis 7,8 auf den gewünschten Feststoffgehalt eingestellt.

Folglich betrifft die vorliegende Erfindung ein Verfahren zur Herstellung eines Pulvers dadurch gekennzeichnet, dass
a) ein pharmazeutischer Wirkstoff in einer wässrigen Lösung / Suspension gelöst /suspendiert wird;
b) eine Hilfsstoffkombination aus zumindest einem 1,4 O-verknüpften Saccharose-Derivat ausgewählt aus den Verbindungen Lactosucrose, Glucosyl-Sucrose, oder Maltosyl-Sucrose in Kombination mit zumindest einem weiteren Hilfsstoff in einer wässrigen Lösung / Suspension gelöst / suspendiert wird;
c) sofern Wirkstoff und Hilfsstoffkombination aus zumindest einem 1,4 O-verknüpften Saccharose-Derivat in Kombination mit zumindest einem weiteren Hilfsstoff in verschiedenen Lösungen / Suspension gelöst / suspendiert sind, diese gemischt werden;
d) die Lösung / Suspension die die Hilfsstoffkombination aus zumindest einem 1,4 O-verknüpften Saccharose-Derivat in Kombination mit zumindest einem weiteren Hilfsstoff und den pharmazeutischen Wirkstoff getrocknet werden.

Handelt es sich bei dem Trocknungsverfahren um Sprühtrocknung so erfolgt die Trocknung unter Punkt d) durch Versprühen der entsprechenden Lösung / Suspension unterhalb von einer Temperatur von 200/120°C (Einström- /Ausströmtemperatur), vorzugsweise zwischen 186/96°C und 60/40°C, beispielsweise bei 180-150/95-80°. Der Prozess ist im Abschnitt "BEISPIELE" anhand einiger Beispiele näher beschrieben.

Die Hilfsstoffkombination enthaltend zumindest ein 1,4 O-verknüpftes Saccharose-Derivat kann auch eine Zuckermischung enthalten oder aus dieser bestehen, die zumindest ein 1,4 O-verknüpfte Saccharose-Derivat und einen weiteren Zucker enthält. Beispiele entsprechend geeigneter Zuckermischungen werden beispielhaft unter dem Punkt "Definitionen" näher beschrieben. Dabei können die Zuckermischungen auch mehrere unterschiedliche 1,4 O-verknüpfte Saccharose-Derivate enthalten, optional auch in Kombination mit weiteren Mono-, Di- und/oder Oligosacchariden. Im Rahmen der Erfindung lassen sich so beispielsweise Zuckermischungen verwenden mit Lactosucrose, Lactose und Saccharose, wobei der Anteil an Lactosucrose in Bezug auf den gesamten Zuckeranteil ≥ 40% (w/w), vorzugsweise ≥ 55% (w/w), oder aber auch ≥ 88% (w/w) oder mehr beträgt. Vorzugsweise handelt es sich bei der Zuckermischung um eine als Nyuka-Oligo® LS55P, oder kurz LS55P, bezeichnete Zuckermischung der Firma Hayashibara Shoji, Inc., Japan, die zumindest 55% Lactosucrose, maximal 25% (w/w) Lactose und maximal 10% (w/w) Saccharose enthält. Nach einer weiteren Ausführungsform handelt es sich bei der Zuckermischung um eine als Nyuka-Oligo® LS90P, oder kurz LS90P, bezeichnete Zuckermischung der Firma Hayashibara Shoji, Inc., Japan, die zumindest 88% Lactosucrose sowie maximal 10% (w/w) Lactose und Saccharose enthält. Ferner lassen sich auch Zuckermischungen aus einer Kombination aus Glucosyl- und Maltosyl-Sucrose verwenden, vorzugsweise wiederum in Kombination mit weiteren Mono-, Di- und /oder Polysacchariden. Folglich eignen sich im Sinne der vorliegenden Erfindung auch entsprechende Zuckermischungen aus Glucosyl- und Maltosyl-Sucrose, Saccharose, Glucose und/oder Fructose, wobei Anteil an Glucosyl- und Maltosyl-Sucrose in Bezug auf den gesamten Zuckeranteil vorzugsweise 25% (w/w) oder mehr beträgt. Nach einer weiteren Ausführungsform beträgt der jeweilige Anteil an Glucosyl- und Maltosyl-Sucrose zumindest 18% (w/w) am Gesamtzuckeranteil. Nach einer weiter bevorzugten Ausführungsform handelt es sich bei der verwendeten Zuckermischung um eine als Coupling Sugar® bezeichnete Zuckermischung der Firma Hayashibara Shoji, Inc., Japan, die zumindest jeweils 18% (w/w) Glucosyl- und Maltosyl-Sucrose, zwischen 11 und 15% (w/w) Saccharose und jeweils zwischen 5 und 9% (w/w) Glucose und Fructose enthält. Ferner eignen sich im Sinne der vorliegenden Erfindung auch eine als Coupling Sugar S® bezeichnete Zuckermischung der Firma Hyashibara Hayashibara Shoji, Inc., Japan, die zumindest 25% (w/w) Glucosyl- und/oder Maltosyl-Sucrose, zwischen 48 und 56 % (w/w) Saccharose und nicht mehr als 10% (w/w) Glucose und Fructose enthält.

Entsprechend der oben beschriebenen erfindungsgemäßen Pulver enthält die zu trocknende Lösung / Suspension nach einer weiteren Ausführungsform zusätzlich ein oder mehrere pharmazeutisch verträgliche Hilfsstoffe und/oder ein oder mehrere Salze. Bei den Hilfsstoffen handelt es sich vorzugsweise um Aminosäuren, Peptide, Alkohole, Polyole, nicht-biologische und/oder biologische Polymere. Weitere im Stand der Technik bekannte Hilfsstoffe, die in Kombination mit mindestens einem 1,4 O-verknüptten Saccharose-Derivat oder einer Zuckermischung, enthaltend mindestens ein 1,4 O-verknüpfte Saccharose-Derivat, erfindungsgemäß eingesetzt werden können sind beispielsweise Lipide, Fettsäuren Fettsäureester, Steroide (z.B. Cholesterol) oder Chelatbildner (z.B. EDTA) sowie diverse Kationen. Besonders bevorzugt sind Hilfsstoffe mit einer hohen Glasübergangstemperatur, beispielsweise von größer 40°C, vorzugsweise von größer 45°C, oder von größer 55°C. Beispiele an geeigneten Hilfsstoffen und Hilfsstoffkombinationen finden sich in dieser Patentschrift unter dem Kapitel "erfindungsgemäße Pulver". Eine weitere Auflistung geeigneter Hilfsstoffe findet sich darüberhinaus auch beispielhaft in Kippe (Eds.), "Handbook of Pharmaceutical Excipients" 3rd Ed., 2000.

Der Gehalt an der Hilfsstoffkombination enthaltend zumindest ein 1,4 O-verknüpftes Saccharose-Derivat in Kombination mit einem weiteren Hilfsstoff, in der zu trocknenden Lösung / Suspension beträgt zwischen 25% und 99,99% (w/w), vorzugsweise zwischen 60% und 99% (w/w), besonders bevorzugt zwischen 60 und 90% (w/w) in Bezug auf den Feststoffgehalt der zu trocknenden Lösung / Suspension. Die Wirkstoffkonzentration beträgt normalerweise zwischen 0,01 und 75% (w/w), vorzugsweise zwischen 0,01 und 50% (w/w), weiter bevorzugt zwischen 0,33 und 50% (w/w), noch weiter bevorzugt zwischen 0,33 und 40% (w/w). Nach einer weiter bevorzugten Ausführungsform beträgt der Anteil des pharmazeutischen Wirkstoffs am Feststoffgehalt der zu trocknende Lösung / Suspension zwischen 0,33 und 35% (w/w), vorzugsweise zwischen 0,33 und 30% (w/w), weiter bevorzugt zwischen 0,33 und 25% (w/w) und noch weiter bevorzugt zwischen 0,33 und 10% (w/w).

Folglich betrifft die vorliegende Erfindung auch Verfahren zur Herstellung eines getrockneten Pulver, vorzugsweise eines gefrier- oder sprühgetrockneten Pulvers, wie oben beschrieben, dadurch gekennzeichnet, dass der Feststoffanteil der zu trocknenden Lösung / Suspension zwischen 25 und 99,99% (w/w), vorzugsweise zwischen 60 und 90% (w/w) einer Hilfsstoffkombination enthält, die zumindest ein 1,4 O-verknüpftes Saccharose-Derivat in Kombination mit einem weiteren Hilfsstoff enthält. Nach einer weiter bevorzugten Ausführungsform betrifft die vorliegende Erfindung ein entsprechendes Verfahren dadurch gekennzeichnet, dass der Feststoffanteil der zu trocknenden Lösung / Suspension einen pharmazeutischen Wirkstoff zwischen 0,01 und 75% (w/w), vorzugsweise zwischen 0,33 und 50% (w/w), besonders bevorzugt zwischen 0,33 und 30% (w/w) enthält.

Nach einer weiteren Ausführungsform des vorliegenden Verfahrens wird eine Lösung /Suspension mit einem Feststoffgehalt von **a)** zumindest 25% (w/w), beispielsweise zwischen 25 bis 99,99% (w/w) an einer Hilfsstoffkombination enthaltend zumindest ein 1,4 O-verknüpftes Saccharose-Derivat in Kombination mit zumindest einem weiteren Hilfsstoff und **b)** zumindest 0,01% (w/w), vorzugsweise 0,01 bis 75% (w/w) eines pharmazeutischen Wirkstoffs, vorzugsweise eines biologischen Makromoleküls, hergestellt und getrocknet, wobei die Summe der Gewichtsprozente maximal 100% (w/w), bezogen auf den Feststoffgehalt der Sprühlösung, beträgt. Nach einer bevorzugten Ausführungsform wird eine Lösung / Suspension mit einem Feststoffgehalt **a)** an einer Hilfsstoffkombination enthaltend zumindest ein 1,4 O-verknüpftes Saccharose-Derivat in Kombination mit zumindest einem weiteren Hilfsstoff von zumindest 60% (w/w), vorzugsweise zwischen 60 bis 90% (w/w), und **b)** 0,01 bis 40% (w/w) eines pharmazeutischen Wirkstoffs, vorzugsweise eines biologischen Makromoleküls, hergestellt und getrocknet, wobei die Summe der Gewichtsprozente der Lösung oder Suspension maximal 100% (w/w), bezogen auf den Feststoffanteil der Sprühlösung, beträgt.

Vorzugsweise enthält die zu trockenden Lösung / Suspension neben dem pharmazeutischen Wirkstoff und zumindest einem 1,4 O-verknüpften Saccharose-Derivat als weiteren Hilfsstoff eine Zuckermischung aus weiteren Mono-, Di- und/oder Oligosacchariden, und/oder eine oder mehrere Aminosäuren und/oder Peptide oder Proteine, wobei die als Hilfsstoff verwendeten Peptide oder Proteine nicht dem pharmazeutischen Wirkstoff entsprechen. Folglich betrifft die vorliegende Erfindung auch ein Verfahren zur Herstellung von Pulvern dadurch gekennzeichnet, dass die zu trocknende Lösung /Suspension in Bezug auf ihren Feststoffgehalt **a)** zumindest 25% (w/w), vorzugsweise zumindest 60% (w/w) an zumindest einem 1,4 O-verknüpften Saccharose-Derivat oder einer Zuckermischung, enthaltend zumindest ein 1,4 O-verknüpften Saccharose-Derivat, **b)** zwischen 1 und 39,99% (w/w) zumindest einer Aminosäure und/oder zumindest eines Peptids und c) zumindest 0,01% (w/w) eines pharmazeutischen Wirkstoffs enthält.

Nach einer weiter bevorzugten Ausführungsform enthält die zu trocknende Lösung /Suspension neben zumindest einem 1,4 O-verknüpften Saccharose-Derivat oder einer Zuckermischung, enthaltend zumindest ein 1,4 O-verknüpften Saccharose-Derivat, zusätzliche eine oder mehrere Aminosäuren als weiteren Hilfsstoff. Als vorteilhaft gelten Lösungen / Suspensionen deren Feststoffanteil **a)** zumindest 25% (w/w), vorzugsweise 60 bis 90% (w/w) an zumindest einem 1,4 O-verknüpften Saccharose-Derivat oder einer Zuckermischung, enthaltend zumindest ein 1,4 O-verknüpften Saccharose-Derivat, **b)** 1 bis 19,99% (w/w) Aminosäuren, **c)** sowie zumindest 0,01 % (w/w) eines pharmazeutischen Wirkstoffs, vorzugsweise eines Peptids/Proteins, wie zum Beispiel eines Antikörper, enthält. Der Anteil des pharmazeutischen Wirkstoffs beträgt hierbei vorzugsweise 0,01 bis maximal 74% (w/w) wobei die Summe aus den Feststoffanteilen maximal 100% (w/w) beträgt. Ein Fachmann ist hierbei in der Lage entsprechende Pulver herzustellen und die Gewichtsanteile so abzustimmen, dass die Summe aus den Feststoffanteilen 100% (w/w) nicht übersteigt. Soll der Anteil (bezogen auf den Gesamtfeststoffgehalt) an pharmazeutischen Wirkstoff beispielsweise 10% (w/w) betragen und der Anteil an zumindest einem 1,4 O-verknüpften Saccharose-Derivat oder einer Zuckermischung, enthaltend zumindest ein 1,4 O-verknüpften Saccharose-Derivat, 80% (w/w) so weiß der Fachmann, das er der Sprühlösung / Suspension maximal 10% (w/w) an Aminosäuren zugeben kann.

Nach einer weiter bevorzugten Ausführungsform enthält die zu trocknende Lösung /Suspension neben zumindest einem 1,4 O-verknüpften Saccharose-Derivat oder einer Zuckermischung enthaltend zumindest ein 1,4 O-verknüpftes Saccharose-Derivat, zusätzlich Isoleucin als weiteren Hilfsstoff. Als vorteilhaft gelten Lösungen / Suspensionen deren Feststoffanteil **a)** zumindest 25% (w/w), vorzugsweise 60 bis 90% (w/w) an zumindest einem 1,4 O-verknüpften Saccharose-Derivat oder einer Zuckermischung, enthaltend zumindest ein 1,4 O-verknüpften Saccharose-Derivat, **b)** 10 bis 19,99% (w/w) Isoleucin, c) sowie zumindest 0,01% (w/w) eines pharmazeutischen Wirkstoffs, vorzugsweise eines Peptids/Proteins, wie zum Beispiel eines Antikörpers, enthält. Der Anteil des pharmazeutischen Wirkstoffs beträgt hierbei vorzugsweise 0,01 bis maximal 65% (w/w) wobei die Summe aus den Feststoffanteilen maximal 100% (w/w) beträgt. Ein Fachmann ist hierbei in der Lage entsprechende Pulver herzustellen und die Gewichtsanteile so abzustimmen, dass die Summe aus den Feststoffanteilen 100% (w/w) nicht übersteigt. Soll der Anteil (bezogen auf den Gesamtfeststoffgehalt) an pharmazeutischen Wirkstoff beispielsweise 10% (w/w) betragen und der Anteil an dem 1,4 O-verknüpften Saccharose-Derivat oder einer Zuckermischung enthaltend zumindest ein 1,4 O-verknüpftes Saccharose-Derivat 80% (w/w), so weiß der Fachmann, das er der Sprühlösung / Suspension maximal 10% (w/w) an Isoleucin zugeben kann.

Nach einer weiteren Ausführungsform enthält die zu trocknende Lösung / Suspension neben zumindest einem 1,4 O-verknüpften Saccharose-Derivat oder einer Zuckermischung, enthaltend zumindest ein 1,4 O-verknüpften Saccharose-Derivat, ein oder mehrere Peptide, vorzugsweise Di- oder Tri-Peptide, beosnders bevorzugt Isoleucinhaltige Tri-Peptide, besonders bevorzugt Tri-Isoleucin. Als vorteilhaft gelten zu trocknende Lösungen oder Suspension deren Feststoffanteil **a)** zumindest 25% (w/w), vorzugsweise 60 bis 99% (w/w), besonders bevorzugt 60 bis 90% (w/w) an zumindest einem 1,4 O-verknüpften Saccharose-Derivat oder einer Zuckermischung, enthaltend zumindest ein 1,4 O-verknüpften Saccharose-Derivat, **b)** 1 bis 19,99% (w/w) eines Peptids, vorzugsweise eines Di- oder Tri-Peptids, besonders bevorzugt Tri-Isoleucin, und **c)** zumindest 0,01% (w/w) eines pharmazeutischen Wirkstoffs, vorzugsweise eines Peptids/Proteins wie zum Beispiel eines Antikörpers, enthält, wobei die Summe aus den Feststoffanteilen maximal 100% (w/w) beträgt. Der Anteil des pharmazeutischen Wirkstoffs beträgt hierbei vorzugsweise 0,01 bis maximal 74% (w/w). Ein Fachmann ist hierbei in der Lage entsprechende Pulver herzustellen und die Gewichtsanteile aufeinander abzustimmen, so dass die Summe aus den Feststoffanteilen 100% (w/w) nicht übersteigt. Soll der Anteil (bezogen auf den Gesamtfeststoffgehalt) an pharmazeutischen Wirkstoff beispielsweise 10% (w/w) betragen und der Anteil an zumindest einem 1,4 O-verknüpften Saccharose-Derivat oder einer Zuckermischung enthaltend zumindest ein 1,4 O-verknüpftes Saccharose-Derivat 80% (w/w) so weiß der Fachmann, das er der zu versprühenden Lösung oder Suspension maximal 10% (w/w) an Peptid, vorzugsweise an Di- oder Tri-Peptid, besondersbevorzugt an Tri-Isoleucin zugeben kann.

Wie bereits erwähnt ist es vorteilhaft zu versprühende Lösungen mit einem pH-Wert zwischen 3 und 11, vorzugsweise 3,5 und 9, besonders bevorzugt zwischen 4,0 und 7,8 herzustellen und zu versprühen. Geeignete Puffersysteme sind dem Fachmann bekannt. Üblicherweise erweist sich die Verwendung von anorganischen oder organischen Salzen als Puffersystem als besonders vorteilhaft.

Typischerweise wird der optimale Hilfsstoff- und Proteingehaltgehalt für jedes Protein oder Peptid experimentell bestimmt. Bevorzugte Formulierungen der Erfindung können noch mindestens einen weiteren Hilfsstoff enthalten, um Pulvereigenschaften wie Dispergierbarkeit und Fließfähigkeit unter Beibehaltung einer überlegenen Aggregathemmung zu verbessern.

### Sprühtrocknung:

Die Versprühung erfolgt in konventionellen Sprühtrocknern, beispielsweise in Geräten der Fa. Niro A/S (Soeborg, DK), Büchi Labortechnik GmbH (Flawil, CH) oder der gleichen. Die optimalen Bedingungen für die Sprühtrocknung hängen jeweils von der entsprechenden Formulierung ab und sind experimentell zu bestimmen. Als Gas wird typischerweise Luft verwendet, geeignet sind aber auch inerte Gase wie Stickstoff oder Argon. Darüber hinaus bestimmt sich die Sprühtrocknungstemperatur, gemeint sind Einström- (*inlet*-) und Ausströmtemperatur (*outlet*-temperature), nach der Temperatursensitivität des verwendeten Wirkstoffs, jeweils in Abhängigkeit der verwendeten Stabilisatoren. Üblich ist eine *inlet*-Temperatur von 50-200°C während die *outlet-*Temperatur zumeist 30-150°C beträgt. Im Rahmen der vorliegenden Erfindung wurde mit einer *inlet*-Temperatur von ungefähr 170-185°C und einer outlet-Temperatur von 80-100°C gearbeitet. Möglich ist allerdings auch eine *inlet-* Temperatur von bis zu 200°C, vorzugsweise 60-185°C und eine *outlet-* Temperatur von bis zu 120°C, vorzugsweise 40-105°C, je nach Stabilisatoranteil. Die Versprühung erfolgt in der Regel bei einem Druck von ungefähr 20-150 psi, vorzugsweise bei ungefähr 30- oder 40-100 psi, beispielsweise bei ungefähr 30, 40, 50, 60, 70, 80, 90 oder 100 psi.

In Bezug auf den Büchi Sprühtrockner B290 beträgt die "Liquid-Feed-Rate" normalerweise zwischen 0,1 und 100 ml/min, vorzugsweise zwischen 0,1 und 30 ml/min, beispielsweise ca. 3 ml/min. In diesem Zusammenhang hat sich eine Aspirator-Flow-Rate von 20-40 m³/h, vorzugsweise von 30-40 m³/h wie zum Beispiel 35 m³/h und eine Zerstäubungsflussrate von 0,3-2,5 m³/, vorzugsweise von 30-40 m³/h wie zum Beispiel 38,3 m³/h und Zerstäubungsflussraten von 0,3-2,5 m³/h, vorzugsweise von ca. 0,67 m³/h, 1,05 m³/h und 1,74 m³/h als besonders geeignet erwiesen.

Die sprühgetrockneten Pulver können optional einer zweiten schonenden Trocknung (Nachtrocknung) unterzogen werden. Ziel ist es einen einheitlichen Restwassergehalt der Pulver, bevorzugt kleiner 2% (w/w), zu erhalten, und damit sowohl die Wirkstoffstabilität als auch Pulvereigenschaften wie Glasübergangstemperatur, Fließfähigkeit und Dispergierbarkeit zu verbessern. Die Bedingungen des Nachtrocknungsprozesses müssen so gewählt sein, dass sich die Bioaktivität des pharmazeutischen Wirkstoffs nicht signifikant verringert. Die sprühgetrockneten Wirkstoff-Pulverformulierungen werden bevorzugt unter trockenen Bedingungen (bei geringer relativer Luftfeuchtigkeit) hergestellt, weiterverarbeitet und gelagert. Der Prozess der Nachtrocknung ermöglicht es, die Pulver trotz relativ hoher Ausgangsrestwassergehalte nach Sprühtrocknung noch weiter im Feuchtigkeitsgehalt zu reduzieren. Überraschenderweise stabilisieren die Hilfsstoffe, welche Gegenstand der Erfindung sind, in den bevorzugten Formulierungen die Proteine auch bei nicht optimalen Prozess- und Lagerbedingungen überlegen.

### Gefriertrocknung:

Die Gefriertrocknung wässriger Lösungen läßt sich gemäß der Beschreibung in Essig, Oschmann: "Lyophilisation", Wissenschaftliche Verlagsgesellschaft, Stuttgart (1993) durchführen. Der therapeutische Wirkstoff wird üblicherweise als wässrige Lösung oder Suspension gefriergetrocknet. Geeignete Konzentrationen und pH-Werte sind zu berücksichtigen. In einer bevorzugten Formulierung ist der pharmazeutische Wirkstoff zunächst in einer wässrigen Lösung mit einem geeigneten Puffersystem gelöst. Der pH-Wert der proteinhaltigen Lösungen liegt generell von 3 bis 11, bevorzugt von 3,5 bis 9 und besonders bevorzugt zwischen 4,0 und 8. Der pH-Wert der Lösung muss entweder unterhalb oder oberhalb des isoelektrischen Punktes des Wirkstoffs eingestellt werden. In einem zweiten Behältnis werden der Hilfsstoff oder eine Mischung aus geeigneten Hilfsstoffen in Reinstwasser oder einer geeigneten Pufferlösung mit einem pH-Wert von 3 bis 11, bevorzugt von 3,5 bis 9 und besonders bevorzugt von 4,0 bis 8,5 gelöst und in einem zweiten Schritt mit der Proteinlösung vermischt. Zuletzt wird die Lösung mit Reinstwasser oder einer geeigneten Pufferlösung mit einem pH-Wert von 3 bis 11, bevorzugt von 3,5 bis 9 und besonders bevorzugt von 4,0 bis 8,5 auf den gewünschten Feststoffgehalt eingestellt. Geeignete Gesamtfeststoffgehalte sind zwischen 0,1 bis 30% (w/w), bevorzugt 0,5 bis 20% (w/w) und besonders bevorzugt zwischen 0,75 bis 15,0% (w/w).

Die Lösungen werden dann in einem konventionellen handelsüblichen Gefriertrockner, wie Christ LPC-16/NT Epsilon 2-12 D der Firma Martin Christ Gefriertrocknungsanlagen GmbH o. a., gefriergetrocknet. Das Produkt ist ein proteinhaltiges Pulver oder Kuchen, welcher dann vor einer weiteren Verarbeitung mit einem geeigneten Verfahren zerkleinert wird, so dass man ein polydisperses Pulver erhält.

Die Temperaturen im Gefriertrockner werden experimentell optimiert und liegen generell zwischen -70°C und +100°C, bevorzugt zwischen -50°C und +40°C. Bevorzugte Parameter für Druck im Gefriertrockner sind von 10*e-5 bis 1013 mbar. Die gefriergetrockneten Proteinformulierungen können nach Ihrer Zerkleinerung bevorzugt einer zweiten schonenden Trocknung (Nachtrocknung) unterzogen werden. Ziel ist es einen einheitlichen Restwassergehalt der Formulierungen kleiner 2% beizubehalten, und damit sowohl die Wirkstoffstabilität als auch Pulvereigenschaften wie Glasübergangstemperatur, Fließfähigkeit und Dispergierbarkeit zu verbessern. Die Bedingungen des Nachtrocknungsprozesses müssen so gewählt sein, dass sich die Aktivität des Wirkstoffs nicht signifikant verringert.

### Eigenschaften der sprühgetrockneten trockenen Pulverformulierungen

Die im Rahmen dieser Erfindung hergestellten trockenen Protein-Pulverformulierungen haben einen Restwassergehalt von unter 15% (w/w), gewöhnlich von unter 10% (w/w), und bevorzugt von unter 6% (w/w). Weiter bevorzugt haben die sprühgetrockneten Protein-Pulverformulierungen einen Restwassergehalt von unter 5% (w/w), besonders bevorzugt von unter 3% (w/w) und am meisten bevorzugt von zwischen 0,2 und 2,0% (w/w). Formulierungen mit einer geringen Restfeuchte zeigen generell eine verbesserte Stabilität während des Abpackens und der Lagerung. Darüber hinaus sind die trockenen Protein-Pulverformulierungen der Erfindung vornehmlich hygroskopisch, d.h. sie neigen dazu Feuchtigkeit aus Ihrer Umgebung zu absorbieren. Um dies zu vermeiden werden solche Pulver für gewöhnlich unter Ausschluss von Luftfeuchtigkeit in Behältern wie Blisterpackungen gelagert. Überraschenderweise zeigte sich bei ausgewählten Fomulierungen der erfindungsgemäßen Pulver, dass die Pulver auch bei einem Monat offener Lagerung unter 43% relativer Luftfeuchtigkeit sowohl in Bezug auf Proteinstabilität als auch Inhalierbarkeit stabil bleiben.

Die stabilisierenden Effekte der hier beschriebenen Hilfsstoffe sind imstande das Protein vor den extremen Belastungen während der Sprühtrocknung sowie der Lagerung zu schützen. In Abwesenheit von Hilfsstoffen sprühgetrocknete pure Proteinformulierungen bilden in großem Ausmaß Aggregate. Prozessbedingte Faktoren wie Hitze, Scherstress und Denaturierung an den Luft-Wasser-Grenzflächen verursachen Aggregation (bis zu ca. 6,6 % Aggregate) während der Sprühtrocknung und angeschlossener Nachtrocknung (bis zu ca. 5,8 % Aggregate). Im Verlauf der Lagerung kommt es durch Abwesenheit der stabilisierenden Hydrathülle der Proteine zu massiver Aggregatbildung (von ca. 11,8 bis ca. 18,9 % Aggregate).

Die bevorzugten sprühgetrockneten Formulierungen der Erfindung sind im Gegensatz zu den puren Proteinformulierungen imstande, die Bildung von Aggregaten sowohl nach dem Sprühtrocknen zu verringern als auch bei unterschiedlichen Lagerbedingungen auf einem sehr geringen Niveau zu halten. Durch die Sprühtrocknung und angeschlossene Vakuumtrocknung bilden sich in den bevorzugten Formulierungen lediglich ca. 0,5 bis ca. 1,8 % Aggregate, im Gegensatz zu bis zu ca. 4,0 % Aggregate bei puren Proteinformulierungen.
Bei einer besonders herausfordernden Lagerungsbedingung (40°C, 75% relative Feuchte), der Forcierten Lagerstabilität, zeichnet sich eine deutliche Überlegenheit der bevorzugten Formulierungen (Aggregate von ca. 1,0 bis ca. 13,1%) gegenüber puren Proteinformulierungen ab (ca. 18,2 bis 18,9% Aggregate) sowie einer analogen Referenzformulierung mit Trehalose als Hilfsstoff ab.

Besonders augenscheinlich wird dieser Vorteil im Vergleich der in Beispiel 4 aufgeführten Formulierung. Der Zusatz an Tri-Isoleucin in der Sprühlösung führt zu einer signifikanten Verbesserung der aerodynamischen Eigenschaften der Pulver. Überraschenderweise sind lediglich die Kombinationen, die zumindest eine 1,4 O-verknüpftes Saccharose-Derivat und Tri-Isoleucin enthalten, insbesondere LS55P und Tri-Isoleucin sowie LS90P und Tri-Isoleucin imstande den pharmazeitischen Wirkstoff, vorliegend beispielhaft ein Protein, vor Aggregatbildung (nur 0,7 bis 4,4% Agregate) zu bewahren. Weder die in WO01/32144 beschriebenen in Kombination mit Tri-Leucin verwendeten Hilfsstoffe Raffinose (12,6% Aggregate) und Hydroxyethylstärke (ca. 18,6% Aggregate) noch die im Stand der Technik als überragender Stabilisator beschriebene Trehalose vermögen das Protein unter den besonders herausfordernden Bedingungen in Kombination mit Tri-Isoleucin vor Aggregation zu schützen. Sowohl die LS55P-Tri-Isoleucin-Formulierungen als auch LS90P-Tri-Isoleucin-Formulierungen zeigen sich auch deutlich vorteilhaft gegenüber einer Saccharose-Tri-Isoleucin-Formulierung (5,6% Aggregate) und Saccharose-Lactose-Tri-Isoleucin-Formulierung (8,8% Aggregate). Dies ist umso überraschender, als das in LS55P neben Saccharose auch bis zu 25% Lactose in vorhanden ist. Es ist offensichtlich, dass der negative Effekt den der reduzierenden Zucker Lactose auf die Proteinstabilität hat im LS55P durch die enthaltene Lactosucrose überkompensiert wird. Ein höherer Anteil Lactosucrose an dem Zuckeranteil der Pulverformulierungen ist noch vorteilhafter für die Proteinstabilität (siehe LS90P-Formulierungen).

Formulierungen, welche bereits bei relativ kurzer Lagerung unter besonders destabilisierenden Bedingungen (1 Woche bei 40°C, 75% relative Feuchte) einen signifikant stabilisierenden Effekt auf die inkorporierten Proteine haben, stabilisieren Proteine auch auf lange Zeit unter weitaus milderen Standardlagerbedingungen (z.B. 1 Jahr trocken, ca. 25°C).

Nach Equlibrierung mit angeschlossener vierwöchiger Lagerung unter trockenen Bedingungen bei 40°C (Equilibrierte Lagerstabilität) zeichnen sich die LS55P und Coupling Sugar enthaltende Pulverformulierungen durch geringe Aggregatgehalte aus (ca. 1,4 bis 3,2% Aggregate), besonders im Vergleich zu puren Proteinpulvern (ca. 11,8% Aggregate).

Nach Vakuumtrocknung mit angeschlossener vierwöchiger Lagerung unter trockenen Bedingungen bei 40°C (Vakuumgetrocknete Lagerstabilität) zeichnen sich die LS55P und Coupling Sugar enthaltende Pulverformulierungen durch geringe Aggregatgehalte aus (ca. 1,1 bis 2,1% Aggregate), besonders im Vergelich zu puren Proteinpulvern (ca. 13,2% Aggregate).

LS55P (80%), Isoleucin (10%) und IgG1 (10%) Formulierungen mit einer Feinpartikelfraktion von ca. 35% zeigen nach Vakuumtrocknung mit angeschlossener Abfüllung unter Stickstoff nach dreimonatiger Lagerung unter trockenen Bedingungen bei 2 bis 8°C, 25°C und 40°C Aggregatgehalte unter 1,9%.

LS55P (80%), Tri-Isoleucin (10%) und lgG1 (10%) Formulierungen mit einem MMAD von ca. 3,9 µm und einer Feinpartikelfraktion von 58,3% nach Sprühtrocknung zeigen nach Vakuumtrocknung mit angeschlossener Abfüllung unter Stickstoff nach dreimonatiger Lagerung unter trockenen Bedingungen bei 2 bis 8°C und 25°C Aggregatgehalte unter 1,9% und bei trockenen Lagerbedingungen bei 40°C (3 Monatsstabilität) zeigen sie Aggregatgehalte unter 2,6%.

Weiterhin zeigen die oben genannten LS55P (80%), Tri-Isoleucin (10%) und IgG1 (10%) Formulierungen nach einmonatiger offener Lagerung bei ca. 43% relativer Luftfeuchte und 25°C (offene 1 Monatsstabilität) weiterhin geringen Aggregatgehalt (ca 1,3%) bei annähernd gleichen geringem MMAD (ca. 3,8 µm) und gleich hoher Feinpartikelfraktion (ca. 59,6%).

LS90P (90%) und IgG1 (10%) Formulierungen mit einem MMAD von ca. 3,8 µm, einem MMD von ca. 2,8 µm und einer Feinpartikelfraktion von ca. 24% nach Sprühtrocknung zeigen nach Vakuumtrocknung mit angeschlossener Abfüllung unter Stickstoff nach ein- bzw. dreimonatiger Lagerung unter trockenen Bedingungen bei 2 bis 8°C, 25°C und 40°C (1 bzw. 3 Monatsstabilität) Aggregatgehalte unter 1, 2 bzw.2,2%.

LS90P (80%), Isoleucin (10%) und IgG1 (10%) Formulierungen mit einer Feinpartikelfraktion von ca. 28% zeigen nach Vakuumtrocknung mit angeschlossener Abfüllung unter Stickstoff nach ein- bzw.dreimonatiger Lagerung unter trockenen Bedingungen bei 2 bis 8°C, 25°C und 40°C (1 bzw. 3 Monatsstabilität) Aggregatgehalte unter 0,9 bzw. 1,1%.

LS90P (80%), Tri-Isoleucin (10%) und IgG1 (10%) Formulierungen mit einem MMAD von ca. 4,8 µm und einer Feinpartikelfraktion von 53,2% nach Sprühtrocknung zeigen nach Vakuumtrocknung mit angeschlossener Abfüllung unter Stickstoff nach ein- bzw. dreimonatiger Lagerung unter trockenen Bedingungen bei 2 bis 8°C, 25°C und 40°C (1 bzw. 3 Monatsstabilität) Aggregatgehalte unter 1, 2 bzw. 2,3%.

Weiterhin zeigen Variationen der oben genannten LS90P (80%), Tri-Isoleucin (10%) und IgG1 (10%) Formulierungen nach ein- bzw. dreimonatiger offener Lagerung bei ca. 43% relativer Luftfeuchte und 25°C (offene 1 bzw. 3 Monatsstabilität) weiterhin geringe Aggregatgehalte zwischen ca 0,5% und 0,8%. Nach Sprühtrocknung liegen die MMADs zwischen ca. 3,9 und 3,3 µm und die FPFs zwischen ca. 55,6 und 58,9%. Nach einmonatiger offener Lagerung bei ca. 43% relativer Luftfeuchte bei 25°C zeigen die oben genannten Formulierungen weiterhin kleine MMADs (ca. 4,1 bis 3,5 *µ*m) und eine hohe Feinpartikelfraktion (ca. 62,3 bis 67,3%).

Durch Variation der Sprühtrocknungsbedingungen lassen sich Pulver herstellen, die vorzugsweise über eine mittlere Teilchengröße (MMD) von weniger als 20 *µ*m, vorzugsweise von weniger als 10 *µ*m verfügen. Nach einer besonders bevorzugten Ausführungsform verfügen diese erfindungsgemäßen Partikel über einen mittlere Teilchengröße von weniger als 7,5 *µ*m, vorzugsweise von weniger als 5 *µ*m. Besonders bevorzugt sind Partikel mit einem mittlere Teilchengröße von weniger als 4 *µ*m und weiter bevorzugt von weniger als 3,5 *µ*m. Im Allgemeinen lassen sich auch Partikel mit einen mittleren Teilchendurchmesser von 0,1-5 *µ*m, vorzugsweise von 0,2-4 *µ*m herstellen. In einer weiteren Ausführungsform werden den entsprechenden Pulvern nichtrespirierbare Teilchen, z.B. Lactose, mit einer Teilchengröße von zumindest 40 *µ*m, vorzugsweise zwischen 40 und 200 *µ*m, zugemischt. Der Anteil beträgt vorzugsweise zumindest 15%, weiter bevorzugt zumindest 20%, noch weiter bevorzugt zumindest 30%, noch weiter bevorzugt zumindest 40% und besonders bevorzugt zumindest 50 oder 60%.

Neben der mittleren Teilchengröße (MMD) hängt die Inhalierbarkeit im wesentlichen von dem mittleren aerodynamischen Teilchendurchmesser (MMAD) ab. Die erfindungsgemäßen Partikel verfügen vorzugsweise über einen MMAD von kleiner 10 *µ*m und weiter bevorzugt von weniger als 7,5 *µ*m. Besonders vorteilhaft sind Pulver bestehend aus Partikeln mit einem MMAD von kleiner 5,5 µm, vorzugsweise von kleiner 5 *µ*m, noch weiter bevorzugt von kleiner 4,5 *µ*m. Die in den Beispielen beschriebenen Pulver lassen sich mit entsprechenden Partikelgrößen durch die Kombination aus optimaler Sprühtrocknungsbedingung und der erfindungsgemäßen Wahl und Konzentration der Hilfsstoffe herstellen. Insbesondere die Beimischung von Aminosäuren und/oder Tri-Peptiden führt zu einer verbesserten Partikelperfomance mit einem erhöhten Anteil an inhalierbaren Partikeln mit einem MMAD von kleiner 7,5 µm, vorzugsweise von kleiner 5,5 µm. Durch Zugaben von Isoleucin oder Tri-Isoleucin konnten inhalierbare Pulver mit einer FPF von größer 28%, vorzugsweise von größer 40, weiter bevorzugt von größer 50 und noch weiter bevorzugt von größer 55% hergestellt werden (siehe BEISPIELE).

Die erfindungsgemäßen Pulver zeichnen sich darüber hinaus durch eine Glasübergangstemperatur von zumindest 40°C, vorzugsweise von zumindest 50°C, weiter bevorzugt von zumindest 55°C, noch weiter bevorzugt von zumindest 60°C aus. Besonders bevorzugte Pulver weisen eine Glasübergangstemperatur von zumindest 65°C auf. Im Allgemeinen beträgt die Glasübergangstemperatur der erfindungsgemäßen Pulver 40 bis 110°C. Demzufolge betrifft die vorliegende Erfindung auch Pulver, vorzugsweise sprühgetrocknete Pulver, enthaltend einen pharmazeutischen Wirkstoff und LS90P, LS55P, Coupling Sugar oder Coupling Sugar S, wobei die Glasübergangstemperatur 40°C und mehr, vorzugsweise zwischen 45 und 60°C oder höher beträgt. Nach einer weiter bevorzugten Ausführungsform beträgt die Glasübergangstemperatur 55°C und mehr, vorzugsweise zwischen 55 und 60°C oder höher.

### Gefriergetrocknete Pulver:

Pulver lassen sich auch alternativ durch Gefriertrocknung mit angeschlossener Pulverisierung herstellen (siehe Beispiele). In den hier abgebildeten Beispielen erfolgte die Pulverisierung denkbar einfachst durch einen Spatel in den Lyophilisationsvials. Das Lyophilsat kann selbstverständlich auch durch geignetete Mühlen wie eine Schneidemühle, Kugelmühle, Stiftsmühle, Mörsermühle, Luftstrahlmühle oder andere geeignete Verfahren pulverisiert werden (siehe Bauer, Frömming, Führer, 6. Auflage).

### Eigenschaften der gefriergetrockneten Pulver:

Die im Rahmen dieser Erfindung hergestellten trockenen Protein-Pulverformulierungen haben einen Restwassergehalt von unter 15% (w/w), gewöhnlich von unter 10% (w/w), und bevorzugt von unter 5% (w/w). Weiter bevorzugt haben die sprühgetrockneten Protein-Pulverformulierungen einen Restwassergehalt von unter 3% (w/w), besonders bevorzugt von unter 2% (w/w) und am meisten bevorzugt von zwischen 0,2 und 1,5% (w/w). Formulierungen mit einer geringen Restfeuchte zeigen generell eine verbesserte Stabilität während des Abpackens und der Lagerung. Darüber hinaus sind die trockenen Protein-Pulverformulierungen der Erfindung vornehmlich hygroskopisch, d.h. sie neigen dazu Feuchtigkeit aus Ihrer Umgebung zu absorbieren. Um dies zu vermeiden werden solche Pulver für gewöhnlich unter Ausschluss von Luftfeuchtigkeit in Behältern wie Blisterpackungen gelagert.

Die stabilisierenden Effekte der hier beschriebenen Hilfsstoffe sind imstande das Protein vor den extremen Belastungen während der Gefriertrocknung sowie der Lagerung zu schützen. In Abwesenheit von Hilfsstoffen gefriergetrocknete pure Proteinformulierungen bilden in großem Ausmaß Aggregate. Prozessbedingte Faktoren wie Einfriertstress, Aufkonzentrierung, pH-Shift und Denaturierung an den Luft-Wasser-Grenzflächen verursachen Aggregation (bis zu ca. 2,1% Aggregate) während der Gefriertrocknung. Im Verlauf der Lagerung kommt es durch Abwesenheit der stabilisierenden Hydrathülle der Proteine zu massiver Aggregatbildung (20,5 % Aggregate).

Die bevorzugten gefriergetrockneten Formulierungen der Erfindung sind im Gegensatz zu den puren Proteinformulierungen imstande, die Bildung von Aggregaten sowohl nach dem Gefriertrocknen zu verringern als auch bei unterschiedlichen Lagerbedingungen auf einem sehr geringen Niveau zu halten. Die Gefriergetrockneten und Pulversisierten Lysophilsate zeichnen sich bei besonders herausfordernden Lagerungsbedingung (40°C, 75% relative Feuchte), der Forcierten Lagerstabilität, durch eine deutliche Überlegenheit der bevorzugten Formulierungen (Aggregate von ca. 1,2 bis ca. 1,5%) gegenüber puren Proteinformulierungen ab (ca. 14,5% Aggregate) sowie einer analogen Referenzformulierung mit Mannitol (ca. 34,0% Aggregate) als Hilfsstoff aus.

Formulierungen, welche bereits bei relativ kurzer Lagerung unter besonders destabilisierenden Bedingungen (1 Woche bei 40°C, 75% relative Feuchte) einen signifikant stabilisierenden Effekt auf die inkorporierten Proteine haben, stabilisieren Proteine auch auf lange Zeit unter weitaus milderen Standardlagerbedingungen (z.B. 1 Jahr trocken, ca. 25°C).

Nach Gefrietrocknung, Pulversierung und Equlibrierung mit angeschlossener vierwöchiger Lagerung unter trockenen Bedingungen bei 40°C (Equilibrierte Lagerstabilität) zeichnen sich die LS55P und Coupling Sugar enthaltende Pulverformulierungen durch geringe Aggregatgehalte aus (ca. 2,6 und 4,6% Aggregate), besonders in Relation zu puren Proteinpulvern (ca. 15,3% Aggregate) sowie einer analogen Referenzformulierung mit Mannitol (ca. 11,6% Aggregate) als Hilfsstoff aus.

Nach Gefrietrocknung, Pulverisierung und Vakuumtrocknung mit angeschlossener vierwöchiger Lagerung unter trockenen Bedingungen bei 40°C (Vakuumgetrocknete Lagerstabilität) zeichnen sich die LS55P und Coupling Sugar enthaltende Pulverformulierungen durch geringe Aggregatgehalte aus (ca. 1,2 und 1,5% Aggregate), besonders in Relation zu puren Proteinpulvern (ca. 14,5% Aggregate) sowie einer analogen Referenzformulierung mit Mannitol (ca. 6,2% Aggregate) als Hilfsstoff aus.

Die erfindungsgemäßen Pulver zeichnen sich darüber hinaus durch eine Glasübergangstemperatur von zumindest 40°C, vorzugsweise von zumindest 50°C, weiter bevorzugt von zumindest 55°C. Im Allgemeinen beträgt die Glasübergangstemperatur der erfindungsgemäßen Pulver 40 bis 110°C, kann im Einzelfall diesen Wert aber auch übersteigen. Demzufolge betrifft die vorliegende Erfindung auch Pulver, vorzugsweise gefriergetrocknete und pulverisierte Pulver, enthaltend einen pharmazeutischen Wirkstoff und LS90P, LS55P, Coupling Sugar oder Coupling Sugar S, wobei die Glasübergangstemperatur 40°C und mehr, vorzugsweise zwischen 45 und 60 C oder höher beträgt. Nach einer weiter bevorzugten Ausführungsform beträgt die Glasübergangstemperatur 55°C und mehr, vorzugsweise zwischen 55 und 60°C oder bis zu 110°C.

### Verwendung der getrockneten Pulvers

Die erfindungsgemäßen Pulver eignen sich zur Herstellung eines Arzneimittels, vorzugsweise zur Herstellung eines inhalativen Arzneimittels.

### Verabreichung der erfindungsgemäßen Pulver

Grundsätzlich lassen sich die erfindungsgemäßen Pulverzubereitungen direkt als trockenes Pulver über sog. Trockenpulver-Inhalatoren, oder aber nach Suspension oder Rekonstitution in Form von Aerosolen über sog. Vernebler applizieren. Die erfindungsgemäßen Inhalationspulver können dabei mittels aus dem Stand der Technik bekannten Inhalatoren appliziert werden.

Erfindungsgemäße Inhalationspulver können beispielsweise mittels Inhalatoren appliziert werden, die eine einzelne Dosis aus einem Vorrat mittels einer Messkammer, wie er in der US 4,570,630A beschrieben wird, oder über andere apparative Vorrichtungen, wie sie in der DE 36 25 685 A beschrieben werden, appliziert werden. Vorzugsweise werden die erfindungsgemäßen Inhalationspulver in Kapseln abgefüllt (zu sogenannten Inhaletten), die in Inhalatoren, wie beispielsweise in der WO 94/28958 beschrieben, zur Anwendung gelangen.

Weitere Beispiele für geeignete Inhalatoren finden sich u.a. in US 5,458,135; US 5,785,049 oder WO 01/00263. Weitere geeignete Inhalatoren sind aus der WO 97/41031; US 3,906,950 und US 4,013,075 bekannt. Weitere Dispersionsinhalatoren für trockene Pulverzubereitungen sind in EP 129 985; EP 472 598; EP 467 172 und US 5,522,385 beschrieben.

Die erfindungsgemäßen Inhalationspulver können beispielsweise mittels des unter dem Namen Turbuhaler® (AstraZeneca LP) bekannten Inhalators beziehungsweise mit Inhalatoren wie sie beispielsweise in der EP 237 507 A offenbart werden, appliziert werden. Andere geeignete Inhalatoren sind der Rotahaler® oder der Discus® (beide von GlaxoSmithKline Corp.), der Spiros^{™} Inhaler (Dura Pharmaceuticals) und der Spinhaler® (Fiscon).

Ein zur Anwendung der erfindungsgemäßen Arzneimittelkombination in Inhaletten besonders bevorzugter Inhalator ist der Abbildung 24 zu entnehmen. Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Sollen die erfindungsgemäßen Inhalationspulver im Sinne der vorstehend genannten bevorzugten Anwendung in Kapseln (Inhaletten) abgefüllt werden, bieten sich Füllmengen von 1 bis 30mg pro Kapsel an.

Die erfindungsgemäßen Pulver können ferner als treibgashaltige oder treibgasfreie Inhalationsaerosole appliziert werden. Hierzu werden die erfindungsgemäßen Pulver in druckverflüssigbaren Lösungsmitteln oder Lösungsmittelgemischen suspendiert oder in einer wässrigen Lösung rekonstituiert. Geeignete Suspensionen oder Lösungen sind im Stand der Technik bekannt. Vorteilhaft ist beispielsweise die Rekonstitution in physiologischen Lösungen mit einem pH von 3-11, vorzugsweise von 4-9. Besonders vorteilhaft ist die Rekonstitution in einer wässrigen Lösung mit einem pH von 5,5-7,8. Die treibgashaltigen Suspensionen oder Lösungen zur Rekonstitution der erfindungsgemäßen Pulver können ferner weitere Hilfsstoffe in Form von Stabilisatoren, Emulgatoren oberflächenaktiven Substanzen, wasserlöslichen organischen Lösungsmitteln enthalten. Entsprechende Substanzen sind dem Fachmann bekannt, und beispielhaft in (Bauer, Lehrbuch der Pharmazeutischen Technologie, Wissenschaftl. Verlagsgesellschaft mbH, Stuttgart, 178-184; Adler, 1998, Journal of Pharmaceutical Sciences, 88(2), 199-208) beschrieben. Entsprechende Inhalationsaerosole, die durch Suspendieren oder Rekonstitution der erfindungsgemäßen Pulver hergestellt werden, sind ebenfalls Gegenstand der vorliegenden Erfindung.

Die zur Herstellung der erfindungsgemäßen Inhalationsaerosole einsetzbaren Treibgase sind ebenfalls aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt chlorierten und fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG11, TG12, TG134a (1,1,1,2-Tetrafluorethan), TG227 (1,1,1,2,3,3,3-Heptafluorpropan) und Mischungen derselben, wobei die Treibgase TG 134a, TG227 und Gemische derselben bevorzugt sind.

Die erfindungsgemäßen treibgashaltigen Inhalationsaerosole können bis zu 5% (w/w) an Wirkstoff enthalten. Erfindungsgemäße Aerosole enthalten beispielsweise 0,002-5% (w/w), 0,01-3% (w/w), 0,015-2% (w/w), 0,1-2% (w/w), 0,5-2% (w/w) oder 0,5-1 % (w/w) an dem pharmazeutischen Wirkstoff. Inhaltationsaerosole mit einer entsprechenden Wirkstoffkonzentration lassen sich durch gezielte Rekonstitution der erfindungsgemäßen Pulver in einer entsprechenden Menge an Lösungsmittel einstellen.

Die vorstehend genannten erfindungsgemäßen treibgashaltigen Inhalationaerosole können mittels im Stand der Technik bekannten Inhalatoren (MDls = metered dose inhalers) appliziert werden. Beispielhaft sei hier auf den Ventolin® (Ventolin Pharmacy) oder die in US 5,32,094 oder US 5,672,581 beschriebenen Inhalatoren verwiesen. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, dass sie vorstehend beschriebene erfindungsgemäße treibgashaltige Aerosole enthalten.

Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.

Die erfindungsgemäßen Pulver können ferner in treibgasfreien Inhalationslösungen oder Suspensionen rekonstituiert werden. Entsprechende treibgasfreie Inhalationslösungen enthalten beispielsweise wässrige oder alkoholische, bevorzugt ethanolische, gegebenenfalls ethanolische im Gemisch mit wässrigen Lösungsmitteln. Im Falle wässrig/ethanolischer Lösungsmittelgemische ist der relative Anteil an Ethanol gegenüber Wasser nicht begrenzt, bevorzugt liegt die maximale Grenze jedoch bei bis 70% (v/v), insbesondere bei bis zu 60% (v/v) Ethanol. Die restlichen Volumenprozente werden von Wasser aufgefüllt. Den erfindungsgemäßen treibgasfreien Inhalationslösungen können Co-Solventien und/oder weitere Hilfsstoffe, wie oben beschrieben, zugesetzt werden. Beispielsweise lassen sich Co-Solventien verwenden, die Hydroxylgruppen oder andere polare Gruppen enthalten, wie z.B. Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen neben den oben aufgeführten z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmacksstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten. Demnach umfasst die vorliegende Erfindung auch treibgasefreie Inhaltationsaerosole, die durch Rekonstituion der erfindungsgemäßen Pulver hergestellt werden.

Zur Applikation der erfindungsgemäßen treibgasfreien Inhalationslösungen sind besonders solche Inhalatoren geeignet, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 *µ*L, bevorzugt weniger als 50 *µ*L, besonders bevorzugt zwischen 10 und 30 *µ*L Wirkstofflösung mit bevorzugt einem Hub zu einem Aerosol mit einer durchschnittlichen Teilchengröße von weniger als 20 µm, bevorzugt weniger als 10 *µ*m, so vernebelt werden können, so dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere in den Figuren 6a und 6b) ausführlich beschrieben. Auf die entsprechenden Figuren 6a und 6b der WO 97/12687 einschließlich der dazugehörigen Beschreibungsteile wird im Rahmen der vorliegenden Erfindung ausdrücklich Bezug genommen. Die dort beschriebenen Vernebler (Devices) sind auch unter der Bezeichnung Respimat® (Boehringer Ingelheim Pharma) bekannt. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so dass inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird im Rahmen der vorliegenden Erfindung auf die Figuren 1-4 - insbesondere auf Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO 94/07607 offenbart; auf diese Schrift wird hiermit inhaltlich Bezug genommen, insbesondere auf die dort offenbarte Figur 1 und deren Beschreibung. Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlassseite mit der Düsenauslassseite verbinden. Auf der Düsenauslassseite ist mindestens eine runde oder nicht-runde Öffnung von 2-10 µm Tiefe und 5-15 *µ*m Breite, wobei die Tiefe bevorzugt bei 4, 5 bis 6,5 Mikrometern und die Länge bei 7-9 Mikrometern beträgt. Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslassseite können die Strahlrichtungen mit einem Winkel von 20-160 Grad gegeneinander geneigt sein, bevorzugt wird ein Winkel von 60-150 Grad, insbesondere bevorzugt 80-100°. Die Düsenöffnungen sind bevorzugt in einer Entfernung von 10-200 µm angeordnet, stärker bevorzugt in einer Entfernung von 10-100 µm, besonders bevorzugt 30-70 µm. Am stärksten bevorzugt sind 50 µm.

Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 µm, bevorzugt bei 3-10 µm.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687 einschließlich der dazugehörigen Beschreibung, auf die an dieser Stelle nochmals inhaltlich Bezug genommen wird, ist ein entsprechender Vernebler (Respimat®) beschrieben. Dieser eignet sich insbesondere zur Applikation der erfindungsgemäßen treibgasfreien Inhalationsaerosole.

Figur 6a zeigt der WO97/12687 zeigt einen Längsschnitt durch den Zerstäuber bei gespannter Feder, Figur 6b der WO97/12687 zeigt einen Längsschnitt durch den Zerstäuber bei entspannter Feder: Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen. Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht. In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inhalatoren, beispielsweise Jet-Stream-Inhalatoren oder anderen stationären Verneblern vernebelt werden.

Dementsprechend betrifft ein weiterer Aspekt der vorliegenden Erfindung Arzneimittel in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat®. Bevorzugt zielt die vorliegende Erfindung auf treibgasfreie Inhalationslösungen oder Suspensionen, enthaltend eines der erfindungsgemäßen Pulver, in Verbindung mit der unter der Bezeichnung Respimat® bekannten Vorrichtung. Ferner betrifft die vorliegende Erfindung vorstehend genannte Vorrichtungen zur Inhalation, bevorzugt den Respimat®, dadurch gekennzeichnet, dass sie vorstehend beschriebene erfindungsgemäße treibgasfreie Inhalationslösungen oder Suspensionen enthalten.

Erfindungsgemäß bevorzugt sind Inhalationslösungen, enthaltend eines der hier beschriebenen erfindungsgemäßen Pulver, in einer einzigen Darreichungsform enthalten.

Die erfindungsgemäßen treibgasfreien Inhalationslösungen oder Suspensionen können neben den vorstehend, zur Applikation im Respimat® vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, dadurch gekennzeichnet, dass es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Weitere geeignete Vernebler für die inhalative Applikation von rekonstituierten Aerosolen sind der AERx^{™} (Aradigm), der Ultravent® (Mallinkrodt) und der Aconll® (Maquest Medical Products).

### Ausführungsbeispiele:

### Material und Methoden

### Materialien

Als IgG1 wurde ein humanisierter monoklonaler Antikörper mit einem Molekulargewicht von ca. 148 kDa von Boehringer Ingelheim, Deutschland, verwendet. Der Antikörper ist von einem murinen Antikörper abgeleitet, in dem die komplementärbestimmenden Regionen des murinen Antikörpers auf ein humanes Immunglobulingerüst übertragen wurden. Es entstand ein chimärer Antikörper mit 95% humanen und 5% murinen Anteil. Der Antikörper wird aus murinen Myelomazelllinien exprimiert. Die Zellen werden mit Hilfe von Tangential Fluss Microflitration entfernt und die zellfreie Lösung durch verschiedene Chromatographiemethoden aufgereinigt. Weitere Schritte umfassen eine Nucleasebehandlung, Behandlung bei niedrigem pH-Wert und eine Nanofiltration. Die Antikörper enthaltende Bulklösung enthält Histidin 25 mM und Glycin 1,6 mM als Puffer und wurde für die Herstellung der Lösung zur Sprühtrocknung mittels Diafiltration auf ca. 100mg/ml aufkonzentriert. Der Bulk für die Herstellung der zu versprühenden Lösung besaß 0,4 bis 0,8% Aggregate. Das Fertigarzneimittel ist bei 2-8°C mindestens 2 Jahre haltbar. Nyuka-Oligo® LS55P, Nyuka-Oligo® LS90P, Coupling Sugar® und Coupling Sugar S® wurden von Hayashibara Shoji, Inc., Japan, bezogen. Saccharose, Lactose, Mannitol, Raffinose, Hydroxyethylstärke und L-Isoleucin wurden von Sigma-Aldrich Chemie GmbH, Deutschland, bezogen. Trehalose stammt von der Georg Breuer GmbH, Deutschland. Tri-Isoleucin wurde von Iris Biotech GmbH, Deutschland bezogen.
Hühnereiweiss-Lysozym (Lysozym), 135500 U/mg, wurde von SERVA Electrophoresis GmbH, Deutschland bezogen. Synthetisches Lachscalcitonin (Calcitonin) wurde von Biotrend Chemikalien GmbH, Deutschland bezogen.

### Sprühtrocknung mit Büchi B-290

Die Sprühtrocknung wurde mit Hilfe eines Büchi Mini Spray Dryer B-290 der Firma Büchi Labortechnik GmbH durchgeführt. Die Sprühtrocknung der Formulierungen wurden prinzipiell gemäß der Beschreibung in "Spray Drying Handbook", 5th Edition., K. Masters, John Wiley and Sons , Inc., NY, NY (1991) ausgeführt:
Der Sprühtrockner ist aus einem Heizsystem, einem Filter, einem Aspirator, einem Trocknungsturm, einem Zyklon, Temperatursensoren zur Messung der Einlass- und der Auslasstemperatur und einem Auffanggefäß aufgebaut. Mit Hilfe einer peristaltischen Pumpe wird die zu versprühende Lösung in eine Zwei-Stoffdüse gepumpt. Dort findet mittels Druckluft die Zerstäubung der Lösung in kleine Tropfen statt. Die Trocknung erfolgt im Sprühturm durch erwärmte Luft, die im Gleichstromverfahren mit Hilfe des Aspirators durch den Sprühturm gesaugt wird. Das Produkt wird im Auffanggefäß nach Passieren des Zyklons aufgefangen.
Es wurden zwei verschieden Zyklone verwendet:
   - Zyklon I: Büchi Cyclone (Produktnummer 4189)
   - Zyklon II: Büchi High-performance Cyclone (Produktnummer 46369)

Der Feststoffanteil in den versprühten Lösungen betrug 10% (w/v) 3,33% und 2,00% in 50 bis 600ml. Die Einlasstemperatur betrug ca. 170 bis 185°C, die Liquid-Feed-Rate ca. 3 bis 3,33 ml/min, die Aspirator-Flow-Rate ca. 36,8 bis 38,3 m³/h und die Zerstäubungsflussrate (AAF = Atomizing Air Flow) ca. 0,67 m³/h, 1,05 m³/h und 1,74 m³/h. Daraus ergibt sich eine Auslasstemperatur von ca. 80-95°C.

### Gefriertrocknung:

Die Gefriertrocknung wurde mit Hilfe einer Gefriertrocknungsanlage Christ LPC-16/NT Epsilon 2-12 D der Firma Martin Christ Gefriertrocknungsanlagen GmbH durchgeführt.
Die Gefriertrocknungsanlage besteht aus der Trocknungskammer, einem Kondensator zum Abscheiden des sublimierten Lösungsmittels, einer Pumpe zum Erzeugen des Vakuums und der elektrischen Ausrüstung. Die Trocknung wird über die Stellflächentemperatur und das Vakuum der Trockenkammer gesteuert.
Der Feststoffanteil der Gefriertrocknungslösung betrug 5% (w/v). Die Lösung wurde in 2R Vials zu je 0,5 ml portioniert und mit gängigen Gefriertrockungsstopfen in die Gefriertrockungsanlage positioniert. Als erstes wurden die Lösungen in 30 Minuten bei -40°C eingefroren. Als zweites erfolgte die Haupttrocknung bei 0,11 mbar in drei Schritten. Zuerst 30 Stunden bei -40°C, dann 8 Stunden bei -30°C und zuletzt 8 Stunden bei -16°C. Der nächste Schritt war die Nachtrocknung und erfolgte 20 Stunden lang bei 20°C und 0,001 mbar. Als letztes wurden die Vials automatisch mit den zu Beginn nur aufgesetzten Gefriertrocknungstopfen verschlossen. Die so erhaltenen Lyophilsate sind mittels eines Spatels innerhalb der Vials pulverisiert worden.

### Röntgendiftraktometrie (Weitwinkelröntgendiffraktometrie (WAXS)):

Um die Kristallinität der getrockneten Proben zu bestimmen, wurden die Proben mit einem Seifert, X- Ray Diffractometer XRD 3000 TT (Fa. Seifert, Ahrensburg, DE) in einem auf 22 °C temperierten Raum untersucht. Die Röntgenröhre Cu- Anode, Cu-Kα-Strahlung mit λ = 0,15418 mm (Primärfilter Ni), wurde mit einer Anodenspannung von 40 kV und einer Stromstärke von 30 mA betrieben. Nach Positionieren des Probentellers im Gerät wurde die Probe im Bereich von 5 bis 40° mit einer Scanrate von 2 θ = 0,05° mit 2 sec Messdauer bei jedem Winkel vermessen.
Die Pulverdiffraktogramme wurden mit der ScanX- Rayflex Applikation, Version 3.07 device XRD 3000 (Scan), bzw. der Rayflex Version 2.1, 1996 (Analyse) am Detektor SC 1000 V aufgenommen.

### Größenausschlusschromatographie (SEC-HPLC)

### a) IgG 1-Proteinaggregate

Um IgG1-Proteinaggregate in den rekonstituierten Pulvern zu quantifizieren wurde eine SEC-HPLC durchgeführt. Die SEC-HPLC wurde mit einer HP1090 der Firma Agilent durchgeführt. Zur Trennung wurde eine TSK3000SWXL Säule (300 x 7.8mm) der Fa. Tosoh Biosep (Tosoh Bioscience, Stuttgart, Deutschland) verwendet. Als Fließmittel wurde ein Puffer aus 0.1 M di-Natriumhydrogenphosphat-Dihydrat, 0.1 M Natriumsulfat entwässert und wurde mit ortho-Phosphorsäure 85% auf pH 6.8 eingestellt. Die aufgegebene Probenmenge betrug 25µl bei einer Proteinkonzentration von 2-10 mg/ml. Der Nachweis des Proteins erfolgte mit Hilfe eines Dioden-Array Detektors der Firma Agilent bei 280nm. Für die Auswertung der Chromatogramme wurde die Software HP-Chemstation der Firma Agilent verwendet.

### b) Calcitonin-Proteinaggregate

Um Calcitonin-Proteinaggregate in den rekonstituierten Pulvern zu quantifizieren wurde eine SEC-HPLC durchgeführt. Die SEC-HPLC wurde mit einer HP1100 der Firma Agilent durchgeführt. Zur Trennung wurde eine TSK2000SWXL Säule (300 x 7.8mm) der Fa. Tosoh Biosep (Tosoh Bioscience, Stuttgart, Deutschland) verwendet. Als Fließmittel wurde ein Puffer aus 0.25 Natriumsulfat mit einem pH von ca. 6 verwendet (Windisch et al. 1997). Alternativ läßt sich ein Puffer aus 0.1 M di-Natriumhydrogenphosphat-Dihydrat, 0.1 M Natriumsulfat entwässert und wurde mit ortho-Phosphorsäure 85% auf pH 6.8 eingestellt, verwenden. Die aufgegebene Probenmenge betrug 20*µ*l bei einer Proteinkonzentration von 0,5-2 mg/ml. Der Nachweis des Proteins erfolgte mit Hilfe eines UV-Detektors der Firma Agilent bei 210nm. Für die Auswertung der Chromatogramme wurde die Software HP-Chemstation der Firma Agilent verwendet.

### c) Lysozym-Restmonomergehalt

Um den Lysozym-Restmonomergehalt in den rekonstituierten Lsozym-Formulierungen zu quantifizieren wurde eine modifizeirte SEC-HPLC (van de Weert, 2000) durchgeführt. Die SEC-HPLC wurde mit einer HP1100 der Firma Agilent durchgeführt. Zur Trennung wurde eine TSK2000SWXL Säule (300 x 7.8mm) der Fa. Tosoh Biosep (Tosoh Bioscience, Stuttgart, Deutschland) verwendet. Als Fließmittel wurde ein Puffer aus 0,05 M di-Natriumhydrogenphosphat-Dihydrat und 0,2 M Natriumchlorid mit ortho-Phosphorsäure 85% auf pH 7.0 eingestellt. Die aufgegebene Probenmenge betrug 25*µ*l bei einer Proteinkonzentration von 2-10 mg/ml. Der Nachweis des Proteins erfolgte mit Hilfe eines UV-Detektors der Firma Agilent bei 280nm. Für die Auswertung der Chromatogramme wurde die Software Agilent Chemstation der Firma Agilent verwendet.
Zur Beurteilung der Formuierungen wird das verbliebene lösliche Monomer nach folgender Methode qauntifiziert. Zunächst wurde eine Eichgerade mit Lysozymstandardlösungen mit Konzentrationen von 2.5 mg/ml, 5.0 mg/ml und 10 mg/ml erstellt. Dabei wurde die AUC der Monomerpeaks in Relation zur den entsprechenden Lysozymkonzentrationen in den untersuchten Standardlösung betrachtet.Der Restmonomergehalt der untersuchten verschiedenen Lysozymformulierungen wurde anhand der Eichgeraden errechnet. Je höher der Restmonomergehalt einer Formulierung ist, desto besser ist die Proteinstabilität.

### Partikelgrößenbestimmung (MMD)

Der Mass Median Diameter oder die mittlere Teilchengrößen der Partikel wurde mit Hilfe des Sympatech Helos der Fa Sympatech GmbH (Clausthal-Zellerfeld, DE) bestimmt. Das Messprinzip beruht auf Laserbeugung, verwendet wird ein Helium-Neon-Laser. 1-3 mg Pulver werden mit einem Luftdruck von 2bar dispergiert, und vor der Fourierlinse (50mm) durch einen parallelen Laserstrahl geführt. Die Partikelgrößenverteilung wird mit einem Fraunhofer-Modell ausgewertet. Pro Pulver wurden zwei Messungen durchgeführt..

### Mass Median Aerodynamic Diameter (MMAD) und Feinpartikelfraktion (FPF)

Für die Messungen wurden jeweils 12-18mg Pulver in Hartgelatine Kapseln (Größe 3) abgefüllt und in den HandiHaler (Pulver-Inhalationsgerät der Firma Boehringer Ingelheim) eingebracht. Mit einem Adapter wurde der HandiHaler an den USP EP/Throat des Impaktorinlets des Messgerätes angekoppelt und das Pulver mit 39,01/min bei einer Sogzeit von 6,15sec ausgebracht. Die Steuerung des Luftdurchsatzes erfolgte über eine externe Steuerwand. Für jedes Pulver wurden zumindest drei Kapseln vermessen.
Mit dem APS 3321 der Firma TSI Inc., MN, USA wird in Kombination mit dem Impaktorinlet 3306 simultan die aerodynamische Teilchengröße (MMAD) über eine Flugzeitbestimmung und die Feinpartikelfraktion (FPF) über einen Einstufenimpaktor (effektiver Cut off Diameter bei 39L/min: 5,0*µ*m) bestimmt. Das Pulver gelangt nach Ausbringung über den EP/USP Throat oder Sample Induction Port zu einer dünnen Kapillare, wo 0,2% der Pulvermenge für die Flugzeitmessung (Time of Flight) unter isokinetischen Bedingungen entnommen wird. Die Flugzeitmessung erfolgt nach dem Passieren der Kapillare über 2 Laserstrahlen, die analog einer Lichtschranke die Flugzeiten für eine definierte Strecke erfassen. Als Ergebnis erhält man eine Anzahlverteilung, die anschließend auf eine Massenverteilung und damit auf den Mass Median Aerodynamic Diameter (MMAD) umgerechnet wird.
Die restlichen 99,8% der Pulverpopulation, die an der Kapillare vorbeigeführt wurden, werden über den Einstufenimpaktor aufgetrennt. Die Fraktion größer 5,0µm scheidet sich im Impaktor aufgrund der Massenträgheit auf einer Prallplatte ab. Die Feinpartikelfraktion (FPF) folgt dem Luftstrom und wird schließlich auf einem Tiefenfilter abgeschieden. Die Bestimmung der Feimpartikelfraktion erfolgte gravimetrisch. Die Feinpartikelfraktion berechnet sich aus dem Anteil des auf dem Filter abgeschiedenen Pulvers in Bezug auf die Gesamtmenge an eingesetztem Pulver, d.h. eingewogenes Pulver pro Kapsel.

### Restwassergehalt:

Der Restwassergehalt in den getrockneten Produkten wurde mittels coulometrischer Titration (Metrohm 737 KF Coulometer mit 703 Titrationsstand, Deutschland) bestimmt. Zur Bestimmung wurde Pulver in Methanol (Hydranal - Methanol dry, VWR /Merck Eurolab) aufgelöst bzw. dispergiert. Die Messlösung (Hydranal -Coulomat- Lösung, VWR / Merck Eurolab) des Metrohm Coulometers wurde zu Beginn der Messungen konditioniert, d.h. die Messlösung wurde auf einen Nullgehalt an Wasser austariert. Die Probe wurde in die Titrationszelle injiziert und vermessen.

### Stabilitätsbestimmung:

Die Pulver bzw. im Pulver enthaltene Proteine wurden nach der Sprühtrocknung auf verschiedene Stabilitäten untersucht. Bei IgG1 und Calcitonin wurde als Maß für die Stabilität der Formulierungen der prozentuale Anteil der Proteinaggregate gewertet. Bei Lysozym wurde als Maß für die Stabilität der Formulierungen der prozentuale Anteil des Restmonomergehaltes gewertet. Den in der Erfindung beschriebenen innovativen Hilfsstoffen wurden partiell als Referenz reine Proteinformulierungen, analoge Trehalose-Formulierungen, analoge Raffinose-Formulierungen, analoge Saccharose-Formulierungen, analoge Saccharose-Lactose-Formulierungen oder analoge Hydroxyethylstärke-Formulierungen gegenüber gestellt. Die Analytik zur Untersuchung auf Aggregate wurde mit einer validierten Größenausschlusschromatographie (SEC-HPLC) mit UV-Detektion (DAD) durchgeführt. Dazu wurden die vorbehandelten Pulver zunächst in Reinstwasser (pH von 6 bis 8) rekonstituiert.
- Forcierte Lagerstabilität: Ausgewählte Formulierungen wurden nach einwöchiger offener Lagerung bei ca. 40°C und ca. 75% relativer Luftfeuchtigkeit (40°C, 75% rF) in offenen Glasvials auf ihre Stabilität untersucht.
- Equilibrierte Lagerstabilität: Ausgewählte Formulierungen wurden nach Sprühtrocknung bei einen Tag bei ca. 22°C und 50 bis 55% relativer Luftfeuchtigkeit in offenen Glasvials gelagert (Equilibrierung). Die Glasvials wurden im Anschluss bei den oben genannten Bedingungen verschlossen und verbördelt und nach vierwöchiger trockener Lagerung bei ca. 40°C auf ihre Stabilität untersucht.
- Vakuumgetrocknete Lagerstabilität: Ausgewählte Formulierungen wurden nach Sprühtrocknung bei einen Tag in einem Vakuumtrockenschrank der Firma Memmert (Deutschland) bei ca. 30°C und ca. 0,15 Millibar in offenen Glasvials gelagert (Vakuumtrocknung). Die Glasvials wurden im Anschluss aus dem Vakuumtrockenschrank entnommen, verschlossen und verbördelt und nach vierwöchiger Lagerung bei ca. 40°C auf ihre Stabilität untersucht.
- 3 Monatsstabilität: Ausgewählte Formulierungen wurden nach Sprühtrocknung in offenen Glasvials vakuumgetrocknet (s. o.). Die Glasvials wurden unter Stickstoff verschlossen und verbördelt und bei drei verschiedenen Temperaturen gelagert. Die Temperaturen waren 2-8°C, 25°C sowie 40°C. Die Pulver wurden nach einem Monat auf Ihre Stabilität untersucht.
- Offene 3 Monatsstabilität: Ausgewählte Formulierungen wurden nach Sprühtrocknung in offenen Glasvials bei ca. 29% und/oder 43% relativer Luftfeuchte und jeweils 25°C gelagert. Die Pulver wurden nach einem und drei Monaten auf Ihre Stabilität untersucht. Bei ausgewählten Formulierungen wurde auch die aerodynamische Performance des Pulvers mittels Time-Of-Flight Messungen (siehe oben) bestimmt.

### Beispiel 1

### Sprühtrocknung einer IgG1 10% (w/v) Formulierung

Pures IgG1 mit einer Konzentration von ca. 109 mg/ml, formuliert in einem Glycin Histidin Puffer pH 6 (siehe Materialien), wurde mit demineralisiertem Wasser (pH ca. 7,5) auf einen Gehalt von 100mg/ml verdünnt und in Abwesenheit von weiteren Hilfsstoffen wie oben beschrieben unter Verwendung des Zyklon I mit einer Zerstäubungsflussrate von ca. 0,67 m³/h sprühgetrocknet. Das Volumen der Lösung umfasste 50 ml. Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 18,9% Aggregate bzw. 18,2% Aggregate.
- Nach einem Tag Equilibrierung und vier Wochen trockener Lagerung bei 40°C hatte die Lösung aus dem rekonstituierten Pulver ca. 11,8% Aggregate.
- Nach einem Tag Vakuumtrocknung und vier Wochen trockener Lagerung bei 40°C hatte die Lösung aus dem rekonstituierten Pulver ca. 13,2% Aggregate.

### Sprühtrocknung einer IgG1 3,33% (w/v) Formulierung

Pures IgG1 mit einer Konzentration von ca. 102.8 mg/ml, formuliert in einem Glycin Histidin Puffer pH 6 (siehe Materialien), wurde mit demineralisiertem Wasser (pH ca. 7,5) auf einen Gehalt von 33mg/ml verdünnt und in Abwesenheit von weiteren Hilfsstoffen wie oben beschrieben unter Verwendung des Zyklon I mit einer Zerstäubungsflussrate von ca. 0,67 m³/h sprühgetrocknet. Das Volumen der Lösung umfasste 150 ml. Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 16,3% Aggregate.
- Nach 1 und 3 Monaten Lagerung bei 2-8°C (3 Monatsstabilität) hatte die Lösung aus dem rekonstituierten Pulver jeweils ca. 4,5% und 4,4% Aggregate.
- Nach 1 und 3 Monaten Lagerung bei 25°C (3 Monatsstabilität) hatte die Lösung aus dem rekonstituierten Pulver jeweils ca. 7,4% und 7,1 % Aggregate.
- Nach 1 und 3 Monaten Lagerung bei 40°C (3 Monatsstabilität) hatte die Lösung aus dem rekonstituierten Pulver jeweils ca. 13,3% und 18,1% Aggregate.
- Nach 1 und 3 Monaten offener Lagerung bei ca. 29% relativer Luftfeuchte und 25°C hatte die Lösung aus dem rekonstituierten Pulver jeweils ca. 5,5% und 6,6% Aggregate.
- Nach 1 und 3 Monaten offener Lagerung bei ca. 43% relativer Luftfeuchte und 25°C hatte die Lösung aus dem rekonstituierten Pulver Pulver jeweils ca. 5,6% und 7,0% Aggregate. Sprühtrocknung einer Trehalose 9% (w/v) lgG1 1 % (w/v) Formulierung

4,5 g Trehalose wurde in ca. 40 ml demineralisiertem Wasser (pH ca. 7,5) gelöst. Als nächstes wurden ca. 4,6 ml pures lgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 12,6% Aggregate.
   Sprühtrocknung einer LS90P 3,00% (w/v) IgG1 0,33% (w/v) Formulierung

4,5 g LS90P wurde in ca. 140 ml demineralisiertem Wasser (pH ca. 7,5) gelöst. Als nächstes wurden ca. 4,864 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3,00% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 1,0% Aggregate.
- Nach 1 und 3 Monaten Lagerung bei 2-8°C (3 Monatsstabilität) hatte die Lösung aus dem rekonstituierten Pulver jeweils ca. 0,6% und 0,9% Aggregate.
- Nach 1 und 3 Monaten Lagerung bei 25°C (3 Monatsstabilität) hatte die Lösung aus dem rekonstituierten Pulver jeweils ca. 0,8% und 1,3% Aggregate.
- Nach 1 und 3 Monaten Lagerung bei 40°C (3 Monatsstabilität) hatte die Lösung aus dem rekonstituierten Pulver ca. 1,1 % und 2,2% Aggregate.
   Der MMD des Pulvers wurde wie oben beschrieben bestimmt.
- Der MMD des Pulvers lag nach Sprühtrocknung bei 2,8 µm.
   Der MMAD und FPF des Pulvers wurde wie oben beschrieben bestimmt.
- Der MMAD des Pulvers lag nach Sprühtrocknung bei 3,8 µm und die Feinpartikelfraktion lag bei 23,6% bezogen auf die Pulvereinwaage in der Kapsel.
   Sprühtrocknung einer LS55P 9,9% (w/v) IgG1 0,1% (w/v) Formulierung

4,950 g LS55P wurde in ca. 40 ml demineralisiertem Wasser (pH ca. 7,5) gelöst. Als nächstes wurden ca. 0,518 ml pures IgG1 mit einer Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9,9% (w/v) Hilfsstoff oder Matrix sowie 0,1% (w/v) Protein und wurde wie oben beschrieben unter Verwendung des Zyklon 1 mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 5,7% Aggregate.
- Nach einem Tag Vakuumtrocknung und vier Wochen trockener Lagerung bei 40°C hatte die Lösung aus dem rekonstituierten Pulver ca. 4,7% Aggregate.
   Sprühtrocknung einer LS55P 9% (w/v) IgG1 1% (w/v) Formulierung

4,5 g LS55P wurde in ca. 40 ml demineralisiertem Wasser (pH ca. 7,5) gelöst. Als nächstes wurden ca. 4,6 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 2,3% Aggregate.
- Nach einem Tag Equilibrierung und vier Wochen trockener Lagerung bei 40°C hatte die Lösung aus dem rekonstituierten Pulver ca. 1,8% Aggregate.
- Nach einem Tag Vakuumtrocknung und vier Wochen trockener Lagerung bei 40°C hatte die Lösung aus dem rekonstituierten Pulver ca. 1,4% Aggregate.
   Sprühtrocknung einer LS55P 6% (w/v) IgG1 4% (w/v) Formulierung

3,0 g LS55P wurde in ca. 15 ml demineralisiertem Wasser (pH ca. 7,5) gelöst. Als nächstes wurden ca. 19,45 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 6% (w/v) Hilfsstoff oder Matrix sowie 4% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 4,0% Aggregate.
   Sprühtrocknung einer LS55P 4% (w/v) IgG1 6% (w/v) Formulierung

2,0 g LS55P wurde in ca. 15 ml demineralisiertem Wasser (pH ca. 7,5) gelöst. Als nächstes wurden ca. 29,18 ml pures lgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 4% (w/v) Hilfsstoff oder Matrix sowie 6% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 6,9% Aggregate.
   Sprühtrocknung einer LS55P 2,5% (w/v) IgG1 7,5% (w/v) Formulierung

1,25 g LS55P wurde in ca. 10 ml demineralisiertem Wasser (pH ca. 7,5) gelöst. Als nächstes wurden ca. 38,84 ml pures IgG1 mit einer Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 2,5% (w/v) Hilfsstoff oder Matrix sowie 7,5% (w/v) Protein und wurde wie oben beschrieben unter Verwendung des Zyklon 1 mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 5,9% Aggregate.
- Nach einem Tag Vakuumtrocknung und vier Wochen trockener Lagerung bei 40°C hatte die Lösung aus dem rekonstituierten Pulver ca. 6,1% Aggregate.
   Sprühtrocknung einer LS55P 1,0% (w/v) IgG1 9,0% (w/v) Formulierung

0,50 g LS55P wurde in ca. 5 ml demineralisiertem Wasser (pH ca. 7,5) gelöst. Als nächstes wurden ca. 41,43 ml pures IgG1 mit, einer Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 1,0% (w/v) Hilfsstoff oder Matrix sowie 9,0% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 10,8% Aggregate.
- Nach einem Tag Vakuumtrocknung und vier Wochen trockener Lagerung bei 40°C hatte die Lösung aus dem rekonstituierten Pulver ca. 8,0% Aggregate.
Sprühtrocknung einer LS55P 0,5% (w/v) IgG1 9,5% (w/v) Formulierung

0,25 g LS55P wurde in ca. 2,5 ml demineralisiertem Wasser (pH ca. 7,5) gelöst. Als nächstes wurden ca. 46,21 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 0,5% (w/v) Hilfsstoff oder Matrix sowie 9,5% (w/v) Protein und wurde wie oben beschrieben unter Verwendung des Zyklon 1 mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 13,7% Aggregate.
   Sprühtrocknung einer LS55P 3,00% (w/v) IgG1 0,33% (w/v) Formulierung

9,0 g LS55P wurde in ca. 280 ml demineralisiertem Wasser (pH ca. 7,5) gelöst. Als nächstes wurden ca. 9,73 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3,0% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 5,0% Aggregate.
Der MMD des Pulvers wurde wie oben beschrieben bestimmt.
- Der MMD des Pulvers lag bei 2,9 µm.
Der MMAD und FPF des Pulvers wurde wie oben beschrieben bestimmt.
- Der MMAD lag bei 4,3 µm und die Feinpartikelfraktion lag bei 15,9% bezogen auf die Pulvereinwaage in der Kapsel.
Sprühtrocknung einer Coupling Sugar 9,9% (w/v) lgG1 0,1% (w/v) Formulierung

6,290 g Coupling Sugar haltiger Sirup (entspricht 4,950 g Coupling Sugar) wurde in ca. 40 ml demineralisiertem Wasser (pH ca. 7,5) gelöst. Als nächstes wurden ca. 0,518 ml pures IgG1 mit einer Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9,9% (w/v) Hilfsstoff oder Matrix sowie 0,1% (w/v) Protein und wurde wie oben beschrieben unter Verwendung des Zyklon 1 mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 14,9% Aggregate.
Sprühtrocknung einer Coupling Sugar 9% (w/v) IgG1 1% (w/v) Formulierung

5,71 g Coupling Sugar haltiger Sirup (entspricht 4,5 g Coupling Sugar) wurde in ca. 40 ml demineralisiertem Wasser (pH ca. 7,5) gelöst. Als nächstes wurden ca. 4,6 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 4,9% Aggregate.
- Nach einem Tag Equlibrierung und vier Wochen trockener Lagerung bei 40°C hatte die Lösung aus dem rekonstituierten Pulver ca. 3,2% Aggregate.
- Nach einem Tag Vakuumtrocknung und vier Wochen trockener Lagerung bei 40°C hatte die Lösung aus dem rekonstituierten Pulver ca. 2,1% Aggregate.
   Sprühtrocknung einer Coupling Sugar 6% (w/v) IgG1 4% (w/v) Formulierung

3,81g Coupling Sugar haltiger Sirup (entspricht 3,0 g Coupling Sugar) wurde in ca. 25 ml demineralisiertem Wasser (pH ca. 7,5) gelöst. Als nächstes wurden ca. 19,45 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 6% (w/v) Hilfsstoff oder Matrix sowie 4% (w/v) Protein und wurde wie oben beschrieben unter Verwendung des Zyklon 1 mit einer Zerstäubungsflussrate von ca. 0,67 m³/h sprühgetrocknet. Der Gehalt an Aggregaten wurde wie oben beschrieben untersucht. Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 5,0% Aggregate.
   Sprühtrocknung einer Coupling Sugar 4% (w/v) IgG1 6% (w/v) Formulierung

2,54g Coupling Sugar haltiger Sirup (entspricht 2,0 g Coupling Sugar) wurde in ca. 15 ml demineralisiertem Wasser (pH ca. 7,5) gelöst. Als nächstes wurden ca. 29,18 ml pures IgG1 mit einer Konzentration von ca. x mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 4% (w/v) Hilfsstoff oder Matrix sowie 6% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 9,9% Aggregate.
   Sprühtrocknung einer Coupling Sugar 2,5% (w/v) IgG1 7,5% (w/v) Formulierung

1,59 g Coupling Sugar haltiger Sirup (entspricht 1,250 g Coupling Sugar) wurde in ca. 8 ml demineralisiertem Wasser (pH ca. 7,5) gelöst. Als nächstes wurden ca. 38,84 ml pures IgG1 mit einer Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 2,5% (w/v) Hilfsstoff oder Matrix sowie 7,5% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 10,8% Aggregate.
   Sprühtrocknung einer Coupling Sugar 1,0% (w/v) IgG1 9,0% (w/v) Formulierung

0,653 g Coupling Sugar haltiger Sirup (entspricht 0,50 g Coupling Sugar) wurde in ca. 5 ml demineralisiertem Wasser (pH ca. 7,5) gelöst. Als nächstes wurden ca. 41,43 ml pures IgG1 mit einer Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 1,0% (w/v) Hilfsstoff oder Matrix sowie 9,0% (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 13,1 % Aggregate.
   Sprühtrocknung einer Coupling Sugar S 9% (w/v) IgG1 1% (w/v) Formulierung

5,86g Coupling Sugar S haltiger Sirup (entspricht 4,5 g Coupling Sugar S) wurde in ca. 40 ml demineralisiertem Wasser (pH ca. 7,5) gelöst. Als nächstes wurden ca. 4,6 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1 % (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 5,4% Aggregate.

### Beispiel 2

### Sprühtrocknung einer Trehalose 8% (w/v) L-Isoleucin 1% (w/v) IgG1 1% (w/v) Formulierung

4,0 g Trehalose und 0,5 g L-Isoleucin wurden im Ultraschallbad in ca. 40 ml demineralisiertem Wasser (pH ca. 7,5) gelöst. Als nächstes wurden ca. 4,6 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1 % (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 22,2% Aggregate.
   Sprühtrocknung einer LS90P 2,66% (w/v) L-Isoleucin 0,33% (w/v) IgG1 0,33% (w/v) Formulierung

4,0 g LS90P und 0,50 g L-Isoleucin wurden in ca. 140 ml demineralisiertem Wasser (pH ca. 7,5) gelöst. Als nächstes wurden ca. 4,864 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3,00% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 0,7% Aggregate.
- Nach 1 und 3 Monaten Lagerung bei 2-8°C (3 Monatsstabilität) hatte die Lösung aus dem rekonstituierten Pulver ca. 0,7% und 1,0% Aggregate.
- Nach 1 und 3 Monaten Lagerung bei 25°C (3 Monatsstabilität) hatte die Lösung aus dem rekonstituierten Pulver ca. 0,8% und 1,1% Aggregate.
- Nach 1 und 3 Monaten Lagerung bei 40°C (3 Monatsstabilität) hatte die Lösung aus dem rekonstituierten Pulver ca. 0,6% und 1,1% Aggregate.

Der MMAD und FPF des Pulvers wurde wie oben beschrieben bestimmt.
- Der MMAD des Pulvers lag nach Sprühtrocknung bei 7,3 µm und die Feinpartikelfraktion lag bei 28,1% bezogen auf die Pulvereinwaage in der Kapsel.
   Sprühtrocknung einer LS55P 8% (w/v) L-Isoleucin 1 % (w/v) IgG1 1% (w/v) Formulierung

4,00 g LS55P und 0,50 g L-Isoleucin wurden im Ultraschallbad in ca. 40 ml demineralisiertem Wasser (pH ca. 7,5) gelöst. Als nächstes wurden ca. 4,60 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1 % (w/v) Protein und wurde wie oben beschrieben unter Verwendung des Zyklon I mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 5,5% Aggregate.
- Nach einem Tag Equilibrierung und vier Wochen trockener Lagerung bei 40°C hatte die Lösung aus dem rekonstituierten Pulver ca. 1,8% Aggregate.
- Nach einem Tag Vakuumtrocknung und vier Wochen trockener Lagerung bei 40°C hatte die Lösung aus dem rekonstituierten Pulver ca. 1,8% Aggregate.
   Sprühtrocknung einer LS55P 2,66% (w/v) L-Isoleucin 0,33% (w/v) IgG1 0,33% (w/v) Formulierung

8,0 g LS55P und 1 g L-Isoleucin wurden im Ultraschallbad in ca. 280 ml demineralisiertem Wasser (pH ca. 7,5) gelöst. Als nächstes wurden ca. 9,7 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon 11 mit einer Zerstäubungsflussrate von ca. 0;67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver lediglich ca. 5,9% Aggregate.
- Nach 1 und 3 Monaten Lagerung bei 2-8°C (3 Monatsstabilität) hatte die Lösung aus dem rekonstituierten Pulver jeweils ca. 1,6% Aggregate.
- Nach 1 und 3 Monaten Lagerung bei 25°C (3 Monatsstabilität) hatte die Lösung aus dem rekonstituierten Pulver jeweils ca. 1,6% und 1,8% Aggregate.
- Nach 1 und 3 Monaten Lagerung bei 40°C (3 Monatsstabilität) hatte die Lösung aus dem rekonstituierten Pulver jeweils ca. 1,6% und 1,8% Aggregate.

Der MMAD und FPF des Pulvers wurde wie oben beschrieben bestimmt.
- Der MMAD lag bei 4,9 *µ*m und die Feinpartikelfraktion lag bei 34,7% bezogen auf die Pulvereinwaage in der Kapsel.
   Sprühtrocknung einer Coupling Sugar 8% (w/v) L-Isoleucin 1% (w/v) IgG1 1% (w/v) Formulierung

5,08 g Coupling Sugar haltiger Sirup (entspricht 4,0 g Coupling Sugar) und 0,50 g L-Isoleucin wurden im Ultraschallbad in ca. 40 ml demineralisiertem Wasser (pH ca. 7,5) gelöst. Als nächstes wurden ca. 4,60 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1 % (w/v) Protein und wurde wie oben beschrieben unter Verwendung des Zyklon I mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver lediglich ca. 7,1 % Aggregate.
- Nach einem Tag Equilibrierung und vier Wochen trockener Lagerung bei 40°C hatte die Lösung aus dem rekonstituierten Pulver ca. 2,5% Aggregate.
- Nach einem Tag Vakuumtrocknung und vier Wochen trockener Lagerung bei 40°C hatte die Lösung aus dem rekonstituierten Pulver ca. 1,1% Aggregate.
   Sprühtrocknung einer Coupling Sugar S 8% (w/v) L-Isoleucin 1% (w/v) IgG1 1% (w/v) Formulierung

5,21 g Coupling Sugar S haltiger Sirup (entspricht 4,0 g Coupling Sugar S) und 0,50 g L-Isoleucin wurden im Ultraschallbad in ca. 40 ml demineralisiertem Wasser (pH ca. 7,5) gelöst. Als nächstes wurden ca. 4,60 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1 % (w/v) Protein und wurde wie oben beschrieben unter Verwendung des Zyklon I mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver lediglich ca. 6,8% Aggregate.

### Beispiel 3

### Sprühtrocknung einer Trehalose 3% (w/v) L-Citrullin 6% (w/v) IgG1 1% (w/v) Formulierung

1,50 g Trehalose und 3,00 g L-Citrullin wurden im Ultraschallbad in ca. 40 ml demineralisiertem Wasser (pH ca. 7,5) gelöst. Als nächstes wurden ca. 4,6 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1 % (w/v) Protein und wurde wie oben beschrieben unter Verwendung des Zyklon I mit einer Zerstäubungsflussrate von ca. 0,67 m³/h sprühgetrocknet. Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 5,9% Aggregate.
   Sprühtrocknung einer LS55P 3% (w/v) L-Citrullin 6% (w/v) IgG1 1% (w/v) Formulierung

1,50 g LS55P und 3,00 g L-Citrullin wurden im Ultraschallbad in ca. 40 ml demineralisiertem Wasser (pH ca. 7,5) gelöst. Als nächstes wurden ca. 4,60 ml pures lgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 2,9% Aggregate.
- Nach einem Tag Equilibrierung und vier Wochen trockener Lagerung bei 40°C hatte die Lösung aus dem rekonstituierten Pulver ca. 1,8% Aggregate.
- Nach einem Tag Vakuumtrocknung und vier Wochen trockener Lagerung bei 40°C hatte die Lösung aus dem rekonstituierten Pulver ca. 1,3% Aggregate.
Sprühtrocknung einer Coupling Sugar 3% (w/v) L-Citrullin 6% (w/v) IgG1 1% (w/v) Formulierung

1,91 g Coupling Sugar haltiger Sirup (entspricht 1,5 g Coupling Sugar) und 3,00 g L-Citrullin wurden im Ultraschallbad in ca. 40 ml demineralisiertem Wasser (pH ca. 7,5) gelöst. Als nächstes wurden ca. 4,60 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1 % (w/v) Protein und wurde wie oben beschrieben unter Verwendung des Zyklon I mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 3,9% Aggregate.
- Nach einem Tag Equilibrierung und vier Wochen trockener Lagerung bei 40°C hatte die Lösung aus dem rekonstituierten Pulver ca. 1,4% Aggregate.

Nach einem Tag Vakuumtrocknung und vier Wochen trockener Lagerung bei 40°C hatte die Lösung aus dem rekonstituierten Pulver ca. 1,3% Aggregate.
Sprühtrocknung einer Coupling Sugar S 3% (w/v) L-Citrullin 6% (w/v) IgG1 1% (w/v) Formulierung

1,95 g Coupling Sugar S haltiger Sirup (entspricht 1,5 g Coupling Sugar S) und 3,00 g L-Citrullin wurden im Ultraschallbad in ca. 40 ml demineralisiertem Wasser (pH ca. 7,5) gelöst. Als nächstes wurden ca. 4,60 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver lediglich ca. 3,1% Aggregate.

### Beispiel 4

### Sprühtrocknung einer Trehalose 2,66% (w/v) Tri-Isoleucin 0,33% (w/v) IgG1 0,33% (w/v) Formulierung

8,0 g Trehalose und 1 g Tri-Isoleucin wurden im Ultraschallbad in ca. 280 ml demineralisiertem Wasser (pH ca. 7,5) gelöst. Als nächstes wurden ca. 12,30 ml pures IgG1 mit einer Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 26,7% Aggregate.
   Sprühtrocknung einer Raffinose 2,66% (w/v) Tri-Isoleucin 0,33% (w/v) IgG1 0,33% (w/v) Formulierung

8,0 g Raffinose und 1 g Tri-Isoleucin wurden im Ultraschallbad in ca. 140 ml demineralisiertem Wasser (pH ca. 7,5) gelöst. Als nächstes wurden ca. 4,87 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon 11 mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 12,6% Aggregate.
   Sprühtrocknung einer Hydroxyethylstärke (HES) 2,66% (w/v) Tri-Isoleucin 0,33% (w/v) IgG1 0,33% (w/v) Formulierung

8,0 g HES und 1 g Tri-Isoleucin wurden im Ultraschallbad in ca. 140 ml demineralisiertem Wasser (pH ca. 7,5) bei ca. 80°C unter Rühren gelöst. Als nächstes wurden der zuvor im Kühlschrank abgekühlten trüben Lösung ca. 4,87 ml pures lgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3% (w/v) Hilfsstoff oder Matrix sowie 0,33% (w/v) Protein und wurde wie oben unter Verwendung des Zyklon II mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 18,6% Aggregate.
- Nach 1 und 3 Monaten offener Lagerung bei 43% relativer Luftfeuchte und 25°C (offene 3 Monatsstabilität) hatte die Lösung aus dem rekonstituierten Pulver jeweils ca. 11,9% und 15,4% Aggregate.
   Sprühtrocknung einer Saccharose 2,00% (w/v) Lactose 0,66% (wv) Tri-Isoleucin 0,33% (w/v)IgG1 0,33% (w/v) Formulierung

6,0 g Saccharose, 2,0 g Lactose und 1 g Tri-Isoleucin wurden im Ultraschallbad in ca. 280 ml demineralisiertem Wasser (pH ca. 7,5) gelöst. Als nächstes wurden ca. 9,73 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 8,8% Aggregate.
   Sprühtrocknung einer Saccharose 2,66% (w/v) Tri-Isoleucin 0,33% (w/v) IgG1 0,33% (w/v) Formulierung

8,0 g Saccharose und 1 g Tri-Isoleucin wurden im Ultraschallbad in ca. 280 ml demineralisiertem Wasser (pH ca. 7,5) gelöst. Als nächstes wurden ca. 9,73 ml pures lgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 5,6% Aggregate.
   Sprühtrocknung einer LS90P 2,66% (w/v) Tri-Isoleucin 0,33% (w/v) IgG1 0,33% (w/v) Formulierung

4,0 g LS90P und 0,50 g Tri-Isoleucin wurden in ca. 140 ml demineralisiertem Wasser (pH ca. 7,5) gelöst. Als nächstes wurden ca. 4,864 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3,00% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 2,3% Aggregate.
- Nach 1 und 3 Monaten Lagerung bei 2-8°C (3 Monatsstabilität) hatte die Lösung aus dem rekonstituierten Pulver ca. 0,7% und 1,0% Aggregate.
- Nach 1 und 3 Monaten Lagerung bei 25°C (3 Monatsstabilität) hatte die Lösung aus dem rekonstituierten Pulver ca. 0,8% und 1,4% Aggregate.
- Nach 1 und 3 Monaten Lagerung bei 40°C (3 Monatsstabilität) hatte die Lösung aus dem rekonstituierten Pulver ca. 0,9% und 2,2% Aggregate.
- Nach 1 und 3 Monaten offener Lagerung bei ca. 29% relativer Luftfeuchte und 25°C hatte die Lösung aus dem rekonstituierten Pulver jeweils ca. 0,4% und 0,7% Aggregate.
- Nach 1 und 3 Monaten offener Lagerung bei ca. 43% relativer Luftfeuchte und 25°C hatte die Lösung aus dem rekonstituierten Pulver Pulver jeweils ca. 0,5% und 0,6% Aggregate.
Der MMD des Pulvers wurde wie oben beschrieben bestimmt.
- Der MMD des Pulvers lag nach Sprühtrocknung bei 4,7 µm.
   Der MMAD und FPF des Pulvers wurde wie oben beschrieben bestimmt.
- Der MMAD des Pulvers lag nach Sprühtrocknung bei 4,8 µm und die Feinpartikelfraktion lag bei 53,2% bezogen auf die Pulvereinwaage in der Kapsel.
   Sprühtrocknung einer LS90P 2,66% (w/v) Tri-Isoleucin 0,33% (w/v) IgG1 0,33% (w/v) Formulierung

4,0 g LS90P und 0,50 g Tri-Isoleucin wurden in ca. 140 ml demineralisiertem Wasser (pH ca. 7,5) gelöst. Als nächstes wurden ca. 4,864 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3,00% (w/v) Hilfsstoff oder Matrix sowie 0,33% (w/v) Protein und wurde wie oben beschrieben unter Verwendung des Zyklon II mit einer Zerstäubungsflussrate von ca. 1,05 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 2,2% Aggregate.
- Nach 1 und 3 Monaten offener Lagerung bei 29% relativer Luftfeuchte und 25°C (offene 3 Monatsstabilität) hatte die Lösung aus dem rekonstituierten Pulver jeweils ca. 0,5% und 0,6% Aggregate.
- Nach 1 und 3 Monaten offener Lagerung bei 43% relativer Luftfeuchte und 25°C (offene 3 Monatsstabilität) hatte die Lösung aus dem rekonstituierten Pulver jeweils ca. 0,5% und 0,7% Aggregate.

Der MMD des Pulvers wurde wie oben beschrieben bestimmt.
- Der MMD des Pulvers lag nach Sprühtrocknung bei 2,7 *µ*m.

Der MMAD und FPF des Pulvers wurde wie oben beschrieben bestimmt.
- Der MMAD des Pulvers lag nach Sprühtrocknung bei 3,6 µm und die Feinpartikelfraktion lag bei 58,0% bezogen auf die Pulvereinwaage in der Kapsel.
   Sprühtrocknung einer LS90P 2,66% (w/v) Tri-Isoleucin 0,33% (w/v) IgG1 0,33% (w/v) Formulierung

4,0 g LS90P und 0,50 g Tri-Isoleucin wurden in ca. 140 ml demineralisiertem Wasser (pH ca. 7,5) gelöst. Als nächstes wurden ca. 4,864 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3,00% (w/v) Hilfsstoff oder Matrix sowie 0,33% (w/v) Protein und wurde wie oben beschrieben unter Verwendung des Zyklon II mit einer Zerstäubungsflussrate von ca. 1,74 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. % Aggregate.
- Nach 1 und 3 Monaten offener Lagerung bei 29% relativer Luftfeuchte und 25°C (offene 3 Monatsstabilität) hatte die Lösung aus dem rekonstituierten Pulver jeweils ca. 0,5% und 0,6% Aggregate.
- Nach 1 Monat offener Lagerung bei 43% relativer Luftfeuchte und 25°C (offene 3 Monatsstabilität) hatte die Lösung aus dem rekonstituierten Pulver jeweils ca. 0,5% und 0,8% Aggregate.

Der MMD des Pulvers wurde wie oben beschrieben bestimmt.
- Der MMD des Pulvers lag nach Sprühtrocknung bei 2,6 µm.

Der MMAD und FPF des Pulvers wurde wie oben beschrieben bestimmt.
- Der MMAD des Pulvers lag nach Sprühtrocknung bei 3,3 *µ*m und die Feinpartikelfraktion lag bei 58,9% bezogen auf die Pulvereinwaage in der Kapsel.
   Sprühtrocknung einer LS90P 1,60% (w/v) Tri-Isoleucin 0,20% (w/v) IgG1 0,20% (w/v) Formulierung

4,0 g LS90P und 0,50 g Tri-Isoleucin wurden in ca. 220 ml demineralisiertem Wasser (pH ca. 7,5) gelöst. Als nächstes wurden ca. 4,864 ml pures lgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 250 ml verdünnt. Die so erhaltene Lösung enthält ca. 1,80% (w/v) Hilfsstoff oder Matrix sowie 0,20% (w/v) Protein und wurde wie oben beschrieben unter Verwendung des Zyklon II mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 2,2% Aggregate.
- Nach 1 und 3 Monaten offener Lagerung bei 29% relativer Luftfeuchte und 25°C (offene 3 Monatsstabilität) hatte die Lösung aus dem rekonstituierten Pulver jeweils ca. 0,5% und 0,5% Aggregate.
- Nach 1 und 3 Monaten offener Lagerung bei 43% relativer Luftfeuchte und 25°C (offene 3 Monatsstabilität) hatte die Lösung aus dem rekonstituierten Pulver jeweils ca. 0,7% und 0,7% Aggregate.

Der MMD des Pulvers wurde wie oben beschrieben bestimmt.
- Der MMD des Pulvers lag nach Sprühtrocknung bei 3,2 µm.

Der MMAD und FPF des Pulvers wurde wie oben beschrieben bestimmt.
- Der MMAD des Pulvers lag nach Sprühtrocknung bei 3,9 µ*m* und die Feinpartikelfraktion lag bei 55,6% bezogen auf die Pulvereinwaage in der Kapsel.
   Sprühtrocknung einer LS90P 2,833 %(w/v) Tri-Isoleucin 0,166% (w/v) IgG1 0,33% (w/v) Formulierung

4,25 g LS90P und 0,25 g Tri-Isoleucin wurden in ca. 140 ml demineralisiertem Wasser (pH ca. 7,5) gelöst. Als nächstes wurden ca. 4,864 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3,00% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet. Der Gehalt an Aggregaten wurde wie oben beschrieben untersucht. Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 1,5% Aggregate.
- Nach 1 und 3 Monaten offener Lagerung bei 29% relativer Luftfeuchte und 25°C (offene 3 Monatsstabilität) hatte die Lösung aus dem rekonstituierten Pulver jeweils ca. 0,5% und 0,5% Aggregate.
- Nach 1 und 3 Monaten offener Lagerung bei 43% relativer Luftfeuchte und 25°C (offene 3 Monatsstabilität) hatte die Lösung aus dem rekonstituierten Pulver ca. 0,5% und 0,6% Aggregate.

Der MMD des Pulvers wurde wie oben beschrieben bestimmt.
- Der MMD des Pulvers lag nach Sprühtrocknung bei 4,8 *µ*m.

Der MMAD und FPF des Pulvers wurde wie oben beschrieben bestimmt.
- Der MMAD des Pulvers lag nach Sprühtrocknung bei 5,2 µm und die Feinpartikelfraktion lag bei 45,7 % bezogen auf die Pulvereinwaage in der Kapsel.
   Sprühtrocknung einer LS90P 2,9166 %(w/v) Tri-Isoleucin 0,0833% (w/v) IgG1 0,33% (w/v) Formulierung

4,375 g LS90P und 0,125 g Tri-Isoleucin wurden in ca. 140 ml demineralisiertem Wasser (pH ca. 7,5) gelöst. Als nächstes wurden ca. 4,864 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 3,00% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 1,2% Aggregate.
- Nach 1 und 3 Monaten offener Lagerung bei 29% relativer Luftfeuchte und 25°C (offene 3 Monatsstabilität) hatte die Lösung aus dem rekonstituierten Pulver jeweils ca. 0,4% und 0,5% Aggregate.
- Nach 1 und 3 Monaten offener Lagerung bei 43% relativer Luftfeuchte und 25°C (offene 3 Monatsstabilität) hatte die Lösung aus dem rekonstituierten Pulver ca. 0,5% und 0,6% Aggregate.

Der MMD des Pulvers wurde wie oben beschrieben bestimmt.
- Der MMD des Pulvers lag nach Sprühtrocknung bei 4,2 µm.

Der MMAD und FPF des Pulvers wurde wie oben beschrieben bestimmt.
- Der MMAD des Pulvers lag nach Sprühtrocknung bei 6,1 µm und die Feinpartikelfraktion lag bei 39,6 % bezogen auf die Pulvereinwaage in der Kapsel.
   Sprühtrocknung einer LS55P 2,66% (w/v) Tri-Isoleucin 0,33% (w/v) IgG1 0,33% (w/v) Formulierung

8,0 g LS55P und 1 g Tri-Isoleucin wurden im Ultraschallbad in ca. 280 ml demineralisiertem Wasser (pH ca. 7,5) gelöst. Als nächstes wurden ca. 9,73 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 2,1% Aggregate.
- Nach 1 und 3 Monaten Lagerung bei 2-8°C (3 Monatsstabilität) hatte die Lösung aus dem rekonstituierten Pulver jeweils ca. 0,8% und 1,5% Aggregate.
- Nach 1 und 3 Monaten Lagerung bei 25°C (3 Monatsstabilität) hatte die Lösung aus dem rekonstituierten Pulver jeweils ca. 0,9% und 1,5% Aggregate.
- Nach 1 und 3 Monaten Lagerung bei 40°C (3 Monatsstabilität) hatte die Lösung aus dem rekonstituierten Pulver jeweils ca. 1,3% und 2,6% Aggregate.
- Nach 1 und 3 Monaten offener Lagerung bei 43% relativer Luftfeuchte und 25°C (offene 3 Monatsstabilität) hatte die Lösung aus dem rekonstituierten Pulver ca. 1,0% und 1,0% Aggregate.

Der MMD des Pulvers wurde wie oben beschrieben bestimmt.
- Nach Sprühtrocknung lag der MMD des Pulvers bei 3,4 *µ*m.

Der MMAD und FPF des Pulvers wurde wie oben beschrieben bestimmt.
- Nach Sprühtrocknung lag der MMAD bei 3,9 µm und die Feinpartikelfraktion bezogen auf die Pulvereinwaage in der Kapsel bei 58,3%.
- Nach einmonatiger offener Lagerung bei ca. 43% relativer Luftfeuchte und 25°C lag der MMAD bei 3,8 µm und die Feinpartikelfraktion bezogen auf die Pulvereinwaage in der Kapsel bei 59,6%.
   Sprühtrocknung einer LS55P 2,833% (w/v) Tri-Isoleucin 0,166% (w/v) IgG1 0,33% (w/v) Formulierung

8,5 g LS55P und 0,5 g Tri-Isoleucin wurden im Ultraschallbad in ca. 280 ml demineralisiertem Wasser (pH ca. 7,5) gelöst. Als nächstes wurden ca. 9,73 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 3,4% Aggregate.
- Nach 1 und 3 Monaten offener Lagerung bei 43% relativer Luftfeuchte und 25°C (offene 3 Monatsstabilität) hatte die Lösung aus dem rekonstituierten Pulver ca. 1,3% und 1,5% Aggregate. Der MMD des Pulvers wurde wie oben beschrieben bestimmt.
- Der MMD des Pulvers lag bei 2,9 µm. Der MMAD und FPF des Pulvers wurde wie oben beschrieben bestimmt.
- Der MMAD lag bei 4,4 µm und die Feinpartikelfraktion lag bei 58,6% bezogen auf die Pulvereinwaage in der Kapsel.
   Sprühtrocknung einer LS55P 2,9166% (w/v) Tri-Isoleucin 0,0833% (w/v) IgG1 0,33% (w/v) Formulierung

8,75 g LS55P und 0,25 g Tri-Isoleucin wurden im Ultraschallbad in ca. 280 ml demineralisiertem Wasser (pH ca. 7,5) gelöst. Als nächstes wurden ca. 9,73 ml pures IgG1 mit einer Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II mit einer Zerstäubungsflussrate von ca. 0,67 m³/h sprühgetrocknet.
Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 4,4% Aggregate.
- Nach 1 und 3 Monaten offener Lagerung bei 43% relativer Luftfeuchte und 25°C (offene 3 Monatsstabilität) hatte die Lösung aus dem rekonstituierten Pulver ca. 0,7% und 0,8% Aggregate.

Der MMD des Pulvers wurde wie oben beschrieben bestimmt.
- Der MMD des Pulvers lag bei 2,9 µm.

Der MMAD und FPF des Pulvers wurde wie oben beschrieben bestimmt.
- Der MMAD lag bei 4,4 µm und die Feinpartikelfraktion lag bei 58,6% bezogen auf die Pulvereinwaage in der Kapsel.

### Beispiel 5:

### Herstellung von weiteren erfindungsgemäßen Pulvern Sprühtrocknung einer Lysozym 3,33% (w/v) Formulierung

5 g Lysozym wird in ca. 140 ml demineralisiertem Wasser (pH ca. 7,5) gelöst und mit demineralisiertem Wasser (pH ca. 7,5) auf ein Volumen von 150ml verdünnt. Die so erhaltene Lösung wird wie oben beschrieben unter Verwendung des Zyklon II sprühgetrocknet.
Der Restmonomergehalt wurde wie oben beschrieben untersucht. Nach forcierter Lagerung hatte die Lösung aus dem rekonstituierten Pulver einen Restmonomergehalt von 35,3%. Der MMD des Pulvers wurde wie oben beschrieben bestimmt. Der MMD des Pulvers lag bei 3,2 *µ*m. Der MMAD und FPF des Pulvers wurde wie oben beschrieben bestimmt. Der MMAD lag bei 4,0 *µ*m und die Fine Particle Fraction lag bei 70,4 % bezogen auf die Pulvereinwaage in der Kapsel.
Sprühtrocknung einer LS90P 3,00% (w/v) Lysozym 0,33% (w/v) Formulierung

9,0 g LS90P wird im Ultraschallbad in ca. 280 ml demineralisiertem Wasser (pH ca. 7,5) gelöst. Als nächstes wird 1 g Lysozym, und mit demineralisiertem Wasser (pH ca. 7,5) auf ein Volumen von 300ml verdünnt. Die so erhaltene Lösung wird wie oben beschrieben unter Verwendung des Zyklon II sprühgetrocknet.
Der Restmonomergehalt wurde wie oben beschrieben untersucht. Nach forcierter Lagerung hatte die Lösung aus dem rekonstituierten Pulver einen Restmonomergehalt von 62,1%. Der MMD des Pulvers wurde wie oben beschrieben bestimmt. Der MMD des Pulvers lag bei 4,0 µm. Der MMAD und FPF des Pulvers wurde wie oben beschrieben bestimmt. Der MMAD lag bei 3,7 µm und die Fine Particle Fraction lag bei 24,7 % bezogen auf die Pulvereinwaage in der Kapsel.
Sprühtrocknung einer LS90P 2,66% (w/v) Isoleucin 0,33 % (w/v) Lysozym 0,33% (w/v) Formulierung

8,0 g LS90P und 1 g Isoleucin werden im Ultraschallbad in ca. 280 ml demineralisiertem Wasser (pH ca. 7,5) gelöst. Als nächstes wird 1 g Lysozym, und mit demineralisiertem Wasser (pH ca. 7,5) auf ein Volumen von 300ml verdünnt. Die so erhaltene Lösung wird wie oben beschrieben unter Verwendung des Zyklon II sprühgetrocknet. Der Restmonomergehalt wurde wie oben beschrieben untersucht. Nach forcierter Lagerung hatte die Lösung aus dem rekonstituierten Pulver einen Restmonomergehalt von 47,9%. Der MMD des Pulvers wurde wie oben beschrieben bestimmt. Der MMD des Pulvers lag bei 3,9 *µ*m. Der MMAD und FPF des Pulvers wurde wie oben beschrieben bestimmt. Der MMAD lag bei 4,1 *µ*m und die Fine Particle Fraction lag bei 29,0 % bezogen auf die Pulvereinwaage in der Kapsel.
Sprühtrocknung einer LS90P 2,66% (w/v) Tri-Isoleucin 0,33 % (w/v) Lysozym 0,33% (w/v) Formulierung .

8,0 g LS90P und 1 g Tri-Isoleucin werden im Ultraschallbad in ca. 280 ml demineralisiertem Wasser (pH ca. 7,5) gelöst. Als nächstes wird 1 g Lysozym, zugegeben und mit demineralisiertem Wasser (pH ca. 7,5) auf ein Volumen von 300ml verdünnt. Die so erhaltene Lösung wird wie oben beschrieben unter Verwendung des Zyklon II sprühgetrocknet.
Der Restmonomergehalt wurde wie oben beschrieben untersucht. Nach forcierter Lagerung hatte die Lösung aus dem rekonstituierten Pulver einen Restmonomergehalt von 47,9%. Der MMD des Pulvers wurde wie oben beschrieben bestimmt. Der MMD des Pulvers lag bei 2,7 *µ*m. Der MMAD und FPF des Pulvers wurde wie oben beschrieben bestimmt. Der MMAD lag bei 3,6 *µ*m und die Fine Particle Fraction lag bei 58,6 % bezogen auf die Pulvereinwaage in der Kapsel.
Sprühtrocknung einer Calcitonin 3,33% (w/v) Formulierung

1 g Calcitonin wird in ca. 25 ml demineralisiertem Wasser (pH ca. 7,5) gelöst und mit demineralisiertem Wasser (pH ca. 7,5) auf ein Volumen von 30ml verdünnt. Die so erhaltene Lösung wird wie oben beschrieben unter Verwendung des Zyklon II sprühgetrocknet.
- Der Gehalt an Aggregaten wurde wie oben beschrieben untersucht. Nach 3 Monaten Lagerung bei 2-8°C (3 Monatsstabilität) hatte die Lösung aus dem rekonstituierten Pulver jeweils ca. 4,1% Aggregate.
- Nach 3 Monaten Lagerung bei 25°C (3 Monatsstabilität) hatte die Lösung aus dem rekonstituierten Pulver jeweils ca. 4,9% Aggregate.
- Nach 3 Monaten Lagerung bei 40°C (3 Monatsstabilität) hatte die Lösung aus dem rekonstituierten Pulver jeweils ca. 7,4% Aggregate.

Der MMAD und FPF des Pulvers wurde wie oben beschrieben bestimmt. Der MMAD lag bei 3,9 *µ*m und die Fine Particle Fraction lag bei 59,0 % bezogen auf die Pulvereinwaage in der Kapsel.
Sprühtrocknung einer LS90P 3,166% (w/v) Calcitonin 0,166% (w/v) Formulierung

4,750 g LS90P wird im Ultraschallbad in ca. 140 ml demineralisiertem Wasser (pH ca. 7,5) gelöst. Als nächstes wird 0,250 g Calcitonin zugegeben und mit demineralisiertem Wasser (pH ca. 7,5) auf ein Volumen von 150 ml verdünnt. Die so erhaltene Lösung wird wie oben beschrieben unter Verwendung des Zyklon II sprühgetrocknet.
Der Gehalt an Aggregaten wurde wie oben beschrieben untersucht.
- Nach 3 Monaten Lagerung bei 2-8°C (3 Monatsstabilität) hatte die Lösung aus dem rekonstituierten Pulver jeweils ca. 3,6% Aggregate.
- Nach 3 Monaten Lagerung bei 25°C (3 Monatsstabilität) hatte die Lösung aus dem rekonstituierten Pulver jeweils ca. 3,9% Aggregate.
- Nach 3 Monaten Lagerung bei 40°C (3 Monatsstabilität) hatte die Lösung aus dem rekonstituierten Pulver jeweils ca. 4,6% Aggregate.

Der MMD des Pulvers wurde wie oben beschrieben bestimmt. Der MMD des Pulvers lag bei 2,6 µm. Der MMAD und FPF des Pulvers wurde wie oben beschrieben bestimmt. Der MMAD lag bei 4,3 µm und die Fine Particle Fraction lag bei 47,3 % bezogen auf die Pulvereinwaage in der Kapsel.
Sprühtrocknung einer LS90P 2,833% (w/v) Isoleucin 0,33 % (w/v) Calcitonin 0,166% (w/v) Formulierung

4,250 g LS90P und 0,50 g Isoleucin werden im Ultraschallbad in ca. 140 ml demineralisiertem Wasser (pH ca. 7,5) gelöst. Als nächstes wird 0,250 g Calcitonin zugegeben und mit demineralisiertem Wasser (pH ca. 7,5) auf ein Volumen von 150 ml verdünnt. Die so erhaltene Lösung wird wie oben beschrieben unter Verwendung des Zyklon II sprühgetrocknet.
Der Gehalt an Aggregaten wurde wie oben beschrieben untersucht.
- Der Gehalt an Aggregaten wurde wie oben beschrieben untersucht. Nach 3 Monaten Lagerung bei 2-8°C (3 Monatsstabilität) hatte die Lösung aus dem rekonstituierten Pulver jeweils ca. 3,3% Aggregate.
- Nach 3 Monaten Lagerung bei 25°C (3 Monatsstabilität) hatte die Lösung aus dem rekonstituierten Pulver jeweils ca. 3,6% Aggregate.
- Nach 3 Monaten Lagerung bei 40°C (3 Monatsstabilität) hatte die Lösung aus dem rekonstituierten Pulver jeweils ca. 3,6% Aggregate.

Der MMD des Pulvers wurde wie oben beschrieben bestimmt. Der MMD des Pulvers lag bei 2,8 µm. Der MMAD und FPF des Pulvers wurde wie oben beschrieben bestimmt. Der MMAD lag bei 4,4 µm und die Fine Particle Fraction lag bei 49,2 % bezogen auf die Pulvereinwaage in der Kapsel.
Sprühtrocknung einer LS90P 2,866% (w/v) Tri-Isoleucin 0,33 % (w/v) Calcitonin 0,166% (w/v) Formulierung

4,250 g LS90P und 0,50 g Tri-Isoleucin werden im Ultraschallbad in ca. 140 ml demineralisiertem Wasser (pH ca. 7,5) gelöst. Als nächstes wird 0,250 g Calcitonin zugegeben und mit demineralisiertem Wasser (pH ca. 7,5) auf ein Volumen von 150 ml verdünnt. Die so erhaltene Lösung wird wie oben beschrieben unter Verwendung des Zyklon II sprühgetrocknet.
- Der Gehalt an Aggregaten wurde wie oben beschrieben untersucht. Nach 3 Monaten Lagerung bei 2-8°C (3 Monatsstabilität) hatte die Lösung aus dem rekonstituierten Pulver jeweils ca. 3,3% Aggregate.
- Nach 3 Monaten Lagerung bei 25°C (3 Monatsstabilität) hatte die Lösung aus dem rekonstituierten Pulver jeweils ca. 3,6% Aggregate.
- Nach 3 Monaten Lagerung bei 40°C (3 Monatsstabilität) hatte die Lösung aus dem rekonstituierten Pulver jeweils ca. 3,9% Aggregate.

Der MMD des Pulvers wurde wie oben beschrieben bestimmt. Der MMD des Pulvers lag bei 2,5 µm. Der MMAD und FPF des Pulvers wurde wie oben beschrieben bestimmt. Der MMAD lag bei 3,5 µm und die Fine Particle Fraction lag bei 60,4 % bezogen auf die Pulvereinwaage in der Kapsel.

### Beispiel 6

### Gefriertrocknung einer IgG1 5% (w/v) Formulierung

Pures IgG1 mit einer Konzentration von ca. 109 mg/ml, formuliert in einem Glycin Histidin Puffer pH 6 (siehe Materialien), wurde mit demineralisiertem Wasser (pH ca. 7,5) auf einen Gehalt von 50mg/ml verdünnt und in Abwesenheit von weiteren Hilfsstoffen gefriergetrocknet. Das Volumen der Lösung umfasste 50 ml und wurde vor der Gefriertrockung auf handelsübliche 2R Vials verteilt. Das Lyophilisat wurde in den 2R Vials mittels eines Spatels lyophilisert und wie oben beschrieben weiter behandelt. Der Gehalt an Aggregaten wurde wie oben beschrieben untersucht.
- Nach einwöchiger offener Lagerung bei 75% relativer Luftfeuchtigkeit und 40°C (Forcierte Lagerstabilität) hatte die Lösung aus dem rekonstituierten Pulver ca. 20,5% Aggregate.
- Nach einem Tag Equilibrierung und vier Wochen trockener Lagerung bei 40°C hatte die Lösung aus dem rekonstituierten Pulver ca. 15,3% Aggregate.
- Nach einem Tag Vakuumtrocknung und vier Wochen trockener Lagerung bei 40°C hatte die Lösung aus dem rekonstituierten Pulver ca. 12,6% Aggregate.
   Gefriertrocknung einer Mannitol 4,5% (w/v) IgG1 0,5% (w/v) Formulierung

2,25 g Mannitol wurde in ca. 40 ml demineralisiertem Wasser (pH ca. 7,5) gelöst. Als nächstes wurden ca. 2,3 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 4,5% (w/v) Hilfsstoff oder Matrix sowie 0,5% (w/v) Protein, wurde auf handelsübliche 2R Vials verteilt, und wie oben beschrieben gefriergetrocknet. Das Lyophilisat wurde in den 2R Vials mittels eines Spatels lyophilisert und wie oben beschrieben weiter behandelt. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 34,0% Aggregate.
- Nach einem Tag Equilibrierung und vier Wochen trockener Lagerung bei 40°C hatte die Lösung aus dem rekonstituierten Pulver ca. 11,6% Aggregate.
- Nach einem Tag Vakuumtrocknung und vier Wochen trockener Lagerung bei 40°C hatte die Lösung aus dem rekonstituierten Pulver ca. 6,2% Aggregate.

### Gefriertrocknung einer LS55P 4,5% (w/v) IgG1 0,5% (w/v) Formulierung

2,25 g LS55P wurde in ca. 40 ml demineralisiertem Wasser (pH ca. 7,5) gelöst. Als nächstes wurden ca. 2,3 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 4,5% (w/v) Hilfsstoff oder Matrix sowie 0,5% (w/v) Protein, wurde auf handelsübliche 2R Vials verteilt, und wie oben beschrieben gefriergetrocknet. Das Lyophilisat wurde in den 2R Vials mittels eines Spatels lyophilisert und wie oben beschrieben weiter behandelt. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 2,5% Aggregate.
- Nach einem Tag Equilibrierung und vier Wochen trockener Lagerung bei 40°C hatte die Lösung aus dem rekonstituierten Pulver ca. 2,6% Aggregate.
- Nach einem Tag Vakuumtrocknung und vier Wochen trockener Lagerung bei 40°C hatte die Lösung aus dem rekonstituierten Pulver ca. 1,2% Aggregate.
   Gefriertrocknung einer Coupling Sugar 4,5% (w/v) IgG1 0,5% (w/v) Formulierung

2,25 g Coupling Sugar wurde in ca. 40 ml demineralisiertem Wasser (pH ca. 7,5) gelöst. Als nächstes wurden ca. 2,3 ml pures IgG1 mit einer Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 4,5% (w/v) Hilfsstoff oder Matrix sowie 0,5% (w/v) Protein, wurde auf handelsübliche 2R Vials verteilt, und wie oben beschrieben gefriergetrocknet. Das Lyophilisat wurde in den 2R Vials mittels eines Spatels lyophilisert und wie oben beschrieben weiter behandelt. Der Gehalt an Aggregaten wurde wie oben beschrieben untersucht.
Für die Lagerstabilität ergaben sich folgende Aggregatgehalte.
- Nach einwöchiger offener Lagerung bei 75% relativer Luftfeuchtigkeit und 40°C (Forcierte Lagerstabilität) hatte die Lösung aus dem rekonstituierten Pulver ca. 5,5% Aggregate.
- Nach einem Tag Equilibrierung und vier Wochen trockener Lagerung bei 40°C hatte die Lösung aus dem rekonstituierten Pulver ca. 4,6% Aggregate.
- Nach einem Tag Vakuumtrocknung und vier Wochen trockener Lagerung bei 40°C hatte die Lösung aus dem rekonstituierten Pulver ca. 1,5% Aggregate.

## Patentansprüche

1. Pulver enthaltend einen pharmazeutischen Wirkstoff und eine Hilfsstoffkombination enthaltend zumindest ein 1,4 O-verknüpftes Saccharose-Derivat ausgewählt aus den Verbindungen:
1,4 O-verknüpfte D-Gal-Saccharose (Lactosucrose);
1,4 O-verknüpfte D-Glu-Saccharose (Glucosyl-Sucrose); oder
1,4 O-verknüpfte Glu-Glu-Saccharose (Maltosyl-Sucrose)
und einen weiteren Hilfsstoff.

2. Pulver nach Anspruch 1 ***dadurch gekennzeichnet,* dass** es Lactosucrose als Saccharose-Derivat enthält.

3. Pulver nach Anspruch 2 ***dadurch gekennzeichnet,* dass** es sich bei dem weiteren Hilfsstoff um ein Mono- oder Di-Saccharid, vorzugsweise um Lactose und Saccharose handelt.

4. Pulver nach einem der Ansprüche 1 bis 3 ***dadurch gekennzeichnet,* dass** der Anteil an Lactosucrose zumindest 55% (w/w) in Bezug auf den im Pulver enthaltenen Zuckeranteil beträgt.

5. Pulver nach Anspruch 1 ***dadurch gekennzeichnet,* dass** es ein Gemisch aus Glucosyl-Sucrose und Maltosyl-Sucrose enthält.

6. Pulver nach Anspruch 5 ***dadurch gekennzeichnet,* dass** es weitere Mono- oder Di-Saccharide als zusätzliche Hilfsstoffe enthält, vorzugsweise Fructose, Glucose und/oder Saccharose.

7. Pulver nach Anspruch 5 oder 6 ***dadurch gekennzeichnet,* dass** der Gesamtanteil an Glucosyl-Sucrose und Maltosyl-Sucrose zumindest 25% (w/w) in Bezug auf den im Pulver enthaltenen Zuckeranteil beträgt.

8. Pulver nach Anspruch 7 ***dadurch gekennzeichnet,* dass** der jeweilige Anteil an Maltosyl-Sucrose und Glucosyl-Sucrose zumindest 18% (w/w) in Bezug auf den im Pulver enthaltenen Zuckeranteil beträgt.

9. Pulver nach einem der Ansprüche 1 bis 8 ***dadurch gekennzeichnet,* dass** es sich bei dem weiteren Hilfsstoff um Aminosäuren, Peptide, weitere Zucker, Zuckeralkohole, Polymere und/oder pharmazeutisch verträgliche Salze handelt.

10. Pulver nach Anspruch 9 ***dadurch gekennzeichnet,* dass** das Pulver zusätzlich zumindest eine Aminosäure oder ein Peptid als Hilfsstoff enthält, wobei das zusätzliche Pepid nicht dem pharmazeutischen Wirkstoff entspricht.

11. Pulver nach Anspruch 10 ***dadurch gekennzeichnet,* dass** es sich bei der Aminosäure um Isoleucin handelt.

12. Pulver nach Anspruch 10 ***dadurch gekennzeichnet,* dass** es sich bei dem Peptid um ein Di- oder Tri-Peptid handelt.

13. Pulver nach Anspruch 10 ***dadurch gekennzeichnet,* dass** es sich bei dem Peptid um ein Peptid mit einem, zwei oder mehreren Isoleucinresten handelt.

14. Pulver nach Anspruch 10 ***dadurch gekennzeichnet,* dass** es sich bei dem Tri-Peptid um Tri-Isoleucin handelt.

15. Pulver nach Anspruch 11 ***dadurch gekennzeichnet,* dass** die Trockenmasse des Pulvers zwischen 60 und 80% (w/w) eines 1,4 O-verknüpften Saccharose-Derivats oder eine Zuckermischung, die zumindest ein 1,4 O-verknüpftes Saccharose-Derivat enthält, und zwischen 1 und 19,99% (w/w) Isoleucin enthält.

16. Pulver nach Anspruch 10 ***dadurch gekennzeichnet,* dass** die Trockenmasse des Pulvers zwischen 60 und 80% (w/w) eines 1,4 O-verknüpften Saccharose-Derivats oder eine Zuckermischung, die zumindest ein 1,4 O-verknüpftes Saccharose-Derivat enthält, und zwischen 1 und 19,99% (w/w) eines Peptids enthält.

17. Pulver nach Anspruch 16, ***dadurch gekennzeichnet,* dass** es sich bei dem Peptid um Tri-Isoleucin handelt.

18. Pulver nach einem der Ansprüche 1 bis 17 ***dadurch gekennzeichnet,* dass** der Anteil der Hilfsstoffkombination zwischen 25 und 99,99% (w/w) der Trockenmasse des Pulvers beträgt.

19. Pulver nach einem der Ansprüche 1 bis 18 ***dadurch gekennzeichnet,* dass** der Anteil an pharmazeutischem Wirkstoff zwischen 0,01 und 75% (w/w) der Trockenmasse des Pulvers beträgt, wobei die Summe der Gewichtsprozente 100% (w/w) beträgt.

20. Pulver nach einem der Ansprüche 1 bis 19 ***dadurch gekennzeichnet,* dass** es sich bei dem pharmazeutischen Wirkstoff um ein biologisches Makromolekül handelt.

21. Pulver nach einem der Ansprüche 1 bis 20 ***dadurch gekennzeichnet,* dass** die Trockenmasse des Pulvers zwischen 60 und 90% (w/w) einer Hilfsstoffkombination mit zumindest einem 1,4 O-verknüpften Saccharose-Derivat oder einer Zuckermischung, die zumindest ein 1,4 O-verknüpftes Saccharose-Derivat enthält und bis zu 40% (w/w) eines pharmazeutischen Wirkstoff enthält, wobei der Anteil an Lactosucrose, Maltosyl-Sucrose und/oder Glucosyl-Sucrose mindestens 20% (w/w) in Bezug auf die Trockenmasse des Pulvers beträgt, wobei die Summe der Gewichtsprozente maximal 100% (w/w) beträgt.

22. Pulver nach einem der Ansprüche 1 bis 21 ***dadurch gekennzeichnet,* dass** die Partikel im Pulver über eine mittlere Teilchengröße (MMD) zwischen 1 und 10 µm verfügen.

23. Pulver nach einem der Ansprüche 1 bis 22, ***gekennzeichnet**,* dass es sich bei dem Pulver um ein sprühgetrocknetes oder gefriergetrocknetes Pulver handelt.

24. Pulver nach einem der Ansprüche 1 bis 23, ***dadurch gekennzeichnet,* dass** die Partikel im Pulver über einen mittleren aerodynamischen Teilchendurchmesser (MMAD) zwischen 1 und 5 µm verfügen.

25. Pharmazeutische Zusammensetzung enthaltend ein Pulver nach einem der Ansprüche 1 bis 24.

26. Verfahren zur Herstellung eines Pulvers gemäß Anspruch 1 bis 24 ***dadurch gekennzeichnet,* dass**
a) ein pharmazeutischer Wirkstoff in einer wässrigen Lösung / Suspension gelöst wird;
b) eine Hilfsstoffkombination aus zumindest einem 1,4 O-verknüpften Saccharose-Derivat ausgewählt aus den Verbindungen Lactosucrose, Glucosyl-Sucrose, oder Maltosyl-Sucrose in Kombination mit zumindest einem weiteren Hilfsstoff in einer wässrigen Lösung / Suspension gelöst wird;
c) sofern Wirkstoff und die Hilfsstoffkombination aus zumindest einem 1,4 O-verknüpften Saccharose-Derivat in Kombination mit zumindest einem weiteren Hilfsstoff in verschiedenen Lösungen / Suspension gelöst wird, diese gemischt werden;
d) die Lösung / Suspension enthaltend die Hilfsstoffkombination aus zumindest einem 1,4 O-verknüpften Saccharose-Derivat in Kombination mit zumindest einem weiteren Hilfsstoff und den pharmazeutischen Wirkstoff getrocknet werden.

27. Verfahren nach Anspruch 26 ***dadurch gekennzeichnet,* dass** es sich bei dem Trocknungsverfahren um Sprühtrocknung oder um Gefriertrocknung handelt.

28. Verfahren nach Anspruch 26 oder 27 ***dadurch gekennzeichnet,* dass** es sich bei dem pharmazeutischen Wirkstoff um ein biologisches Makromolekül handelt.

29. Verfahren nach einem der Ansprüche 26 bis 28 ***dadurch gekennzeichnet,* dass** es sich bei dem 1,4 O-verknüpften Saccharose-Derivat um Lactosucrose handelt.

30. Verfahren nach Anspruch 29 ***dadurch gekennzeichnet,* dass** die zu trocknende Lösung oder Suspension Lactose und Saccharose als weitere Hilfsstoffe enthält.

31. Verfahren nach Anspruch 29 oder 30 ***dadurch gekennzeichnet,* dass** der Anteil an Lactosucrose zumindest 55% (w/w) des in der zu trocknende Lösung oder Suspension vorliegenden Zuckeranteils beträgt.

32. Verfahren nach Anspruch 26 oder 27 ***dadurch gekennzeichnet,* dass** es sich bei dem 1,4 O-verknüpften Saccharose-Derivat um ein Gemisch aus Glucosyl-Sucrose und Maltosyl-Sucrose handelt.

33. Verfahren nach Anspruch 32 ***dadurch gekennzeichnet,* dass** die zu trocknende Lösung oder Suspension als zusätzliche Hilfsstoffe weitere Mono- und Di-Saccharide, vorzugsweise Fructose, Saccharose und/oder Glucose enthält.

34. Verfahren nach Anspruch 32 oder 33 ***dadurch gekennzeichnet,* dass** der Gesamtanteil an Glucosyl-Sucrose und Maltosyl-Sucrose zumindest 25% (w/w) des in der zu trocknenden Lösung oder Suspension vorliegenden Zuckeranteils beträgt.

35. Verfahren nach Anspruch 34 ***dadurch gekennzeichnet,* dass** der jeweilige Anteil an Maltosyl-Sucrose und Glucosyl-Sucrose zumindest 18% (w/w) des in der zu trocknenden Lösung oder Suspension vorliegenden Zuckeranteils beträgt.

36. Verfahren nach einem der Ansprüche 26 bis 35 ***dadurch gekennzeichnet,* dass** die zu trocknende Lösung oder Suspension als weitere Hilfsstoffe Aminosäuren, Peptide, weitere Zucker, Zuckeralkohole, Polymere und/oder pharmazeutisch verträgliche Salze enthält.

37. Verfahren nach Anspruch 36 ***dadurch gekennzeichnet,* dass** es sich bei dem zusätzlichen Hilfsstoff um eine Aminosäure handelt.

38. Verfahren nach Anspruch 37 ***dadurch gekennzeichnet,* dass** es sich bei der Aminosäure um Isoleucin handelt.

39. Verfahren nach Anspruch 36 ***dadurch gekennzeichnet,* dass** es sich bei dem zusätzlichen Hilfsstoff um ein Peptid handelt, wobei das Peptid nicht dem pharmazeutischen Wirkstoff entspricht.

40. Verfahren nach Anspruch 39 ***dadurch gekennzeichnet,* dass** es sich bei dem Peptid um ein Isoleucin-haltiges Peptid handelt.

41. Verfahren nach Anspruch 39 ***dadurch gekennzeichnet,* dass** es sich bei dem Peptid um ein Di- oder Tri-Peptid handelt.

42. Verfahren nach Anspruch 41 ***dadurch gekennzeichnet,* dass** es sich bei dem Tri-Peptid um Tri-Isoleucin handelt.

43. Verfahren nach Anspruch 38 ***dadurch gekennzeichnet,* dass** die Trockenmasse der zu trocknenden Lösung oder Suspension zwischen 60 und 80% (w/w) eines 1,4 O-verknüpften Saccharose-Derivats oder einer Zuckermischung, die zumindest ein 1,4 O-verknüpftes Saccharose-Derivat enthält und zwischen 1 bis 19,99% (w/w) Isoleucin enthält, wobei die Summe der Gewichtsprozente maximal 100% (w/w) beträgt.

44. Verfahren nach Anspruch 41 oder 42 ***dadurch gekennzeichnet,* dass** die Trockenmasse der zu trocknenden Lösung oder Suspension zwischen 60 und 80% (w/w) eines 1,4 O-verknüpften Saccharose-Derivats oder eine Zuckermischung, die zumindest ein 1,4 O-verknüpftes Saccharose-Derivat enthält und zwischen 1 und 19,99% (w/w) eines Tri-Peptids enthält.

45. Verfahren nach einem der Ansprüche 26 bis 44 ***dadurch gekennzeichnet,* dass** der Hilffstoffanteil zwischen 25 und 99% (w/w) der Trockenmasse der zu trocknenden Lösung oder der Suspension beträgt.

46. Verfahren nach einem der Ansprüche 26 bis 45 ***dadurch gekennzeichnet,* dass** der Anteil an pharmazeutischem Wirkstoff zwischen 0,01 und 75% (w/w) der Trockenmasse der zu trocknenden Lösung oder der Suspension beträgt, wobei die Summe der Gewichtsprozente maximal 100% beträgt.

47. Verwendung des getrockneten Pulvers nach einem der Ansprüche 1 bis 24 zur Herstellung eines Arzneimittels.

48. Verwendung des sprühgetrockneten Pulvers nach Anspruch 24 zur Herstellung eines inhalativen Arzneimittels.

## Claims

1. Powder comprising a pharmaceutical active substance and a combination of excipients comprising at least one 1,4 O-linked saccharose derivative selected from the compounds:
1,4 O-linked D-Gal-saccharose (lactosucrose);
1,4 O-linked D-Glu-saccharose (glucosyl sucrose); or
1,4 O-linked Glu-Glu-saccharose (maltosyl sucrose)
and a further excipient.

2. Powder according to Claim 1, ***characterized in that*** it comprises lactosucrose as the saccharose derivative.

3. Powder according to Claim 2, ***characterized in that*** the other excipient is a mono- or disaccharide, preferably lactose and saccharose.

4. Powder according to one of Claims 1 to 3, ***characterized in that*** the lactosucrose content is at least 55% (w/w) of the sugar content in the powder.

5. Powder according to Claim 1, ***characterized in that*** it comprises a mixture of glucosyl sucrose and maltosyl sucrose.

6. Powder according to Claim 5, ***characterized in that*** it contains further mono- or disaccharides as additional excipients, preferably fructose, glucose and/or saccharose.

7. Powder according to Claim 5 or 6, ***characterized in that*** the total content of glucosyl sucrose and maltosyl sucrose is at least 25% (w/w) of the sugar content in the powder.

8. Powder according to Claim 7, ***characterized in that*** the respective content of maltosyl sucrose and glucosyl sucrose is at least 18% (w/w) of the sugar content in the powder.

9. Powder according to one of Claims 1 to 8, ***characterized in that*** the other excipient is an amino acid, peptide, another sugar, a sugar alcohol, a polymer and/or a pharmaceutically acceptable salt.

10. Powder according to Claim 9, ***characterized in that*** the powder additionally comprises at least one amino acid or one peptide as an excipient, wherein the additional peptide is not identical to the pharmaceutical active substance.

11. Powder according to Claim 10, ***characterized in that*** the amino acid is isoleucine.

12. Powder according to Claim 10, ***characterized in that*** the peptide is a di- or tripeptide.

13. Powder according to Claim 10, ***characterized in that*** the peptide is a peptide with one, two or several isoleucine residues.

14. Powder according to Claim 10, ***characterized in that*** the tripeptide is tri-isoleucine.

15. Powder according to Claim 11, ***characterized in that*** the dry mass of the powder comprises between 60 and 80% (w/w) of a 1,4 O-linked saccharose derivative or a sugar mixture which comprises at least one 1,4 O-linked saccharose derivative, and comprises between 1 and 19.99% (w/w) isoleucine.

16. Powder according to Claim 10, ***characterized in that*** the dry mass of the powder comprises between 60 and 80% (w/w) of a 1,4 O-linked saccharose derivative or a sugar mixture which comprises at least one 1,4 O-linked saccharose derivative, and comprises between 1 and 19.99% (w/w) of a peptide.

17. Powder according to Claim 16, ***characterized in that*** the peptide is tri-isoleucine.

18. Powder according to one of Claims 1 to 17, ***characterized in that*** the content of the excipient combination is between 25 and 99.99% (w/w) of the dry mass of the powder.

19. Powder according to one of Claims 1 to 18, ***characterized in that*** the content of pharmaceutical active substance is between 0.01 and 75% (w/w) of the dry mass of the powder and the sum of the weight percentages is 100% (w/w).

20. Powder according to one of Claims 1 to 19, ***characterized in that*** the pharmaceutical active substance is a biological macromolecule.

21. Powder according to one of Claims 1 to 20, ***characterized in that*** the dry mass of the powder comprises between 60 and 90% (w/w) of a combination of excipients with at least one 1,4 O-linked saccharose derivative or a sugar mixture which comprises at least one 1,4 O-linked saccharose derivative, and comprises up to 40% (w/w) of a pharmaceutical active substance, and the content of lactosucrose, maltosyl sucrose and/or glucosyl sucrose is at least 20% (w/w) of the dry mass of the powder and the maximum sum of the weight percentages is 100% (w/w).

22. Powder according to one of Claims 1 to 21, ***characterized in that*** the particles in the powder have a mass mean diameter (MMD) between 1 and 10 µm.

23. Powder according to one of Claims 1 to 22, ***characterized in that*** the powder is a spray-dried or freeze-dried powder.

24. Powder according to one of Claims 1 to 23, ***characterized in that*** the particles in the powder have a mass mean aerodynamic diameter (MMAD) between 1 and 5 µm.

25. Pharmaceutical formulation comprising a powder according to one of Claims 1 to 24.

26. Method of preparing a powder according to Claims 1 to 24, ***characterized in that***
a) a pharmaceutical active substance is dissolved in an aqueous solution/suspension;
b) an excipient combination of at least one 1,4 O-linked saccharose derivative selected from the compounds lactosucrose, glucosyl sucrose or maltosyl sucrose in combination with at least one further excipient is dissolved in an aqueous solution/suspension;
c) if the active substance and the excipient combination of at least one 1,4 O-linked saccharose derivative combined with at least one further excipient are dissolved in different solutions/suspension, these solutions are mixed;
d) the solution/suspension comprising the excipient combination of at least one 1,4 O-linked saccharose derivative combined with at least one further excipient and the pharmaceutical active substance is dried.

27. Method according to Claim 26, ***characterized in that*** the drying method is spray-drying or freeze-drying.

28. Method according to Claim 26 or 27, ***characterized in that*** the pharmaceutical active substance is a biological macromolecule.

29. Method according to one of Claims 26 to 28, ***characterized in that*** the 1,4 O-linked saccharose derivative is lactosucrose.

30. Method according to Claim 29, ***characterized in that*** the solution or suspension to be dried comprises lactose and saccharose as further excipients.

31. Method according to Claim 29 or 30, ***characterized in that*** the content of lactosucrose is at least 55% (w/w) or the sugar content present in the solution or suspension to be dried.

32. Method according to Claim 26 or 27, ***characterized in that*** the 1,4 O-linked saccharose derivative is a mixture of glucosyl sucrose and maltosyl sucrose.

33. Method according to Claim 32, ***characterized in that*** the solution or suspension to be dried comprises further mono- and disaccharides, preferably fructose, saccharose and/or glucose as additional excipients.

34. Method according to Claim 32 or 33, ***characterized in that*** the total content of glucosyl sucrose and maltosyl sucrose is at least 25% (w/w) of the sugar content present in the solution or suspension to be dried.

35. Method according to Claim 34, ***characterized in that*** the respective content of maltosyl sucrose and glucosyl sucrose is at least 18% (w/w) of the sugar content present in the solution or suspension to be dried.

36. Method according to one of Claims 26 to 35, ***characterized in that*** the solution or suspension to be dried contains amino acids, peptides, further sugars, sugar alcohols, polymers and/or pharmaceutically acceptable salts as further excipients.

37. Method according to Claim 36, ***characterized in that*** the additional excipient is an amino acid.

38. Method according to Claim 37, ***characterized in that*** the amino acid is isoleucine.

39. Method according to Claim 36, ***characterized in that*** the additional excipient is a peptide, **characterized in that** the peptide is not identical to the pharmaceutical active substance.

40. Method according to Claim 39, ***characterized in that*** the peptide is an isoleucine-containing peptide.

41. Method according to Claim 39, ***characterized in that*** the peptide is a di- or tripeptide.

42. Method according to Claim 41, ***characterized in that*** the tripeptide is a tri-isoleucine.

43. Method according to Claim 38, ***characterized in that*** the dry mass of the solution or suspension to be dried is between 60 and 80% (w/w) of a 1,4 O-linked saccharose derivative or a sugar mixture which comprises at least one 1,4 O-linked saccharose derivative and between 1 to 19.99% (w/w) isoleucine, wherein the maximum sum of the weight percentages is 100% (w/w).

44. Method according to Claim 41 or 42, ***characterized in that*** the dry mass of the solution or suspension to be dried is between 60 and 80% (w/w) of a 1,4 O-linked saccharose derivative or a sugar mixture which comprises at least one 1,4 O-linked saccharose derivative and between 1 and 19.99% (w/w) of a tripeptide.

45. Method according to one of Claims 26 to 44, ***characterized in that*** the content of excipient is between 25 and 99% (w/w) of the dry mass of the solution or suspension to be dried.

46. Method according to one of Claims 26 to 45, ***characterized in that*** the content of pharmaceutical active substance is between 0.01 and 75% (w/w) of the dry mass of the solution or suspension to be dried, **characterized in that** the maximum sum of the weight percentages is 100%.

47. Use of the dried powder according to one of Claims 1 to 24 for the preparation of a medicinal product.

48. Use of the spray-dried powder according to Claim 24 for the preparation of a medicinal product for inhalation.

## Revendications

1. Poudre contenant un principe actif pharmaceutique et une combinaison d'adjuvants contenant au moins un dérivé du saccharose lié 1,4 O choisi parmi les composés :
D-Gal-saccharose lié 1,4 O (lactosucrose) ;
D-Glu-saccharose lié 1,4 O (glucosyl-sucrose) ; ou
Glu-Glu-saccharose lié 1,4 O (maltosyl-sucrose)
et un autre adjuvant.

2. Poudre selon la revendication 1 **caractérisée en ce qu'**elle contient du lactosucrose comme dérivé du saccharose.

3. Poudre selon la revendication 2 **caractérisée en ce que** l'autre adjuvant est un mono- ou disaccharide, de préférence le lactose et le saccharose.

4. Poudre selon l'une des revendications 1 à 3 **caractérisée en ce que** la proportion de lactosucrose est d'au moins 55 % (m/m) par rapport à la proportion de sucre contenue dans la poudre.

5. Poudre selon la revendication 1 **caractérisée en ce qu'**elle contient un mélange de glucosyl-sucrose et de maltosyl-sucrose.

6. Poudre selon la revendication 5 **caractérisée en ce qu'**elle contient d'autres mono- ou disaccharides comme adjuvants supplémentaires, de préférence le fructose, le glucose et/ou le saccharose.

7. Poudre selon la revendication 5 ou 6 **caractérisée en ce que** la proportion totale de glucosyl-sucrose et de maltosyl-sucrose est d'au moins 25 % (m/m) par rapport à la proportion de sucre contenue dans la poudre.

8. Poudre selon la revendication 7 **caractérisée en ce que** la proportion de maltosyl-sucrose et la proportion de glucosyl-sucrose sont d'au moins 18 % (m/m) par rapport à la proportion de sucre contenue dans la poudre.

9. Poudre selon l'une des revendications 1 à 8 **caractérisée en ce que**, concernant l'autre adjuvant, il s'agit d'aminoacides, de peptides, d'autres sucres, d'alcools dérivés de sucres, de polymères et/ou de sels pharmaceutiquement acceptables.

10. Poudre selon la revendication 9 **caractérisée en ce que** la poudre contient en outre au moins un aminoacide ou un peptide comme adjuvant, où le peptide supplémentaire ne correspond pas au principe actif pharmaceutique.

11. Poudre selon la revendication 10 **caractérisée en ce que** l'aminoacide est l'isoleucine.

12. Poudre selon la revendication 10 **caractérisée en ce que** le peptide est un di- ou tripeptide.

13. Poudre selon la revendication 10 **caractérisée en ce que** le peptide est un peptide ayant un, deux ou plusieurs groupements isoleucine.

14. Poudre selon la revendication 10 **caractérisée en ce que** le tripeptide est la tri-isoleucine.

15. Poudre selon la revendication 11 **caractérisée en ce que** la masse sèche de la poudre contient entre 60 et 80 % (m/m) d'un dérivé du saccharose lié 1,4 O ou un mélange de sucres, qui contient au moins un dérivé du saccharose lié 1,4 O, et entre 1 et 19,99 % (m/m) d'isoleucine.

16. Poudre selon la revendication 10 **caractérisée en ce que** la masse sèche de la poudre contient entre 60 et 80 % (m/m) d'un dérivé du saccharose lié 1,4 O ou un mélange de sucres, qui contient au moins un dérivé du saccharose lié 1,4 0, et entre 1 et 19,99 % (m/m) d'un peptide.

17. Poudre selon la revendication 16 **caractérisée en ce que** le peptide est la tri-isoleucine.

18. Poudre selon l'une des revendications 1 à 17 **caractérisée en ce que** la proportion de la combinaison d'adjuvants est entre 25 et 99,99 % (m/m) de la masse sèche de la poudre.

19. Poudre selon l'une des revendications 1 à 18 **caractérisée en ce que** la proportion de principe actif pharmaceutique est entre 0,01 et 75 % (m/m) de la masse sèche de la poudre, la somme des pourcentages en masse étant 100 % (m/m).

20. Poudre selon l'une des revendications 1 à 19 **caractérisée en ce que** le principe actif pharmaceutique est une macromolécule biologique.

21. Poudre selon l'une des revendications 1 à 20 **caractérisée en ce que** la masse sèche de la poudre contient entre 60 et 90 % (m/m) d'une combinaison d'adjuvants avec au moins un dérivé du saccharose lié 1,4 O ou d'un mélange de sucres qui contient au moins un dérivé du saccharose lié 1,4 O et jusqu'à 40 % (m/m) d'un principe actif pharmaceutique, où la proportion de lactosucrose, de maltosyl-sucrose et/ou de glucosyl-sucrose est d'au moins 20 % (m/m) par rapport à la masse sèche de la poudre, la somme des pourcentages en masse étant 100 % (m/m) au maximum.

22. Poudre selon l'une des revendications 1 à 21 **caractérisée en ce que** les particules dans la poudre disposent d'une taille particulaire moyenne (MMD) entre 1 et 10 *µ*m.

23. Poudre selon l'une des revendications 1 à 22 **caractérisée en ce que** la poudre est une poudre séchée par pulvérisation ou lyophilisée.

24. Poudre selon l'une des revendications 1 à 23 **caractérisée en ce que** les particules dans la poudre disposent d'un diamètre particulaire aérodynamique moyen (MMAD) entre 1 et 5 µm.

25. Composition pharmaceutique contenant une poudre selon l'une des revendications 1 à 24.

26. Procédé de production d'une poudre selon les revendications 1 à 24 **caractérisé en ce que**
a) un principe actif pharmaceutique est dissous dans une solution/suspension aqueuse ;
b) une combinaison d'adjuvants d'au moins un dérivé du saccharose lié 1,4 O choisi parmi les composés lactosucrose, glucosyl-sucrose ou maltosyl-sucrose en combinaison avec au moins un autre adjuvant est dissoute dans une solution/suspension aqueuse ;
c) dans la mesure où le principe actif et la combinaison d'adjuvants d'au moins un dérivé du saccharose lié 1,4 O en combinaison avec au moins un autre adjuvant sont dissous dans des solutions/suspensions différentes, celles-ci sont mélangées ;
d) la solution/suspension contenant la combinaison d'adjuvants d'au moins un dérivé du saccharose lié 1,4 O en combinaison avec au moins un autre adjuvant et le principe actif pharmaceutique est séchée.

27. Procédé selon la revendication 26 **caractérisé en ce que** le procédé de séchage est le séchage par pulvérisation ou la lyophilisation.

28. Procédé selon la revendication 26 ou 27 **caractérisé en ce que** le principe actif pharmaceutique est une macromolécule biologique.

29. Procédé selon l'une des revendications 26 à 28 **caractérisé en ce que** le dérivé du saccharose lié 1,40 est le lactosucrose.

30. Procédé selon la revendication 29 **caractérisé en ce que** la solution ou suspension à sécher contient du lactose et du saccharose comme autres adjuvants.

31. Procédé selon la revendication 29 ou 30 **caractérisé en ce que** la proportion de lactosucrose est d'au moins 55 % (m/m) de la proportion de sucre présente dans la solution ou suspension à sécher.

32. Procédé selon la revendication 26 ou 27 **caractérisé en ce que** le dérivé du saccharose lié 1,4 O est un mélange de glucosyl-sucrose et de maltosyl-sucrose.

33. Procédé selon la revendication 32 **caractérisé en ce que** la solution ou suspension à sécher contient comme adjuvants supplémentaires d'autres mono- et disaccharides, de préférence le fructose, le saccharose et/ou le glucose.

34. Procédé selon la revendication 32 ou 33 **caractérisé en ce que** la proportion totale de glucosyl-sucrose et de maltosyl-sucrose est d'au moins 25 % (m/m) de la proportion de sucre présente dans la solution ou suspension à sécher.

35. Procédé selon la revendication 34 **caractérisé en ce que** la proportion de maltosyl-sucrose et la proportion de glucosyl-sucrose sont d'au moins 18 % (m/m) de la proportion de sucre présente dans la solution ou suspension à sécher.

36. Procédé selon l'une des revendications 26 à 35 **caractérisé en ce que** la solution ou suspension à sécher contient comme autres adjuvants des aminoacides, des peptides, d'autres sucres, des alcools dérivés de sucres, des polymères et/ou des sels pharmaceutiquement acceptables.

37. Procédé selon la revendication 36 **caractérisé en ce que** l'adjuvant supplémentaire est un aminoacide.

38. Procédé selon la revendication 37 **caractérisé en ce que** l'aminoacide est l'isoleucine.

39. Procédé selon la revendication 36 **caractérisé en ce que** l'adjuvant supplémentaire est un peptide, le peptide ne correspondant pas au principe actif pharmaceutique.

40. Procédé selon la revendication 39 **caractérisé en ce que** le peptide est un peptide contenant de l'isoleucine.

41. Procédé selon la revendication 39 **caractérisé en ce que** le peptide est un di- ou tripeptide.

42. Procédé selon la revendication 41 **caractérisé en ce que** le tripeptide est la tri-isoleucine.

43. Procédé selon la revendication 38 **caractérisé en ce que** la masse sèche de la solution ou suspension à sécher contient entre 60 et 80 % (m/m) d'un dérivé du saccharose lié 1,4 O ou d'un mélange de sucres, qui contient au moins un dérivé du saccharose lié 1,4 O et entre 1 et 19,99 % (m/m) d'isoleucine, la somme des pourcentages en masse étant 100 % (m/m) au maximum.

44. Procédé selon la revendication 41 ou 42 **caractérisé en ce que** la masse sèche de la solution ou suspension à sécher contient entre 60 et 80 % (m/m) d'un dérivé du saccharose lié 1,4 O ou un mélange de sucres, qui contient au moins un dérivé du saccharose lié 1,4 O, et entre 1 et 19,99 % (m/m) d'un tripeptide.

45. Procédé selon l'une des revendications 26 à 44 **caractérisé en ce que** la proportion d'adjuvants est entre 25 et 99 % (m/m) de la masse sèche de la solution ou suspension à sécher.

46. Procédé selon l'une des revendications 26 à 45 **caractérisé en ce que** la proportion de principe actif pharmaceutique est entre 0,01 et 75 % (m/m) de la masse sèche de la solution ou suspension à sécher, la somme des pourcentages en masse étant 100 % au maximum.

47. Utilisation de la poudre séchée selon l'une des revendications 1 à 24 pour la production d'un médicament.

48. Utilisation de la poudre séchée par pulvérisation selon la revendication 24 pour la production d'un médicament pour l'inhalation.
